# EUROPEAN PATENT APPLICATION

(11) **EP 4 704 532 A1**
(43) Date of publication of application: **04.03.2026**
(21) Application number: 25191537.7
(22) Date of filing: 24.07.2025
(51) Int. Cl.: H10K 50/11, H10K 85/60, H10K 101/00

(54) **AN ORGANIC LIGHT EMITTING DIODE WITH LOW VOLTAGE, HIGH EFFICIENCY AND LONG LIFESPAN**

(30) Priority: 28.08.2024 KR 20240116261; 15.05.2025 KR 20250063505
(71) Applicant: SFC Co., Ltd., Cheongju-si, Chungcheongbuk-do 28122 (KR)
(72) Inventor: Kim, Kyeong-Hyeon, 28122 Cheongju-si (KR); Jang, Hyuk Woo, 28122 Cheongju-si (KR); Choi, Do Yeong, 28122 Cheongju-si (KR); Park, Seo Youn, 28122 Cheongju-si (KR); Kim, Kyeong Wan, 28122 Cheongju-si (KR)
(74) Representative: Epping - Hermann - Fischer

(57) **Abstract**

Disclosed is an organic light-emitting diode with high efficiency. The organic light-emitting diode includes a compound represented by Chemical Formula 1 as an emission layer material therein. Chemical Formula 1 is as defined in the description.

## Description

### BACKGROUND OF THE INVENTION

### 1. Field of the Invention

The present disclosure relates to an organic light-emitting diode with low voltage, high efficiency, and long lifespan. More specifically, the present disclosure relates to an organic light-emitting diode that can exhibit low voltage, high efficiency, and long lifespan by respectively using compounds having a specific structure as an emission layer material in the organic light-emitting diode.

### 2. Description of the Related Art

Organic light-emitting diodes (OLEDs), based on self-luminescence, are used to create digital displays with the advantage of having a wide viewing angle and being able to be made thinner and lighter than liquid crystal displays. In addition, an OLED display exhibits a very fast response time. Accordingly, OLEDs find applications in the full color display field or the illumination field.

In general, the term "organic light-emitting phenomenon" refers to a phenomenon in which electrical energy is converted to light energy by means of an organic material. An organic light-emitting diode using the organic light-emitting phenomenon has a structure usually including an anode, a cathode, and an organic material layer interposed therebetween.

In this regard, the organic material layer may have, for the most part, a multilayer structure consisting of different materials, for example, a hole injection layer, a hole transport layer, an emission layer, an electron transport layer, and an electron injection layer in order to enhance the efficiency and stability of the organic light-emitting diode. In the organic light-emitting diode having such a structure, application of a voltage between the two electrodes injects a hole from the anode and an electron from the cathode to the organic layer. In the luminescent zone, the hole and the electron recombine to produce an exciton. When the exciton returns to the ground state from the excited state, the molecule of the organic layer emits light. Such an organic light-emitting diode is known to have characteristics such as self-luminescence, high luminance, high efficiency, low driving voltage, a wide viewing angle, high contrast, and high-speed response.

Materials used as organic layers in OLEDs may be divided according to functions into luminescent materials and charge transport materials, for example, a hole injection material, a hole transport material, an electron transport material, and an electron injection material. Electron blocking materials or hole blocking materials may be added as necessary.

In OLEDs, the most critical issues are lifespan and efficiency. As displays have become larger in area, resolving issues related to efficiency and lifespan has become imperative. Here, efficiency, lifespan, and driving voltage are interrelated; as efficiency increases, the driving voltage tends to decrease, and a lower driving voltage reduces Joule heating during operation, thereby decreasing crystallization of organic materials and ultimately extending the device lifespan.

However, simply improving the organic material layers does not necessarily maximize efficiency. This is because achieving both long lifespan and high efficiency requires an optimal combination of factors such as the energy levels and T1 values between the respective organic layers, as well as intrinsic material properties (e.g., mobility, interfacial characteristics).

Generally, in an OLED, electrons are transferred from the electron transport layer to the emission layer, and holes are transferred from the hole transport layer to the emission layer, where they recombine to form excitons.

A technology in which the emission layer in an organic light-emitting diode (OLED) is formed as two or more layers in order to enhance the stability and luminous efficiency of the OLED is being implemented in commercial products. For example, in the case of forming two emission layers, one of the two emission layers corresponds to an exciton recombination region, while the other corresponds to a triplet-triplet fusion region. This structure allows energy to be transferred from the first emission layer, which is adjacent to the hole transport layer, to the second emission layer. In this context, it is advantageous for the materials used in the emission layers to have relatively high triplet energy levels and high electron mobility.

As an example of prior art relating to OLEDs comprising two or more emission layers, Korean Laid-open Patent Publication No. 10-2021-0077686 (published on June 25, 2021) discloses an organic electroluminescent device in which the emission region includes a first emission layer and a second emission layer, the first and second emission layers are directly adjacent to each other, the first emission layer is positioned between the anode and the second emission layer, and at least one of the first or second emission layers comprises a compound having at least one deuterium atom.

However, despite various attempts made in the related art, including those described above, to develop methods for fabricating OLEDs including a plurality of emission layers, there remains a continued need to develop OLEDs that offer further improved light-emission efficiency and long lifespan properties simultaneously.

### Related Art Document

Korean Patent Publication No. 10-2021-0077686 A (June 25, 2021)

### SUMMARY OF THE INVENTION

Accordingly, the present disclosure is to provide an organic light-emitting diode (OLED) including a plurality of emission layers wherein a pyrene-based compound having a specific structure is contained in at least one of the emission layers whereby the organic light-emitting diode exhibits low voltage, high efficiency, and long lifespan characteristics.

To achieve the goal, the present disclosure provides an organic light-emitting diode including: a first electrode; a second electrode facing the first electrode; and a first emission layer containing a first host and a first dopant and a second emission layer containing a second host and a second dopant, both layer being interposed between the first electrode and the second electrode, wherein at least one of the first host and the second host includes a compound represented by the following Chemical Formula 1]: 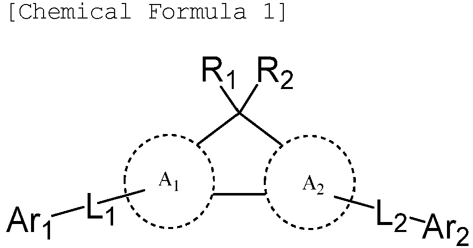 wherein,
A₁ is a substituted or unsubstituted aromatic hydrocarbon ring of 6 to 14 carbon atoms,
A₂ is a substituted or unsubstituted aromatic hydrocarbon ring of 6 to 14 carbon atoms, or a substituted or unsubstituted aliphatic hydrocarbon ring-fused aromatic hydrocarbon ring of 8 to 20 carbon atoms wherein the-aromatic ring moiety within the aromatic hydrocarbon ring fused to the aliphatic hydrocarbon ring has 6 to 14 carbon atoms,
the substituents R₁ and R₂, which are same or different, are each independently any one selected from a substituted or unsubstituted alkyl of 1 to 30 carbon atoms, a substituted or unsubstituted aryl of 6 to 50 carbon atoms, a substituted or unsubstituted heteroaryl of 2 to 50 carbon atoms, and a substituted or unsubstituted aliphatic hydrocarbon ring-fused aryl of 8 to 30 carbon atoms,
the substituents Ar₁ and Ar₂, which are same or different, are each independently any one selected from a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted alkoxy of 1 to 30 carbon atoms, a substituted or unsubstituted aryloxy of 6 to 30 carbon atoms, a substituted or unsubstituted cycloalkyloxy of 3 to 30 carbon atoms, a substituted or unsubstituted heteroaryloxy of 2 to 30 carbon atoms, a substituted or unsubstituted alkylthio of 1 to 30 carbon atoms, a substituted or unsubstituted arylthio of 6 to 30 carbon atoms, a substituted or unsubstituted cycloalkylthio of 3 to 30 carbon atoms, a substituted or unsubstituted heteroarylthio of 2 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
L₁ and L₂, which are same or different, are each independently a linker selected from a single bond, a substituted or unsubstituted arylene of 6 to 24 carbon atoms, a substituted or unsubstituted heteroarylene of 3 to 24 carbon atoms, and a substituted or unsubstituted aliphatic hydrocarbon ring-fused arylene of 8 to 24 carbon atoms,
   wherein the term "substituted" in the expression "a substituted or unsubstituted" used for the compounds of Chemical Formula 1 means having at least one substituent selected from the group consisting of a deuterium atom, a tritium atom, a cyano, a halogen, a thiol, a hydroxy, a nitro, alkyl of 1 to 30 carbon atoms, halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocyclic alkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with at least one hydrogen atom on the substituent being substitutable with a deuterium or tritium atom.

Also, the present disclosure may provide a compound represented by Chemical Formula 1 which can be used in an emission layer in organic light-emitting diodes.

The organic light-emitting diode (OLED) according to the present disclosure can exhibit excellent device characteristics such as low driving voltage, high light-emission efficiency, and long lifespan.

Particularly, when at least one of the multiple emission layers employs a compound represented by Chemical Formula 1 while at least one of the remaining emission layers employs an anthracene compound represented by Chemical Formula 2, the OLED can exhibit low-voltage driving, high luminous efficiency, and long lifespan characteristics.

### BRIEF DESCRIPTION OF THE DRAWINGS

FIGURE is a schematic diagram of an organic light-emitting diode according to an embodiment of the present disclosure.

### DETAILED DESCRIPTION

Below, a detailed description will be given of the present disclosure. In each drawing of the present disclosure, sizes or scales of components may be enlarged or reduced from their actual sizes or scales for better illustration, and known components may not be depicted therein to clearly show features of the present disclosure. Therefore, the present disclosure is not limited to the drawings. When describing the principle of the embodiments of the present disclosure in detail, details of well-known functions and features may be omitted to avoid unnecessarily obscuring the presented embodiments.

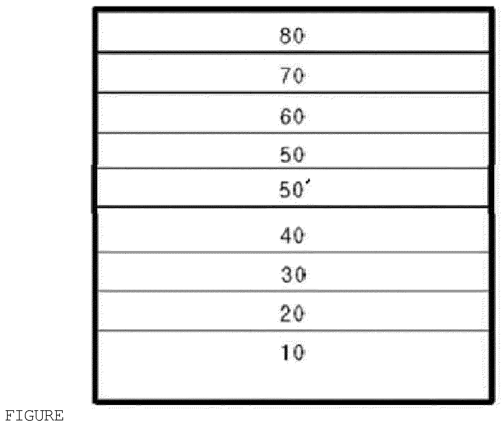

In the drawing, for convenience of description, sizes of components may be exaggerated for clarity. For example, since sizes and thicknesses of components in drawings are arbitrarily shown for convenience of description, the sizes and thicknesses are not limited thereto. Furthermore, throughout the description, the terms "on" and "over" are used to refer to the relative positioning, and mean not only that one component or layer is directly disposed on another component or layer but also that one component or layer is indirectly disposed on another component or layer with a further component or layer being interposed therebetween. Also, spatially relative terms, such as "below", "beneath", "lower", and "between" may be used herein for ease of description to refer to the relative positioning.

Throughout the specification, when a portion may "include" a certain constituent element, unless explicitly described to the contrary, it may not be construed to exclude another constituent element but may be construed to further include other constituent elements. Further, throughout the specification, the word "on" means positioning on or below the object portion, but does not essentially mean positioning on the lower side of the object portion based on a gravity direction.

The present disclosure provides an organic light-emitting diode including: a first electrode; a second electrode facing the first electrode; and a first emission layer containing a first host and a first dopant and a second emission layer containing a second host and a second dopant, both layers being interposed between the first electrode and the second electrode, wherein at least one of the first host and the second host includes a compound represented by the following Chemical Formula 1: 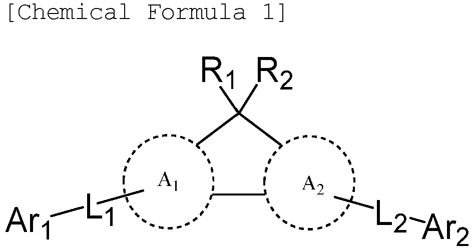 wherein,
A₁ is a substituted or unsubstituted aromatic hydrocarbon ring of 6 to 14 carbon atoms,
A₂ is a substituted or unsubstituted aromatic hydrocarbon ring of 6 to 14 carbon atoms, or a substituted or unsubstituted aliphatic hydrocarbon ring-fused aromatic hydrocarbon ring of 8 to 20 carbon atoms wherein the aromatic ring moiety within the aromatic hydrocarbon ring fused to the aliphatic hydrocarbon ring has 6 to 14 carbon atoms,
the substituents R₁ and R₂, which are same or different, are each independently any one selected from a substituted or unsubstituted alkyl of 1 to 30 carbon atoms, a substituted or unsubstituted aryl of 6 to 50 carbon atoms, a substituted or unsubstituted heteroaryl of 2 to 50 carbon atoms, and a substituted or unsubstituted aliphatic hydrocarbon ring-fused aryl of 8 to 30 carbon atoms,
the substituents Ar₁ and Ar₂, which are same or different, are each independently any one selected from a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted alkoxy of 1 to 30 carbon atoms, a substituted or unsubstituted aryloxy of 6 to 30 carbon atoms, a substituted or unsubstituted cycloalkyloxy of 3 to 30 carbon atoms, a substituted or unsubstituted heteroaryloxy of 2 to 30 carbon atoms, a substituted or unsubstituted alkylthio of 1 to 30 carbon atoms, a substituted or unsubstituted arylthio of 6 to 30 carbon atoms, a substituted or unsubstituted cycloalkylthio of 3 to 30 carbon atoms, a substituted or unsubstituted heteroarylthio of 2 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
L₁ and L₂, which are same or different, are each independently a linker selected from a single bond, a substituted or unsubstituted arylene of 6 to 24 carbon atoms, a substituted or unsubstituted heteroarylene of 3 to 24 carbon atoms, and a substituted or unsubstituted aliphatic hydrocarbon ring-fused arylene of 8 to 24 carbon atoms,
   wherein the term "substituted" in the expression "a substituted or unsubstituted" used for the compounds of Chemical Formula 1 means having at least one substituent selected from the group consisting of a deuterium atom, a tritium atom, a cyano, a halogen, a thiol, a hydroxy, a nitro, alkyl of 1 to 30 carbon atoms, halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocyclic alkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with at least one hydrogen atom on the substituent being substitutable with a deuterium or tritium atom.

The expression indicating the number of carbon atoms, such as "a substituted or unsubstituted alkyl of 1 to 30 carbon atoms", "a substituted or unsubstituted aryl of 5 to 50 carbon atoms", etc. means the total number of carbon atoms of, for example, the alkyl or aryl radical or moiety alone, exclusive of the number of carbon atoms of substituents attached thereto. For instance, a phenyl group with a butyl at the para position falls within the scope of an aryl of 6 carbon atoms, even though it is substituted with a butyl radical of 4 carbon atoms.

As used herein, the term "aryl" means an organic radical derived from an aromatic hydrocarbon by removing one hydrogen that is bonded to the aromatic hydrocarbon. When the aryl group is substituted, the substituents may be fused with one another to form an additional ring. The aryl group may also include an organic radical obtained by the removal of a single hydrogen atom from an arene ring formed by the fusion of two arene rings.

Concrete examples of the aryl include aromatic radical groups such as phenyl, o-biphenyl, m-biphenyl, p-biphenyl, **o-**terphenyl, m-terphenyl, p-terphenyl, naphthyl, anthryl, phenanthryl, pyrenyl, indenyl, fluorenyl, spirobifluorenyl, tetrahydronaphthyl, perylenyl, chrysenyl, naphthacenyl, fluoranthenyl, and triphenylenyl, but are not limited thereto. The aryl group may also include organic radicals obtained by the removal of one hydrogen atom from a fused arene ring formed by two arene rings, such as a fluorene ring fused with a phenylene ring or a fluorene ring fused with a phenanthrene ring.

In addition, at least one hydrogen atom on the aryl group may be substituted by a deuterium atom, a halogen atom, a hydroxy, a nitro, a cyano, a silyl, an amino, a germanium, an amidino, a hydrazino, a hydrazone, a carboxyl, a sulfonic acid, a phosphoric acid, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, or an alkyl heteroaryl of 3 to 24 carbon atoms.

As used herein, the term "aromatic hydrocarbon ring" refers to an aromatic ring composed of carbon and hydrogen atoms and the term "aliphatic hydrocarbon ring" refers to a hydrocarbon ring that is composed of carbon and hydrogen atoms, but does not belong to the aromatic hydrocarbon rings. Particularly, the aliphatic hydrocarbon ring may have a bonding structure of the sp³ orbital for at least 30% of the carbon atoms as ring members, with 0 to 3 double and/or triple bonds within the ring. More particularly, the aliphatic hydrocarbon ring may have a bonding structure of the sp³ orbital for at least 50% of the carbon atoms as ring members, with 0 to 2 double and/or triple bonds within the ring.

The term "aliphatic hydrocarbon ring-fused aryl", as used herein, refers to a cyclic radical in which two adjacent carbon atoms as ring members of an aliphatic hydrocarbon ring and two adjacent carbon atoms as ring members of an aryl are fused with each other to share one double bond therebetween, with non-aromaticity across the molecule. Specific examples include, but are not limited to, tetrahydronaphthyl, tetrahydrobenzocycloheptene, tetrahydrophenanthrene, tetrahydroanthracenyl, and octahydrotriphenylene.

The substituent "heteroaryl", used in the compound of the present disclosure, means a hetero aromatic radical of 2 to 24 carbon atoms, bearing as ring member(s) one to three heteroatoms selected from among N, O, P, Si, S, Ge, Se, and Te. In the aromatic radical, two or more rings may be fused. One or more hydrogen atoms on the heteroaryl may be substituted by the same substituents as on the aryl.

Concrete examples of the heteroaryl include thiophenyl, furanyl, pyrrolyl, imidazolyl, thiazolyl, oxazolyl, oxadiazolyl, triazolyl, pyridyl, bipyridyl, pyrimidinyl, triazinyl, acridinyl, carbazolyl, acenaphthoquinoxalinyl, indenoquinazolinyl, indenoisoquinolinyl, indenoquinolinyl, pyridoindolyl, pyridazinyl, pyrazinyl, quinolinyl, quinazolinyl, quinoxalinyl, phthalazinyl, pyridopyrimidinyl, pyridopyrazinyl, pyrazinopyrazinyl, isoquinolinyl, indolyl, carbazolyl, benzoxazolyl, benzimidazolyl, benzothiazolyl, benzocarbazolyl, benzofuranyl, benzothiophenyl, benzoselenophenyl, dibenzothiophenyl, dibenzofuranyl, dibenzoselenophenyl, phenanthrolinyl, thiazolinyl, isoxazolyl, thiadiazolyl, benzothiazolyl, phenoxazinyl, phenothiazinyl, azadibenzofuranyl, azadibenzothiophenyl, azadibenzoselenophenyl, indolocarbazolyl, and the like, but are not limited thereto.

In addition, the term "heteroaromatic ring", as used herein, refers to an aromatic hydrocarbon ring bearing at least one heteroatom as an aromatic ring member. In the heteroaromatic ring, one to three carbon atoms of the aromatic hydrocarbon may be substituted by at least one selected particularly from N, O, P, Si, S, Ge, Se, and Te.

As used herein, the term "aliphatic hydrocarbon ring-fused heteroaryl" refers to the same cyclic substituent as in the aliphatic hydrocarbon ring-fused aryl, with the exception that a heteroaryl, instead of an aryl, is substituted. Specific examples include, but are not limited to, tetrahydroindole, tetrahydrobenzofuranyl, tetrahydrobenzothiophene, tetrahydrocarbazole, tetrahydrodibenzofuranyl, tetrahydroquinoline, and tetrahydroquinoxaline.

Furthermore, the term "heteroaromatic ring", as used herein, refers to an aromatic hydrocarbon ring bearing at least one heteroatom as an aromatic ring member. In the heteroaromatic ring, one to three carbon atoms of the aromatic hydrocarbon may be substituted by at least one selected particularly from N, O, P, Si, S, Ge, Se, and Te.

As used herein, the term "fused ring in which an aromatic hydrocarbon ring is fused with an aliphatic hydrocarbon ring" means a fused ring in which an aromatic hydrocarbon ring has two adjacent carbon atoms in common with an aliphatic hydrocarbon ring, as exemplified by a tetrahydronaphthalene ring, dihydroindene, and the like, in which the benzene ring shares two adjacent carbon atoms with the cyclohexane ring.

In the present disclosure, the term "fused ring with a heteroaromatic hydrocarbon ring and an aliphatic hydrocarbon ring fused to each other" refer to a cyclic radical in which two adjacent carbon atoms as ring members of an aromatic hydrocarbon ring and two adjacent carbon atoms as ring members of an aliphatic hydrocarbon ring are fused with each other to share two carbon atoms therebetween, as exemplified by hexahydrodibenzofuran in which a benzofuran ring and a cyclohexane ring are fused by sharing their respective two adjacent carbon atoms as ring members.

As used herein, the term "alkyl" refers to an alkane missing one hydrogen atom and includes linear or branched structures. Examples of the alkyl substituent useful in the present disclosure include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclo hexylmethyl, octyl, n- octyl, tert- octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like. At least one hydrogen atom of the alkyl may be substituted by the same substituent as in the aryl.

As used herein, the term "halogenated alkyl" refers to an alkyl with at least one hydrogen atom substituted by a halogen. Preferably, the halogen may be a fluorine atom.

The term "cyclo" as used in substituents of the compounds of the present disclosure, such as cycloalkyl, cycloalkoxy, etc., refers to a structure responsible for a mono- or polycyclic ring of saturated hydrocarbons within an alkyl radical, an alkoxy radical, etc. Concrete examples of cycloalkyl include cyclopropyl, cyclobutyl, cyclopentyl, cyclohexyl, methylcyclopentyl, methylcyclohexyl, ethylcyclopentyl, ethylcyclohexyl, adamantyl, dicyclopentadienyl, decahydronaphthyl, norbornyl, bornyl, isobornyl, and so on. One or more hydrogen atoms on the cycloalkyl may be substituted by the same substituents as on the aryl. This is true of the cycloalkoxy.

In the present disclosure, the term "heterocycloalkyl" refers to a cycloalkyl with at least one heteroatom substituted as a ring member for a carbon atom within the ring. Preferably, one to three carbon atoms within the ring may be substituted by at least one selected from N, O, P, S, Si, Ge, Se, and Te.

The term "aromatic hydrocarbon ring- or heteroaromatic ring-fused cycloalkyl", as used herein, refers to a cyclic radical in which two adjacent carbon atoms as ring members of an aromatic or heteroaromatic hydrocarbon ring and two adjacent carbon atoms as ring members of a cycloalkyl are fused with each other to share one double bond therebetween, with non-aromaticity across the molecule. Specific examples include tetrahydronaphthyl, tetrahydrophenanthrene, tetrahydroquinoline, tetrahydroquinoxaline, and cyclopentabenzofuran.

The term "aromatic hydrocarbon ring-fused heterocyclic alkyl" is same as the aromatic hydrocarbon ring-fused cycloalkyl, with the exception that at least one carbon atom within the cycloalkyl ring is substituted by a heteroatom, with non-aromaticity across the molecule. Preferably, one to three carbon atoms within the cycloalkyl ring are substituted by at least one selected from N, O, P, S, Si, Ge, Se, and Te. Specific examples of aromatic hydrocarbon ring-heterocyclic alkyl include hexahydrodibenzofuranyl, hexahydrocarbozole, hexahydrodibenzothiophene, and dihydrobenzodioxine.

As used herein, the "heteroaliphatic ring-fused aryl or heteroaryl" is same as the aliphatic hydrocarbon ring-fused aryl or heteroaryl, except for a heteroaliphatic ring substituted for the aliphatic hydrocarbon ring, with non-aromaticity across the molecule thereof. Specific examples include cromane, dihydropyranopyridine, thiocromane, dihydrobenzodioxine, dihydrothiopyranopyridine, and dihydropyranopyrimidine.

The term "heteroaliphatic ring" refers to an aliphatic hydrocarbon bearing at least one heteroatom as a ring member. Preferably, one to three carbon atoms within an aliphatic hydrocarbon are substituted by at least one heteroatom selected from N, O, and S.

The term "alkoxy," as used in the compounds of the present disclosure, refers to an alkyl or cycloalkyl singularly bonded to oxygen. Specific examples of the alkoxy include methoxy, ethoxy, propoxy, isobutoxy, sec-butoxy, pentoxy, iso-amyloxy, hexyloxy, cyclobutyloxy, cyclopentyloxy, adamantyloxy, dicyclopentyloxy, bornyloxy, isobornyloxy, and the like, but are not limited thereto. One or more hydrogen atoms on the alkoxy may be substituted by the same substituents as on the aryl.

Specifice examples of the arylalkyl used in the compounds of the present disclosure include phenylmethyl (benzyl), phenylethyl, phenylpropyl, naphthylmethyl, naphthylethyl, and the like, but are not limited thereto. One or more hydrogen atoms on the arylalkyl may be substituted by the same substituents as on the aryl.

Specific examples of the alkylaryl used in the compound of the present disclosure include tolyl, xylenyl, dimethylnaphthyl, t-butylphenyl, t-butylnaphthyl, and t-butylphenanthryl, but are not limited thereto. One or more hydrogen atoms on the alkylaryl may be substituted by the same substituents as on the aryl.

As used herein, the term "alkenyl" refers to an alkyl substituent containing a carbon-carbon double bond between two carbon atoms and the term "alkynyl" refers to an alkyl substituent containing a carbon-carbon triple bond between two carbon atoms.

As used herein, the term "alkylene" refers to an organic radical regarded as derived from an alkane by removal two hydrogen atoms from one carbon atom for methylene or different carbon atoms for ethylene or higher, such as methylene, ethylene, propylene, isopropylene, isobutylene, sec-butylene, tert-butylene, pentylene, iso-amylene, hexylene, and the like. One or more hydrogen atoms on the alkylene may be substituted by the same substituents as on the aryl.

In the present disclosure, the term "amine" is intended to encompass -NH₂, alkylamine, arylamine, alkylarylamine, arylheteroarylamine, heteroarylamine, and the like. The term "arylamine" refers to a functional group including -NH₂, in which one or two of the hydrogen atoms in -NH₂ are substituted by aryl; the term "alkylamine" to a functional group including -NH₂, in which one or two of the hydrogen atoms in -NH₂ are substituted by alkyl; the term "alkylarylamine" to a functional group including -NH₂, in which one of the hydrogen atoms in -NH₂ is substituted by alkyl and the other hydrogen atom is substituted by aryl; the term "arylheteroarylamine to a functional group including -NH₂, in which one of the hydrogen atoms in -NH₂ is substituted by aryl and the other hydrogen atom is substituted by heteroaryl; and the term "heteroarylamine" to a functional group including -NH₂, in which one or two of the hydrogen atoms in -NH₂ are substituted by heteroaryl. Examples of the arylamine include a substituted or unsubstituted monoarylamine and a substituted or unsubstituted diarylamine. Such examples are true of the alkylamine and the heteroarylamine.

Each of the aryl radicals in the arylamine, heteroarylamine, and arylheteroarylamine may be a monocyclic or polycyclic one. Each of the heteroaryl radicals in the heteroarylamine, and arylheteroarylamine may be a mono- or polycyclic one.

The term "silyl" used in the compounds of the present disclosure is intended to encompass -SiH₃, alkylsilyl, arylsilyl, alkylarylsilyl, arylheteroarylsilyl, heteroarylsilyl, and the like. The arylsilyl refers to a functional group based on -SiH₃ in which at least one of the three hydrogen atoms bonded to the silicon atom is substituted by aryl; the alkylsilyl to a functional group based on -SiH₃ in which at least one of the three hydrogen atoms bonded to the silicon atom is substituted by alkyl; the alkylarylsilyl to a functional group based on - SiH₃ in which at least two of the three hydrogen atoms bonded to the silicon atom are substituted by alkyl and aryl, respectively, as exemplified by a silyl having one or two alkyl radicals and correspondingly two or one aryl radicals; the arylheteroarylsilyl to a functional group based on -SiH₃ in which at least two of the three hydrogen atoms bonded to the silicon atom are substituted by aryl and heteroaryl, as exemplified by a silyl having one or two aryl radicals and correspondingly two or one heteroaryl radical; and the heteroarylsilyl to a functional group based on -SiH₃ in which at least one of the three hydrogen atoms bonded to the silicon atom is substituted by heteroaryl. Examples of the arylsilyl include a substituted or unsubstituted monoarylsilyl, a substituted or unsubstituted diarylsilyl, and a substituted or unsubstituted triarylsilyl. Such examples are true of the alkylsilyl and the heteroarylsilyl.

Here, each of the aryl radicals in the arylsilyl, heteroarylsilyl, and arylheteroarylsilyl in the heteroarylamine may be a mono- or polycyclic aryl, and each of the heteroaryl radicals in the arylsilyl, heteroarylsilyl, and arylheteroarylsilyl may be a mono- or polycyclic heteroaryl.

Furthermore, specific examples of the silyl include trimethylsilyl, triethylsilyl, triphenylsilyl, trimethoxysilyl, dimethoxyphenylsilyl, diphenylmethylsilyl, diphenylvinylsilyl, methylcyclobutylsilyl, and dimethylfurylsilyl. One or more hydrogen atoms on the silyl may be substituted by the same substituents as on the aryl.

As used herein, the term "germanium" (or germyl or germane) is intended to encompass -GeH₃, alkyl germanium, aryl germanium, heteroaryl germanium, alkylaryl germanium, alkylheteroaryl germanium, and arylheteroaryl germanium and can be accounted for by the definitions for silyl radicals, with the exception that the silicone atom (Si) in the silyl radical is substituted by a germanium atom (Ge).

Specific examples of the germanium radical include trimethylgermane, triethylgermane, triphenylgermane, trimethoxygermane, dimethoxyphenylgermane, diphenylmethylgermane, diphenylvinylgermane, methylcyclobutylgermane, and dimethylfurylgermane. One or more hydrogen atoms on the germanium radical may be substituted by the same substituents as on the aryl.

As used herein, the substituent (B) adjacent to a substituent (A) within an aromatic ring means a substituent (B) bonded to a carbon atom(s) as a ring member(s) adjacent to a carbon atom as a ring member having the substituent (A) bonded thereto, within the aromatic ring. Also, the substituent (B) adjacent to a substituent (A) within an aliphatic ring means a substituent (B) bonded to a carbon atom(s) as a ring member(s) adjacent to a carbon atom as a ring member having the substituent (A) bonded thereto, within the aliphatic ring. Further, the substituent (B) adjacent to a substituent (A) within an aliphatic chain structure means a substituent (B) bonded to a carbon atom(s) as a ring member(s) adjacent to a carbon atom as a ring member having the substituent (A) bonded thereto, within the aliphatic chain structure.

In a preferable embodiment, the substituent accounted for by the term "substituted" in the expression "substituted or unsubstituted" used for compounds of Chemical Formula 1 may be at least one selected from the group consisting of a deuterium atom, a cyano, a halogen, a hydroxy, a nitro, an alkyl of 1 to 12 carbon atoms, a halogenated alkyl of 1 to 12 carbon atoms, an alkenyl of 2 to 12 carbon atoms, an alkynyl of 2 to 12 carbon atoms, a cycloalkyl of 3 to 12 carbon atoms, a heteroalkyl of 1 to 12 carbon atoms, an aryl of 6 to 18 carbon atoms, an arylalkyl of 7 to 20 carbon atoms, an alkylaryl of 7 to 20 carbon atoms, a heteroaryl of 2 to 18 carbon atoms, a heteroarylalkyl of 3 to 18 carbon atoms, an alkyl heteroaryl of 3 to 18 carbon atoms, an aromatic hydrocarbon ring-fused cycloalkyl of 9 to 20 carbon atoms, a heteroaromatic ring-fused cycloalkyl of 7 to 20 carbon atoms, an aromatic hydrocarbon ring-fused heterocycloalkyl of 9 to 20 carbon atoms, an aliphatic hydrocarbon ring-fused aryl of 9 to 20 carbon atoms, an aliphatic hydrocarbon ring-fused heteroaryl of 7 to 20 carbon atoms, an alkoxy of 1 to 12 carbon atoms, an amine of 1 to 18 carbon atoms, a silyl of 1 to 18 carbon atoms, a germanium of 1 to 18 carbon atoms, an aryloxy of 6 to 18 carbon atoms, and an arylthionyl of 6 to 18 carbon atoms **6.** At least one hydrogen atom within each substituent may be substituted with a deuterium or tritium atom.

In a more preferable embodiment of the present disclosure, the substituted or unsubstituted aromatic hydrocarbon ring-fused cycloalkyl of 7 to 30 carbon atoms may be a substituted or unsubstituted aromatic hydrocarbon ring-fused cycloalkyl of 9 to 20 carbon atoms.

In a more preferable embodiment of the present disclosure, the substituted or unsubstituted heteroaromatic ring-fused cycloalkyl of 5 to 30 carbon atoms may be a substituted or unsubstituted heteroaromatic ring-fused cycloalkyl of 7 to 20 carbon atoms.

In a more preferable embodiment of the present disclosure, the substituted or unsubstituted aromatic hydrocarbon ring-fused heterocycloalkyl of 6 to 30 carbon atoms may be a substituted or unsubstituted aromatic hydrocarbon ring-fused heterocycloalkyl of 9 to 20 carbon atoms.

In a more preferable embodiment of the present disclosure, the substituted or unsubstituted aliphatic hydrocarbon ring-fused aryl of 8 to 30 carbon atoms may be a substituted or unsubstituted aliphatic hydrocarbon ring-fused aryl of 9 to 20 carbon atoms.

In a more preferable embodiment of the present disclosure, the substituted or unsubstituted aliphatic hydrocarbon ring-fused heteroaryl of 5 to 30 carbon atoms may be a substituted or unsubstituted aliphatic hydrocarbon ring-fused heteroaryl of 7 to 20 carbon atoms.

In a more preferable embodiment of the present disclosure, the substituted or unsubstituted heteroaliphatic ring-fused aryl of 6 to 30 carbon atoms may be a substituted or unsubstituted heteroaliphatic ring-fused aryl of 7 to 20 carbon atoms.

In a more preferable embodiment of the present disclosure, the substituted or unsubstituted heteroaliphatic ring-fused heteroaryl of 5 to 30 carbon atoms may be a substituted or unsubstituted heteroaliphatic ring-fused heteroaryl of 6 to 20 carbon atoms.

As used herein, the expression "R₃₁ to R₃₇ may be linked to the A₁ to A₃ ring moieties to additionally form a mono- or polycyclic aliphatic or aromatic ring" means that each substituent selected from R₃₁ to R₃₇ (i.e., each of the R₃₁ to R₃₇ substituents) may form an additional ring by removing one hydrogen radical from the substituent and one hydrogen radical from each of the rings A₁ to A₃ for the purpose of forming a mono- or polycyclic aliphatic or aromatic ring, and then linking them together. Likewise, in the expression "a linkage may be made between R₃₂ and R₃₃, between R₃₄ and R₃₅, and between R₃₆ and R₃₇ to additionally form a mono- or polycyclic aliphatic or aromatic ring," when, for example, R₃₂ and R₃₃ form a ring, it means that one hydrogen radical is removed from R₃₂ and one hydrogen radical is removed from R₃₃, and the two are then connected to additionally form a ring.

As a material for at least one emission layer in the organic light-emitting diode including a plurality of emission layers according to the present disclosure, the compound represented by Chemical Formula 1 is used. The compound represented by Chemical Formula 1 is based on a five-membered ring that has R₁ and R₂ as substituents at one ring member. The five-membered ring is fused with an A₁ and A₂ rings at opposite sides thereof, respectively. The A₁ ring is a substituted or unsubstituted aromatic hydrocarbon ring of 6 to 14 carbon atoms while the A₂ ring is a substituted or unsubstituted aromatic hydrocarbon ring of 6 to 14 carbon atoms or a substituted or unsubstituted aliphatic hydrocarbon ring-fused aromatic hydrocarbon ring of 8 to 20 carbon atoms, wherein the aromatic ring moiety within the aliphatic hydrocarbon ring-fused aromatic hydrocarbon ring contains 6 to 14 carbon atoms and the A₁ and A₂ rings are bonded to 'L₁-Ar₁' and 'L₂-Ar₂', respectively. Compared to compounds wherein none or only one of the A₁ and A₂ rings has any one of the substituents, the structure represented by Chemical Formula 1 was found to relatively decrease the T₁ value that is maintained lower than the T₁ value with the dopant used in the same layer, thus increasing the occurrence rate of triplet-triplet fusion. As a result, it becomes possible to realize an OLED exhibiting high efficiency and long lifetime characteristics compared to conventional OLEDs.

According to an embodiment, R₁ and R₂ in Chemical Formula 1, which are same or different, are each independently at least one selected from a substituted or unsubstituted alkyl of 1 to 20 carbon atoms.

According to an embodiment, A₁ in Chemical Formula 1 may be any one of a substituted or unsubstituted benzene ring, a substituted or unsubstituted naphthalene ring, and a substituted or unsubstituted phenanthrene ring, with more preference for a substituted or unsubstituted phenanthrene ring.

According to an embodiment, A₂ in Chemical Formula 1 may be a substituted or unsubstituted aromatic hydrocarbon ring of 6 to 14 carbon atoms.

According to an embodiment, Ar₁ and Ar₂ in Chemical Formula 1, which are same or different, are each independently any one selected from a substituted or unsubstituted aryl of 6 to 18, a substituted or unsubstituted heteroaryl of 2 to 18, a substituted or unsubstituted aliphatic hydrocarbon ring-fused aryl of 8 to 20 carbon atoms, a substituted or unsubstituted a silyl of 1 to 30 carbon atoms, and a substituted or unsubstituted germanium of 1 to 30 carbon atoms.

According to an embodiment, concrete examples of the compound represented by Chemical Formula 1 include, but are not limited to, the following Compounds 1-1 to 1-108:

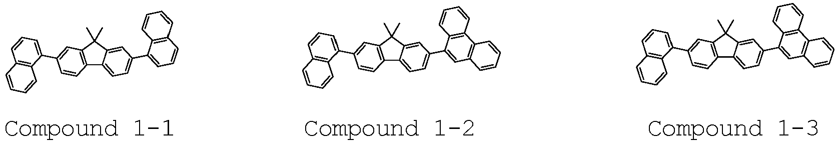

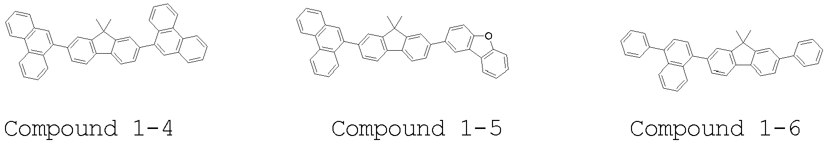

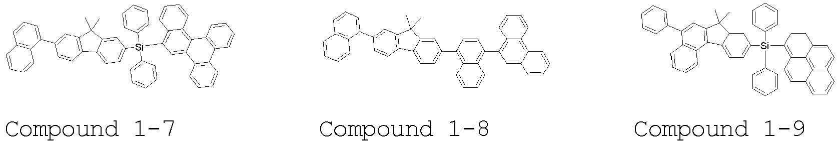

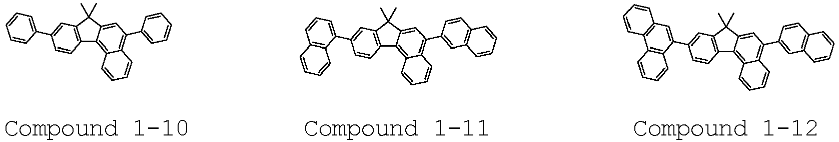

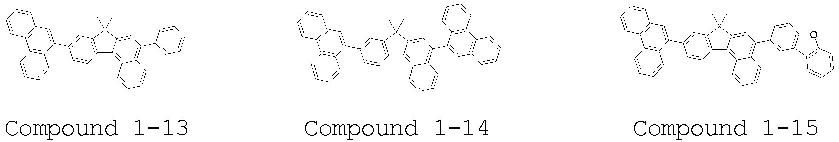

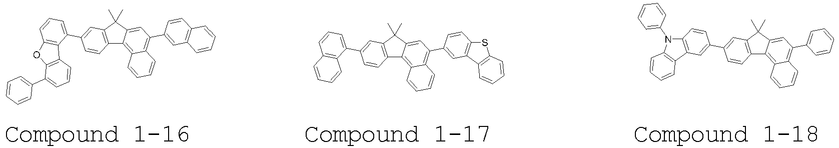

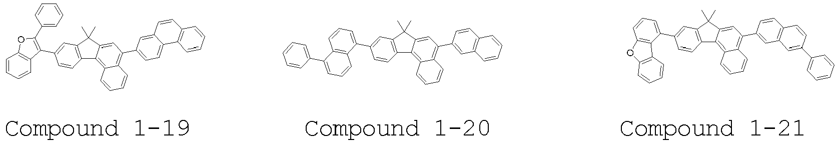

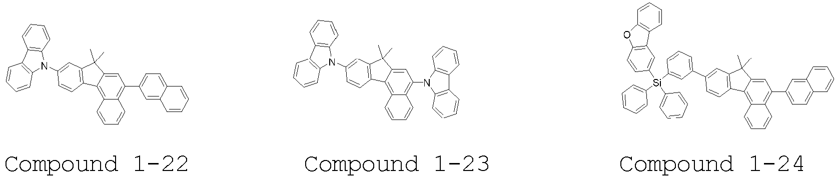

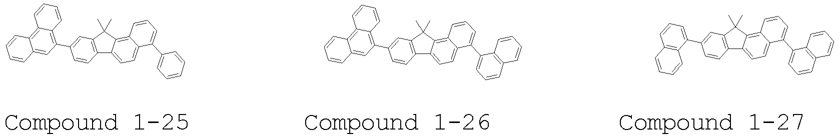

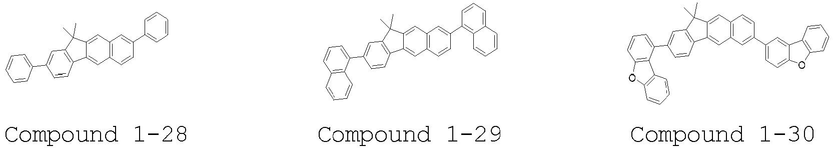

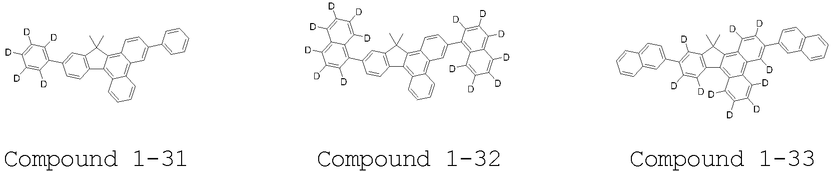

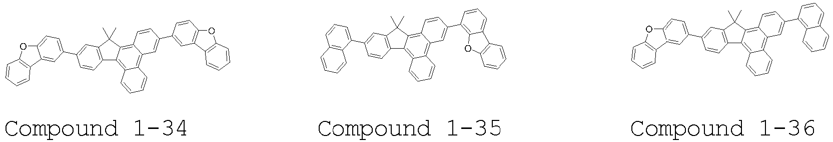

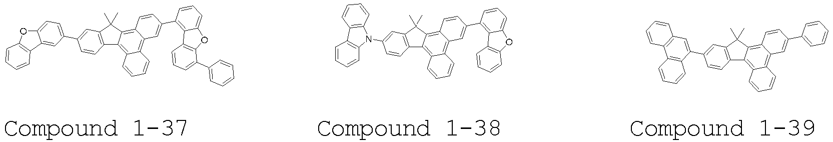

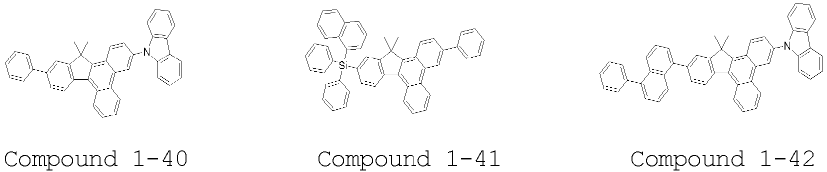

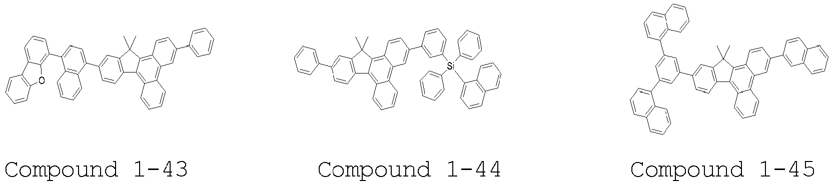

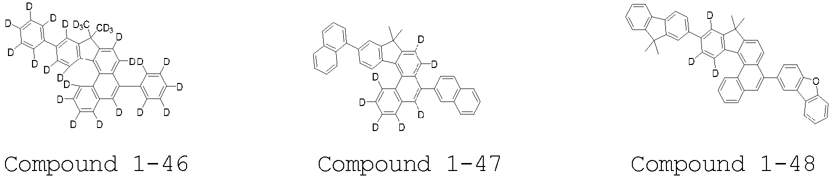

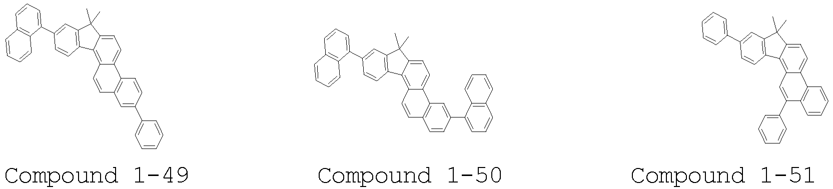

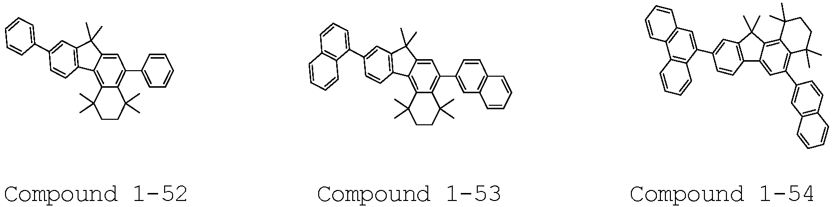

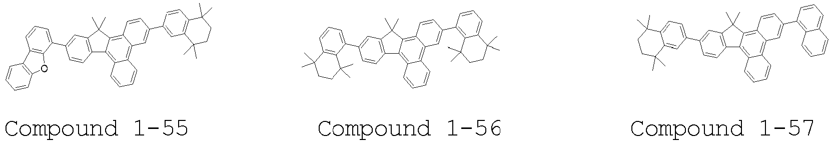

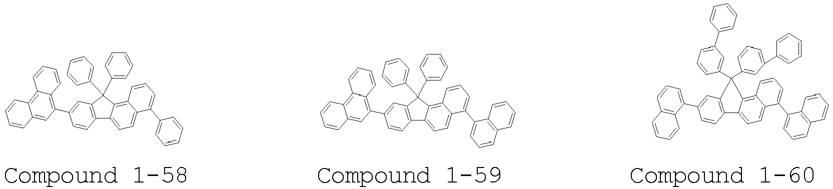

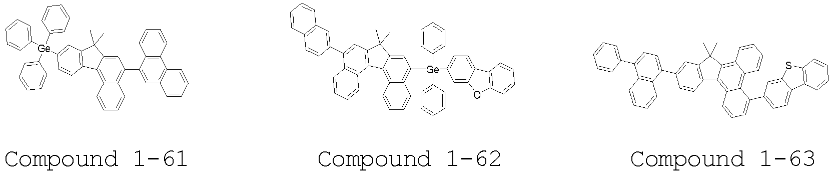

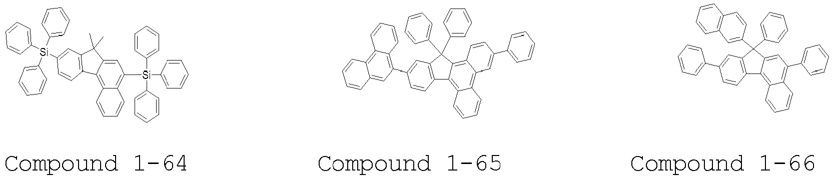

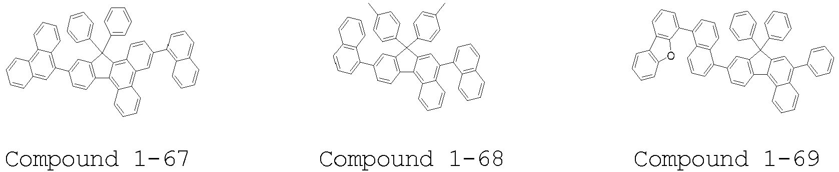

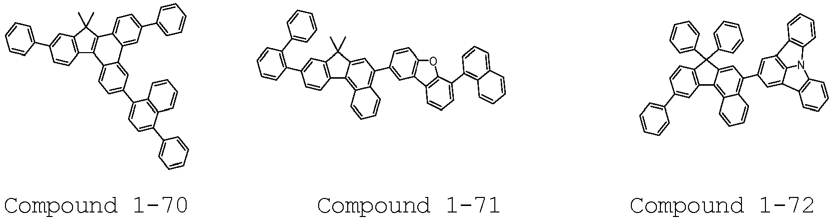

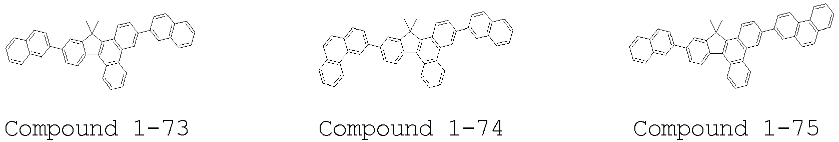

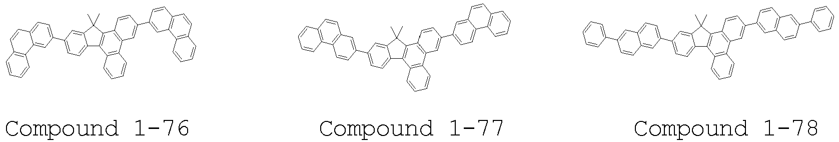

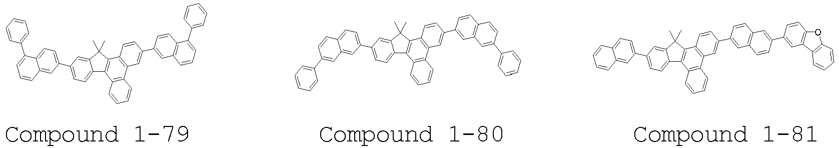

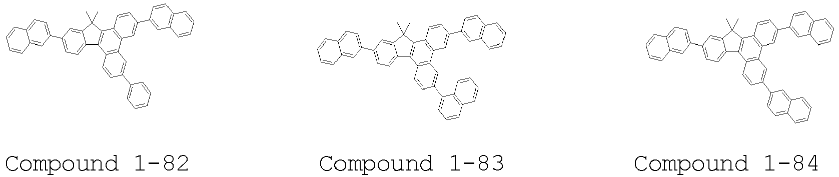

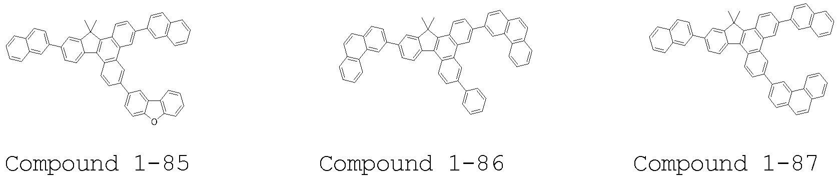

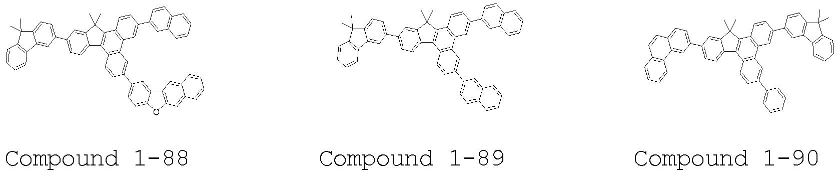

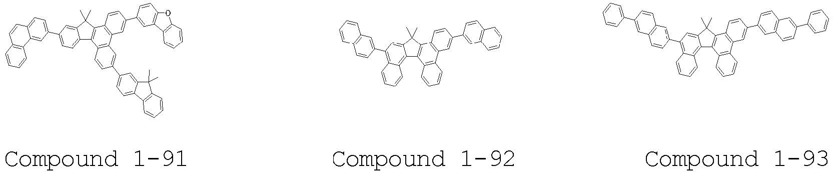

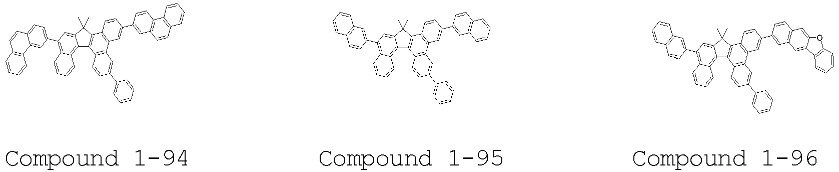

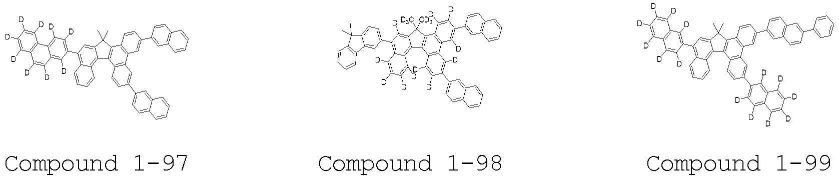

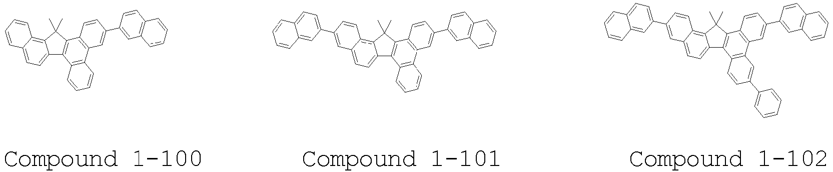

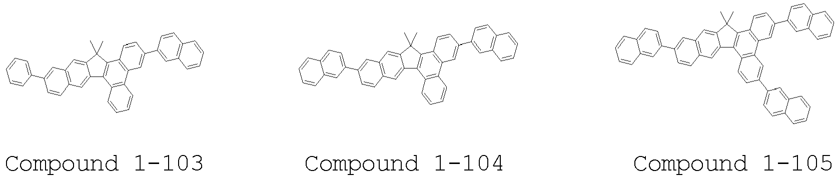

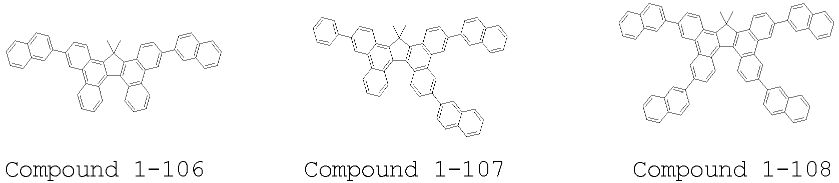

Throughout the description of the present disclosure, the phrase "(organic layer or emission layer) includes at least one organic compound" may be construed to mean that "(organic layer) may include a single organic compound species or two or more different species of organic compounds falling within the scope of the present disclosure".

In the organic light-emitting diode according to the present disclosure, the compound represented by Chemical Formula 1 may be used as a host in the first or second emission layer. That is, the OLED according to the present disclosure may include one or more of the compounds represented by Chemical Formula 1 as a host material in the emission layer.

Of the first and second hosts within the organic light-emitting diode according to an embodiment of the present disclosure, one may include a compound represented by Chemical Formula 1 while the other may include an anthracene compound represented by Chemical Formula 2. For instance, the first host within the organic light-emitting diode according to the present disclosure may include a compound represented by Chemical Formula 1 while the second host may include an anthracene compound represented by Chemical Formula 2, and *vice versa.* 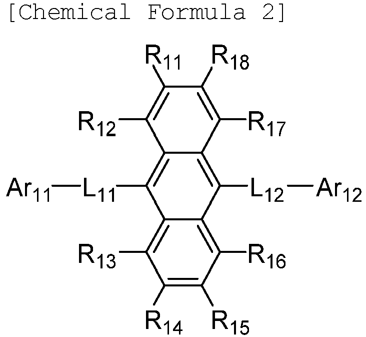 wherein,
the substituents R₁₁ to R₁₈, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted alkoxy of 1 to 30 carbon atoms, a substituted or unsubstituted aryloxy of 6 to 30 carbon atoms, a substituted or unsubstituted cycloalkyloxy of 3 to 30 carbon atoms, a substituted or unsubstituted heteroaryloxy of 2 to 30 carbon atoms, a substituted or unsubstituted alkylthio of 1 to 30 carbon atoms, a substituted or unsubstituted arylthio of 6 to 30 carbon atoms, a substituted or unsubstituted cycloalkylthio of 3 to 30 carbon atoms, a substituted or unsubstituted heteroarylthio of 2 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
L₁₁ and L₁₂, which are same or different, are each independently any one linker selected from a single bond, a substituted or unsubstituted arylene of 6 to 24 carbon atoms, a substituted or unsubstituted heteroarylene of 3 to 24 carbon atoms, and a substituted or unsubstituted aliphatic hydrocarbon ring-fused arylene of 8 to 24 carbon atoms,
Ar₁₁ and Ar₁₂, which are same or different, are each independently any one selected from a substituted or unsubstituted aryl of 6 to 30 carbon atoms, a substituted or unsubstituted heteroaryl of 3 to 30 carbon atoms, a substituted or unsubstituted cycloalkyl of 3 to 30 carbon atoms, a substituted or unsubstituted heterocyclic alkyl of 3 to 30 carbon atoms, and a substituted or unsubstituted, aliphatic hydrocarbon ring-fused aryl of 8 to 24 carbon atoms,
with the hydrogen atoms bonded to the carbon atoms within Chemical Formula 2 being substitutable with 0 to 60 deuterium atoms or tritium atoms,
wherein the term "substituted" in the expression "substituted or unsubstituted" used for the compound of Chemical Formula 2 means having at least one substituent selected from the group consisting of a deuterium atom, a tritium atom, a cyano, a halogen, a thiol, a hydroxy, a nitro, alkyl of 1 to 30 carbon atoms, halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, aromatic hydrocarbon ring-fused cycloalkyl of 7 to 30 carbon atoms, aromatic heterocyclic ring-fused cycloalkyl of 5 to 30 carbon atoms, aromatic hydrocarbon ring-fused heterocyclic alkyl of 6 to 30 carbon atoms, an aliphatic hydrocarbon ring-fused aryl of 7 to 30 carbon atoms, aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with one or more hydrogen atoms within the substituents being substitutable with deuterium atoms or tritium atoms.

In an embodiment, R₁₁ to R₁₈ within Chemical Formula 2, which are same or different, are each independently any one selected from a hydrogen atom or a deuterium atom.

In an embodiment, the compound represented by Chemical Formula 2 may contain at least one deuterium atom.
When the first host or the second host in the organic light-emitting diode of the present disclosure contains at least one of the anthracene compounds represented by Chemical Formula 2, Ar₁₂ in the anthracene compound represented by Chemical Formula 2 may be a substituent represented by the following Structural Formula 12: 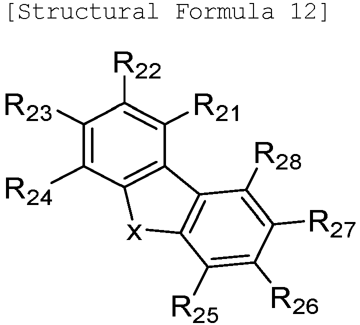 wherein,
X is O or S,
one of R₂₁ to R₂₄ is a single bond to L₁₂ in Chemical Formula 2, and
R₂₁ to R₂₈ exclusive of the single bond to L₁₂ are same or different and are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
wherein the term "substituted" in the expression "substituted or unsubstituted" used for the compound of Structural Formula 12 means having at least one substituent selected from the group consisting of a deuterium atom, a tritium atom, a cyano, a halogen, a thiol, a hydroxy, a nitro, alkyl of 1 to 30 carbon atoms, halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, aromatic hydrocarbon ring-fused cycloalkyl of 7 to 30 carbon atoms, aromatic heterocyclic ring-fused cycloalkyl of 5 to 30 carbon atoms, aromatic hydrocarbon ring-fused heterocyclic alkyl of 6 to 30 carbon atoms, an aliphatic hydrocarbon ring-fused aryl of 7 to 30 carbon atoms, aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, a thioxy of 1 to 30 carbon atoms, and a thionyl of 1 to 30 carbon atoms, with one or more hydrogen atoms within the substituents being substitutable with deuterium atoms or tritium atoms.

In an embodiment, R₂₁ to R₂₈ in Structural Formula 12 may each be independently any one selected from a hydrogen atom, a deuterium atom, a substituted or unsubstituted aryl of 6 to 30 carbon atoms, a substituted or unsubstituted heteroaryl of 3 to 30 carbon atoms, and a substituted or unsubstituted aliphatic hydrocarbon ring-fused aryl of 8 to 24 carbon atoms.

In a more preferred embodiment, R₂₁ to R₂₈ in Structural Formula 12, which are same or different, may each be independently any one selected from a hydrogen atom, a deuterium atom, and a substituted or unsubstituted aryl of 6 to 18 carbon atoms.

In an embodiment, X in Structural Formula 12 may be O.

In an embodiment, one of the first host and second host within the organic light-emitting diode according to the present disclosure may contain a compound represented by Chemical Formula 1 while the other may contain two types of the anthracene compounds represented by Chemical Formula 2. In this regard, the anthracene compounds represented by Chemical Formula 2 may be different anthracene compounds. For instance, the first host within the organic light-emitting diode according to the present disclosure may include the compound represented by Chemical Formula 1 and the second host may include two or more types of the anthracene compounds represented by Chemical Formula 2, and *vice versa.* The compound represented by Chemical Formula 2 may be anthracene compounds different from each other.

In an embodiment, when one of the first host and the second host within the organic light-emitting diode according to the present disclosure includes a compound represented by Chemical Formula 1 while the other includes two different types of the anthracene compounds represented by Chemical Formula 2, the Ar₁₂ radical may be a substituted or unsubstituted aryl of 6 to 30 carbon atoms in at least one of the anthracene compounds represented by Chemical Formula 2, and in addition, the Ar₁₂ radical may be a substituted or unsubstituted heteroaryl of 2 to 30 carbon atoms and preferably a substituted or unsubstituted heteroaryl of 3 to 18 carbon atoms in at least one of the additionally used anthracene compounds represented by Chemical Formula 2.

In a preferred embodiment, when one of the first host and the second host within the organic light-emitting diode according to the present disclosure includes a compound represented by Chemical Formula 1 while the other includes two different types of the anthracene compounds represented by Chemical Formula 2, the Ar₁₂ radical may be a substituted or unsubstituted aryl of 6 to 30 carbon atoms in at least one of the anthracene compounds represented by Chemical Formula 2, and in addition, the Ar₁₂ radical may be a substituent represented by Structural Formula 12.

In an embodiment, the first emission layer within the organic light-emitting diode according to the present disclosure may contain at least one of the compounds represented by Chemical Formula 1 as the first host while the second emission layer may contain at least one of the compounds represented by Chemical Formula 2 as the second host.

In an embodiment, when one of the first host and the second host within the organic light-emitting diode according to the present disclosure includes two types of the compounds represented by Chemical Formula 1 while the other includes an anthracene compound represented by Chemical Formula 2, the first emission layer may contain the compound represented by Chemical Formula 1 as the first host and a host compound different from the compound represented by Chemical Formula 1 at a ratio of 1:9 to 9:1 and more preferably at a ratio of 3:7 **to 7:3.**

In an embodiment, the first emission layer within the organic light-emitting diode according to the present disclosure may contain both a compound represented by Chemical Formula 1 and an additional host compound different from the compound represented by Chemical Formula 1 as a first host. In this case, the first host used in the first emission layer may include the compound represented by Chemical Formula 1 and the compound different from the compound represented by Chemical Formula 1 at a ratio of 1:9 to 9:1 and more preferably at a ratio of 3:7 to 7:3.

In cases where the first host is comprised of two or more compounds, these compounds may be mixed and deposited, co-deposited, or laminated for use. That is, the first host according to the present disclosure may comprise, in addition to one compound represented by Chemical Formula 1, one or more additional compounds that differ therefrom, such that two or more host compounds may be mixed and deposited, co-deposited, or laminated. In the case of lamination, the compound different from that represented by Chemical Formula 1 may be laminated above or below the layer comprising the compound of Chemical Formula 1.

Here, when the compound different from that of Chemical Formula 1 is deposited in a mixed state, it means that the compounds are mixed in a single deposition source and deposited by sublimation or vaporization. In the case of co-deposition, it means that two or more hosts are respectively sublimated or vaporized from multiple deposition sources and simultaneously deposited.

That is, the emission layer may be formed by depositing a thin film using multiple host materials respectively from different deposition sources (co-deposition), or by depositing a pre-mixed host blend, or by forming the emission layer via a lamination method.

Here, the host compound different from that represented by Chemical Formula 1 may be a compound with a structure different from that of Chemical Formula 1, or a compound that, while still falling within the structure category of Chemical Formula 1, is distinct from a previously used compound (i.e., not identical to the compound already used).

Examples of the compound having a structure different from that of Chemical Formula 1 for use in the first emission layer include conventionally known anthracene-based compounds, benzanthracene-based compounds, phenanthrene-based compounds, pyrene-based compounds, fluorene-based compounds, spiro-fluorene-based compounds, chrysene-based compounds, carbazole-based compounds, and biphenyl-based compounds. Among these, the anthracene-based compounds refer to compounds that contain at least one, preferably 1 to 3 anthracene groups within the molecule. The remaining types, that is, benzanthracene-based compounds, phenanthrene-based compounds, pyrene-based compounds, fluorene-based compounds, spiro-fluorene-based compounds, chrysene-based compounds, carbazole-based compounds, and biphenyl-based compounds likewise refer to compounds containing at least one, preferably 1 to 3 groups of the respective type within the molecule.

In an embodiment, when one of the first or second hosts in the organic light-emitting diode according to the present disclosure includes the compound represented by Chemical Formula 1, and the other includes an anthracene compound represented by Chemical Formula 2, the other host (i.e., the one not including Chemical Formula 1) may include two or more different anthracene compounds represented by Chemical Formula 2 preferably at a ratio of 1:9 to 9:1 and more preferably at a ratio of 3:7 to 7:3. Preferably, the second emission layer may contain, as a second host, two different types of the anthracene compounds represented by Chemical Formula 2 at a ratio of 1:9 to 9:1 and preferably at a ratio of 3:7 to 7:3.

In an embodiment, when the first emission layer of the organic light-emitting diode according to the present disclosure may include one or more compounds represented by Chemical Formula 1 as the first host and the second emission layer may include one or more compounds represented by Chemical Formula 2 as the second host, the second emission layer may further contain, in addition to the compound represented by Chemical Formula 2, a host compound different from that of Chemical Formula 2. In this regard, the second host used in the second emission layer may include a compound represented by Chemical Formula 2 and a host compound different from the compound represented by Chemical Formula 2 at a ratio of 1:9 to 9:1 and more preferably at a ratio of 3:7 to 7:3.

When the second host comprises two or more compounds, they may be mixed in a single deposition source and deposited by sublimation or vaporization, or each may be sublimated or vaporized from separate deposition sources and co-deposited onto the second emission layer.

Here, the host compound different from that of Chemical Formula 2 may be a compound having a structure different from that of Chemical Formula 2, or a compound that, although classified within the same structural group as Chemical Formula 2, is distinct from any compound already used.

Further, compounds having a structure different from Chemical Formula 2 for use in the second emission layer may include conventionally known anthracene-based compounds, benzanthracene-based compounds, phenanthrene-based compounds, pyrene-based compounds, fluorene-based compounds, spiro-fluorene-based compounds, chrysene-based compounds, carbazole-based compounds, and biphenyl-based compounds, the descriptions of which are the same as previously stated.

In an embodiment, the first host in the first emission layer of the OLED according to the present disclosure, may consist solely of a compound represented by Chemical Formula 1, and the second host in the second emission layer may include a compound represented by Chemical Formula 2 and a compound different therefrom at a ratio of 1:9 to 9:1 and more preferably 3:7 to 7:3.

In an embodiment, the first host in the first emission layer of the OLED according to the present disclosure may include a compound represented by Chemical Formula 1 and a compound different therefrom at a ratio of 1:9 to 9:1 and more preferably 3:7 to 7:3, and the second host in the second emission layer may consist solely of a compound represented by Chemical Formula 2.

In yet another embodiment, the first host in the first emission layer may include the compound represented by Chemical Formula 1 and a compound different therefrom at a ratio of 1:9 to 9:1 and more preferably 3:7 to 7:3, and the second host in the second emission layer may include the compound represented by Chemical Formula 2 and a compound different therefrom at a ratio of 1:9 to 9:1 and more preferably 3:7 to 7:3.

In an embodiment, concrete examples of the compound represented by Chemical Formula 2 include, but are not limited to:

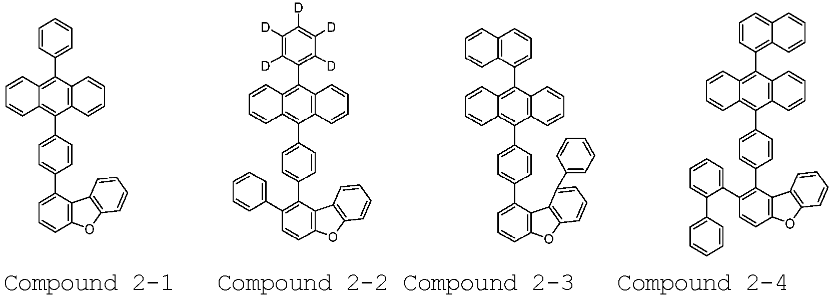

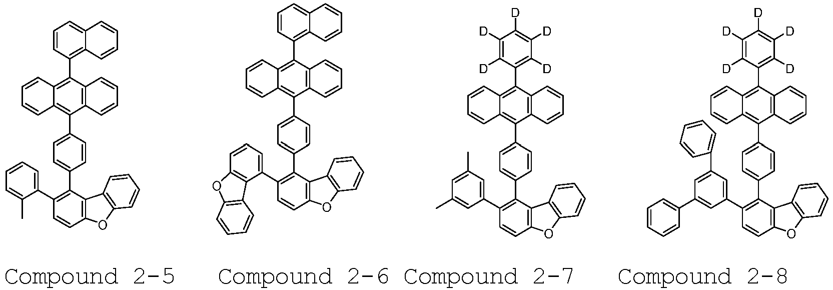

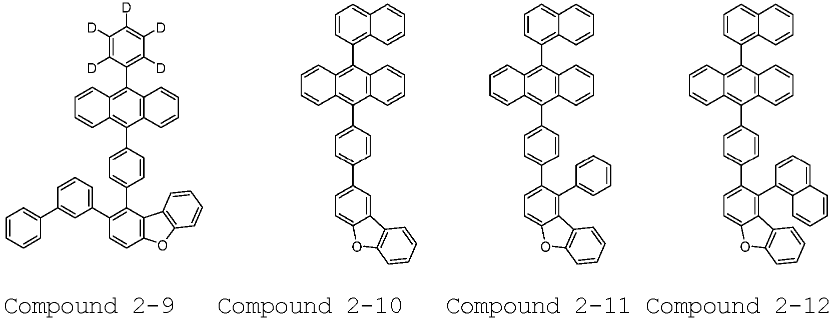

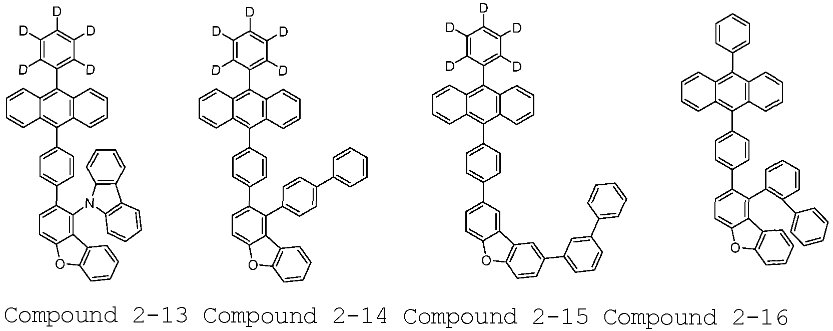

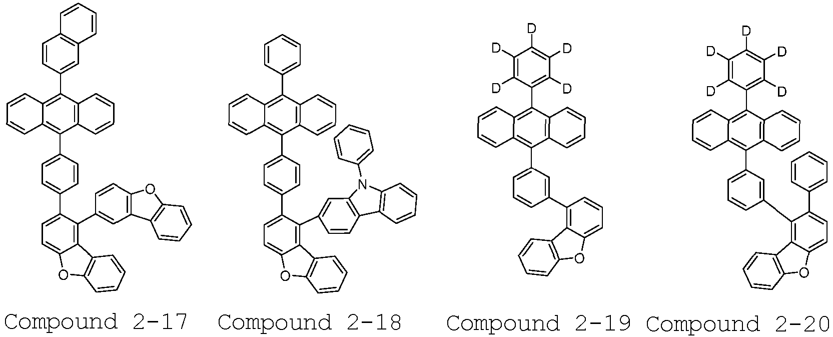

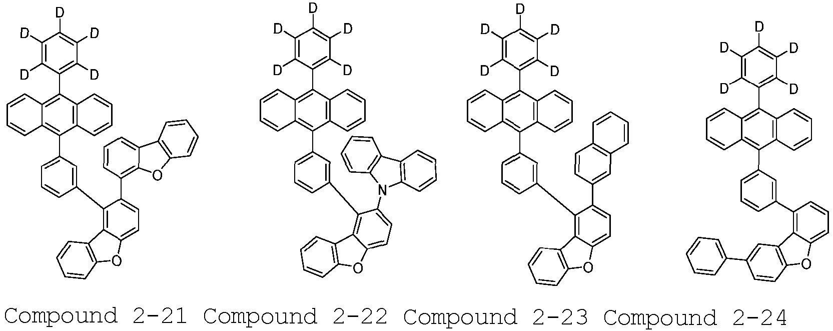

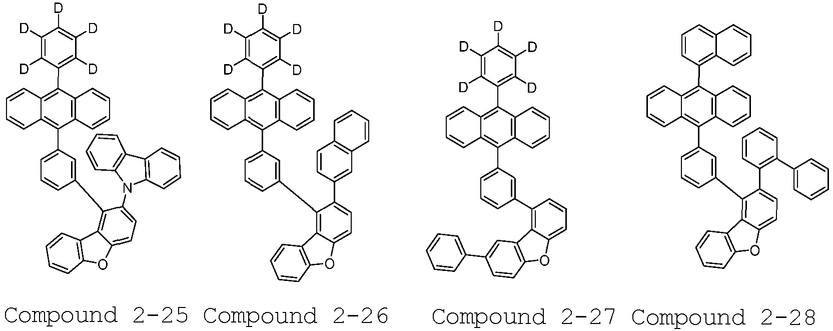

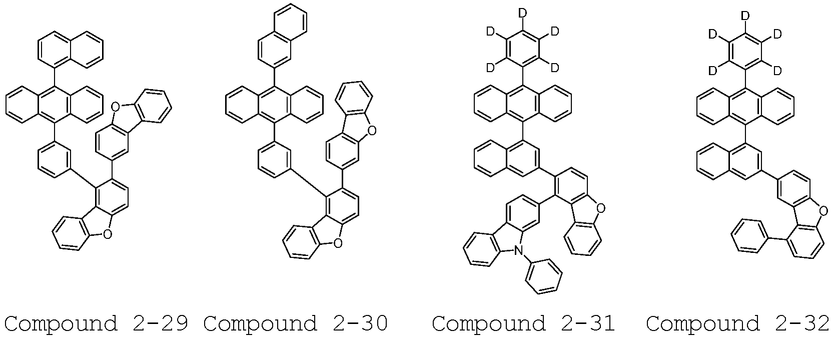

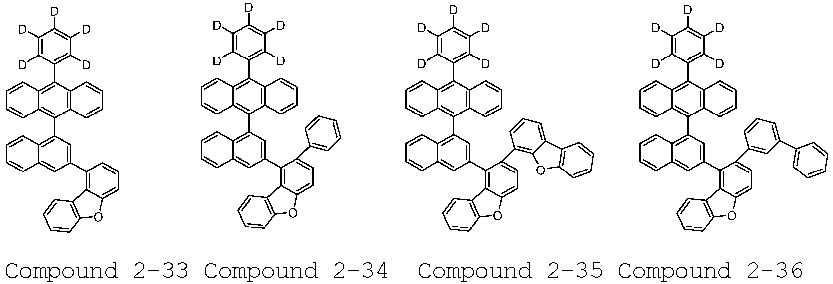

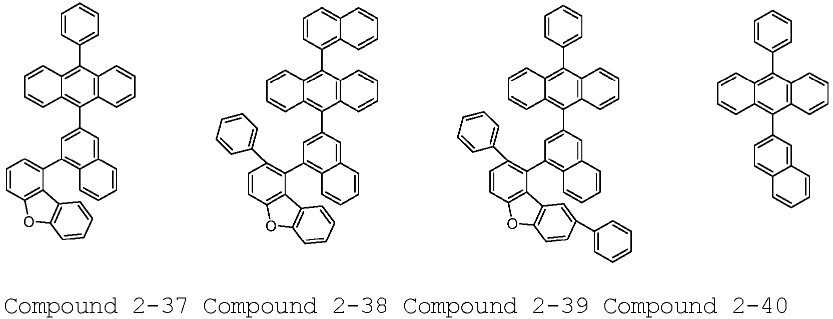

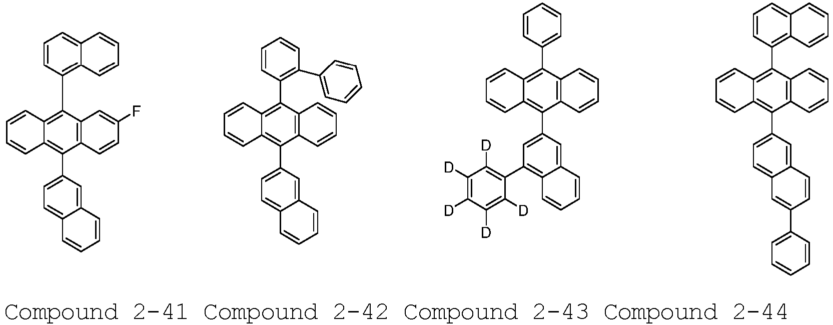

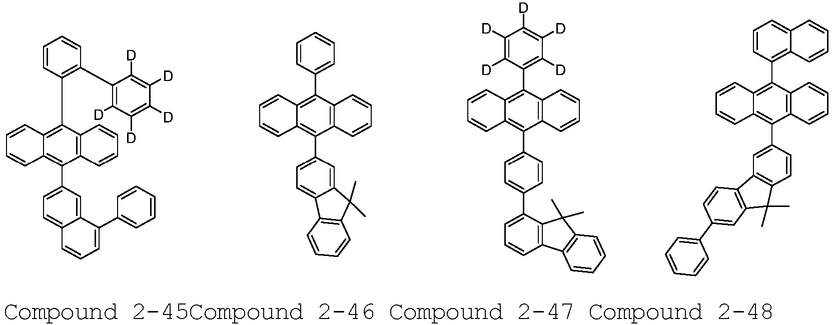

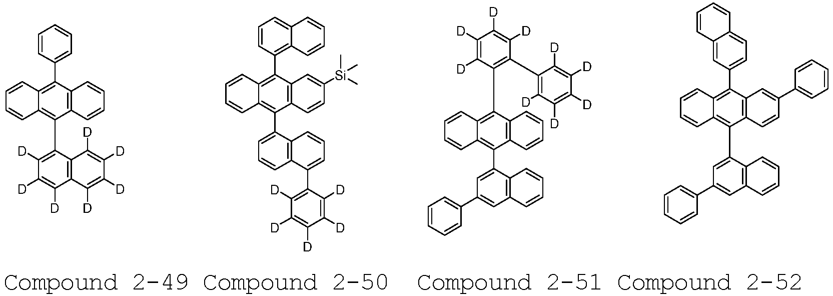

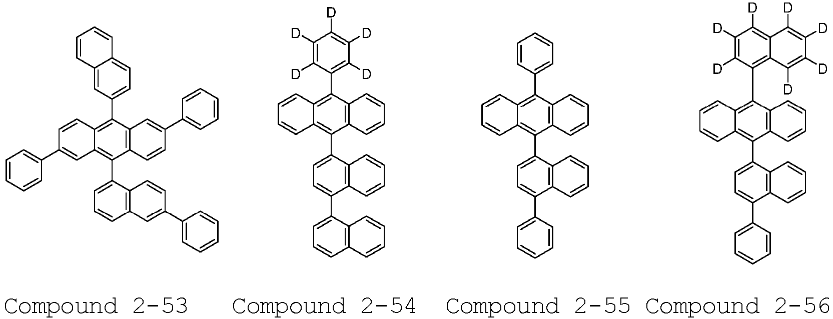

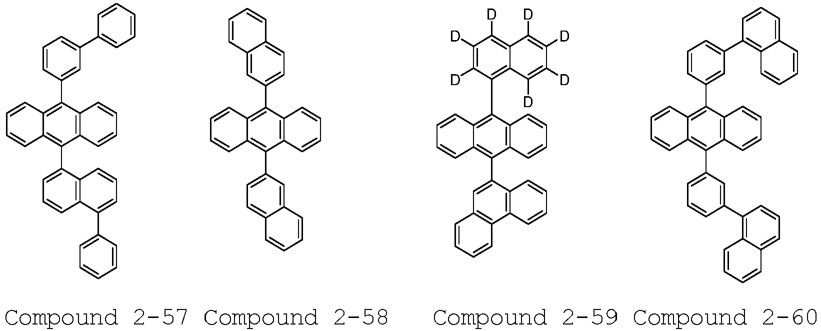

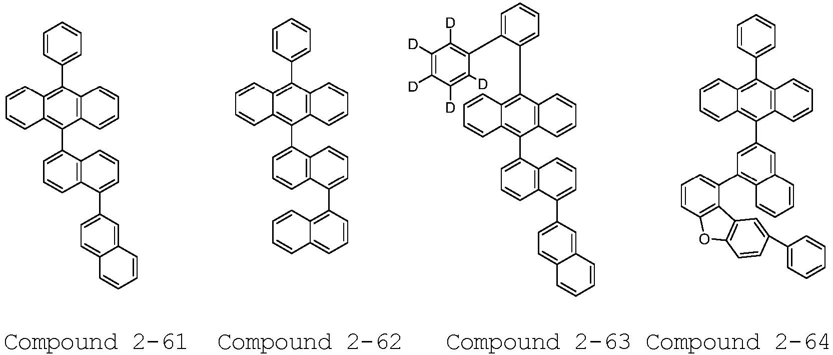

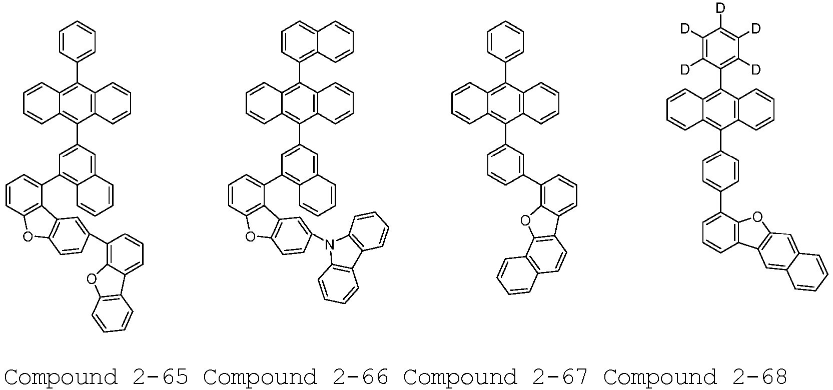

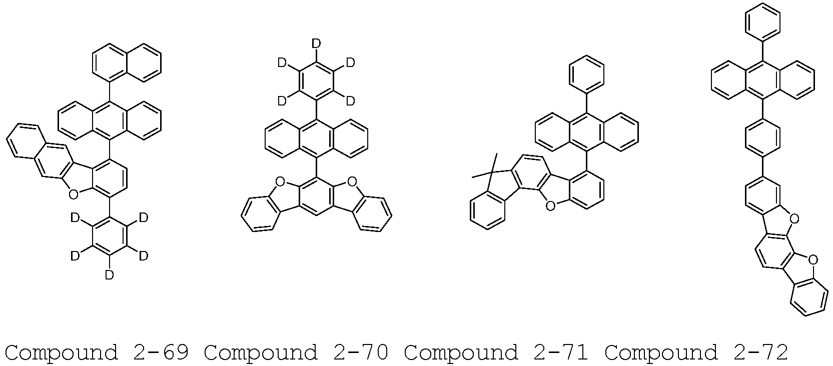

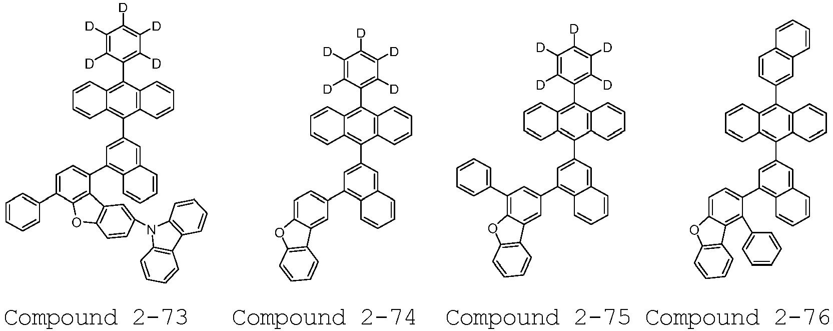

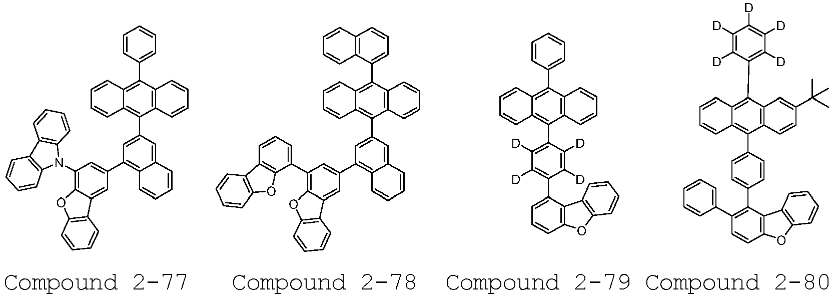

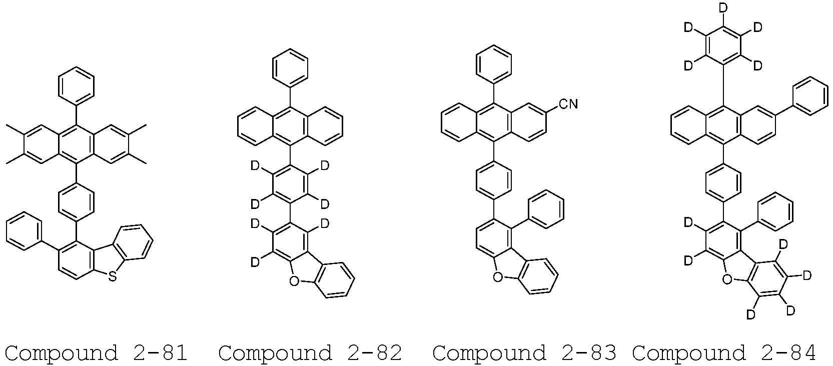

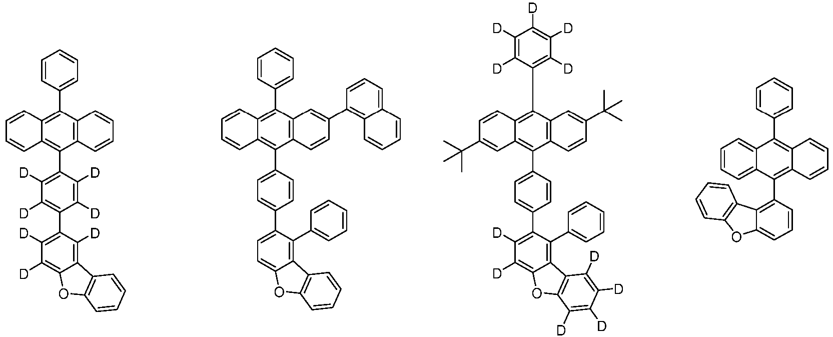

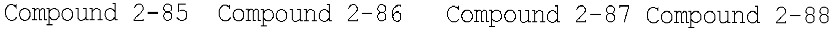

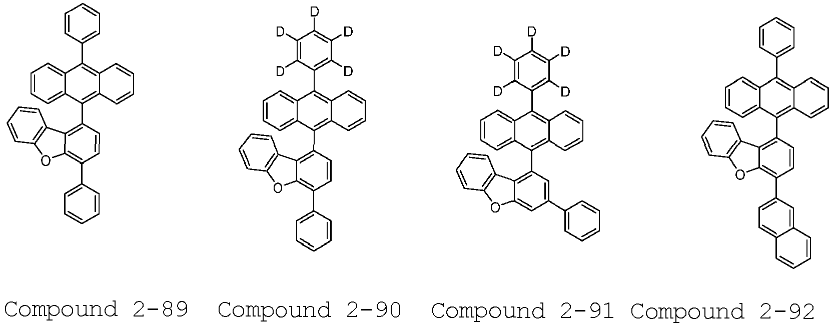

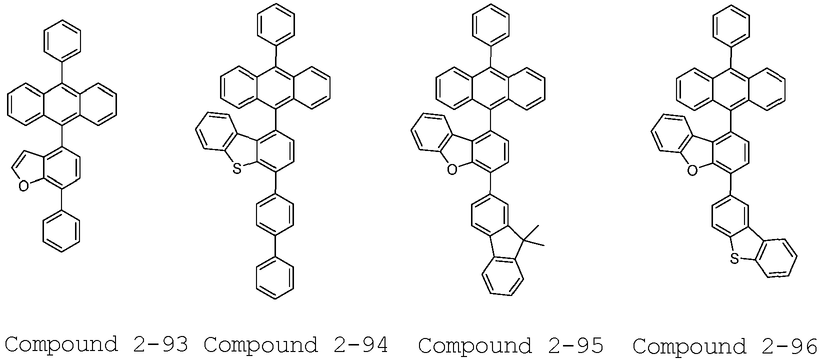

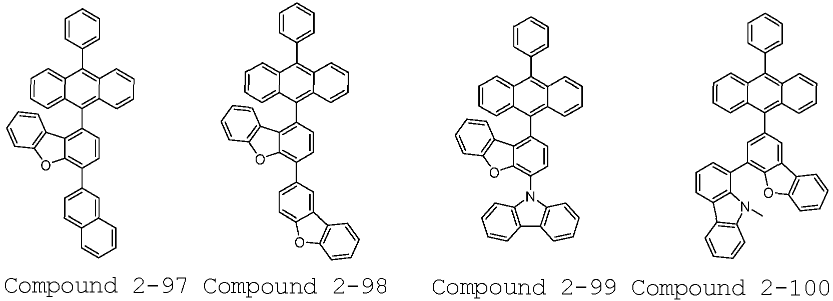

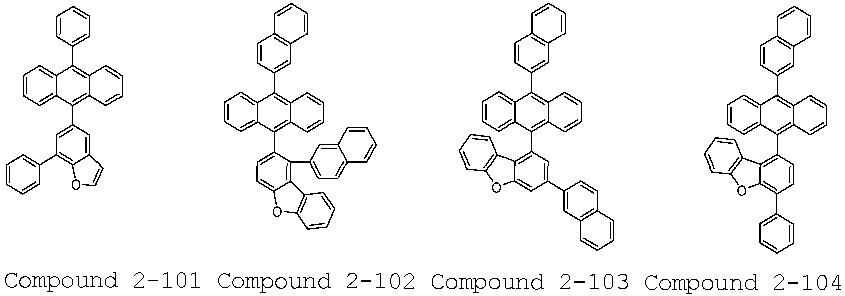

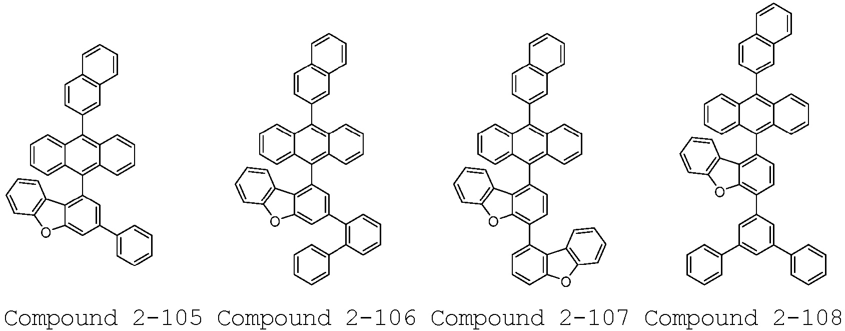

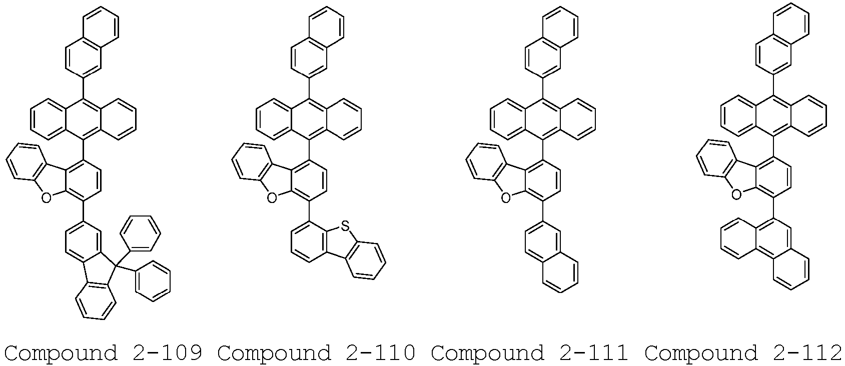

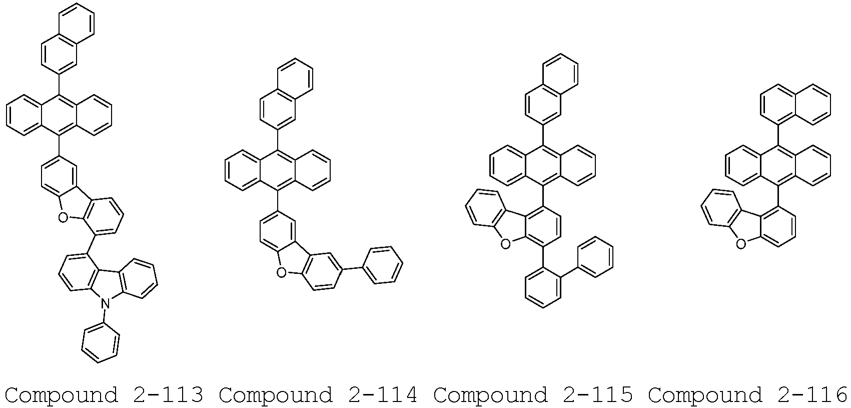

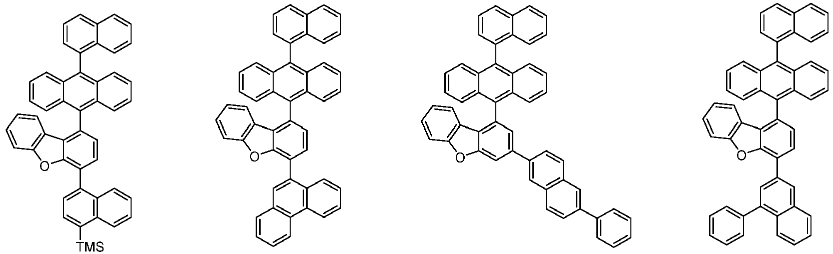

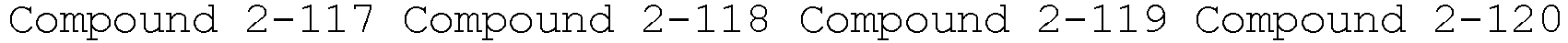

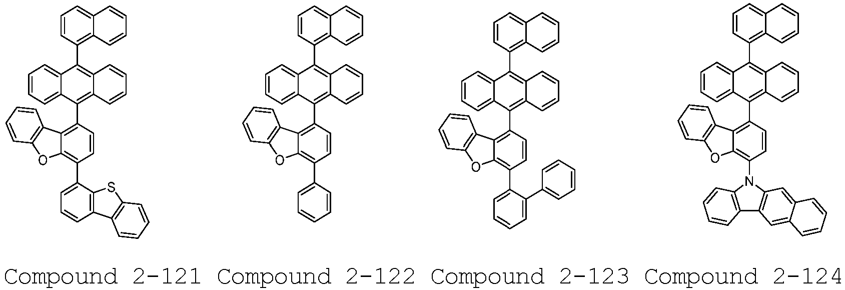

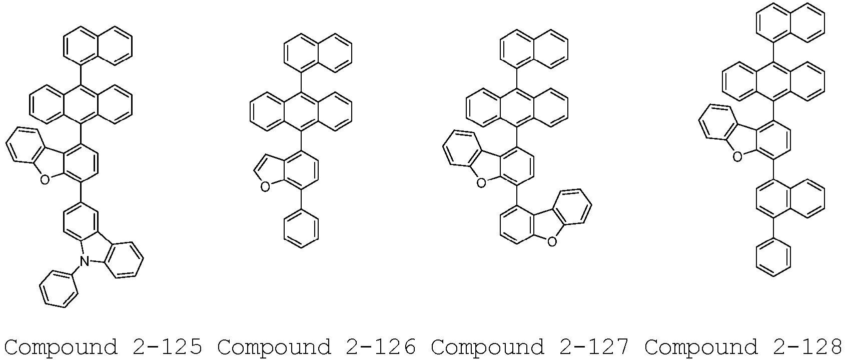

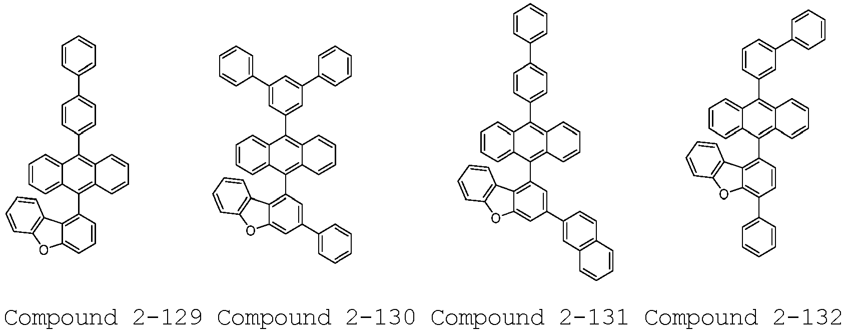

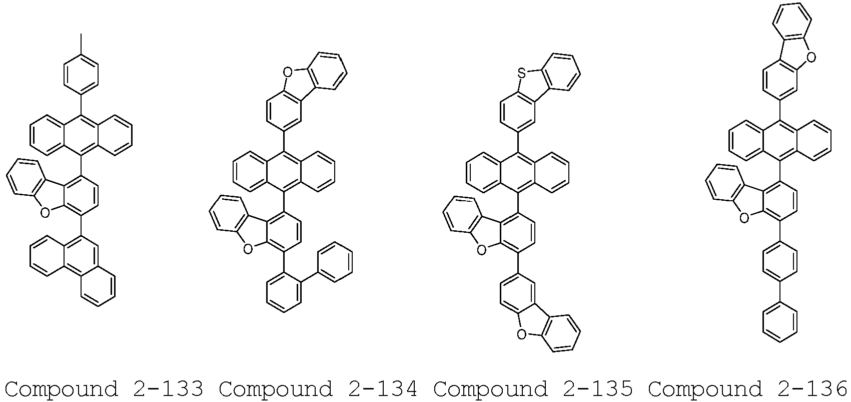

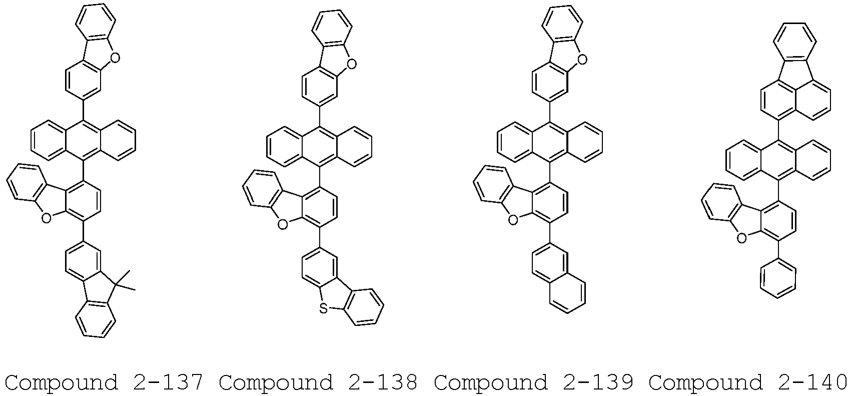

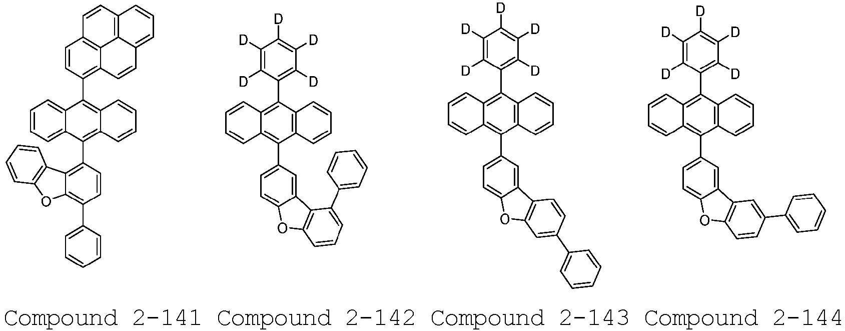

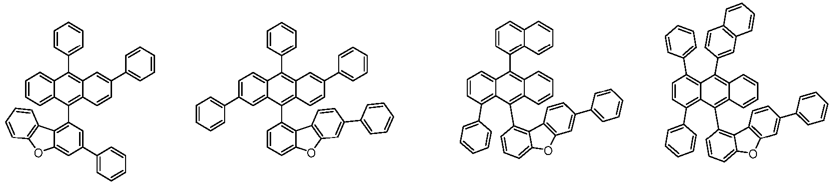

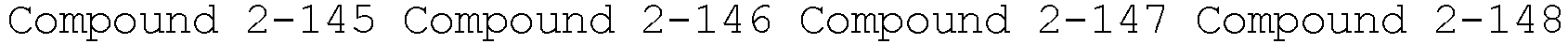

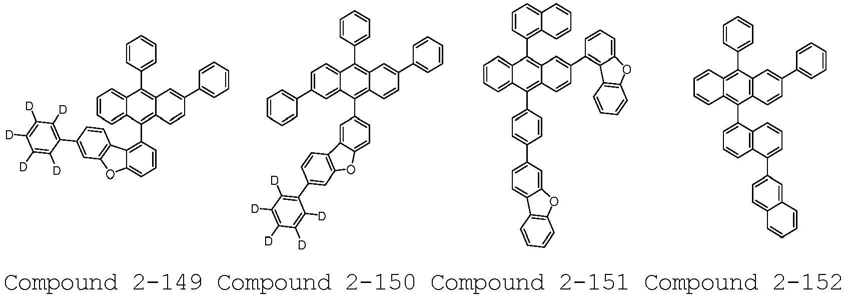

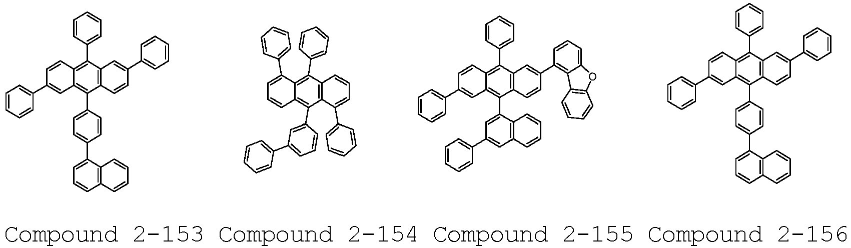

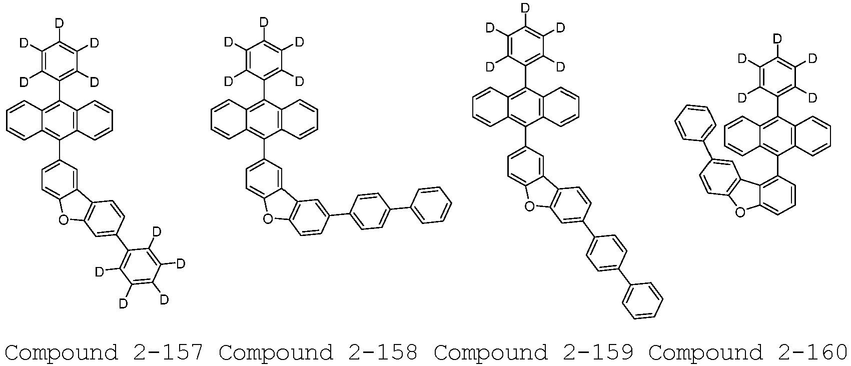

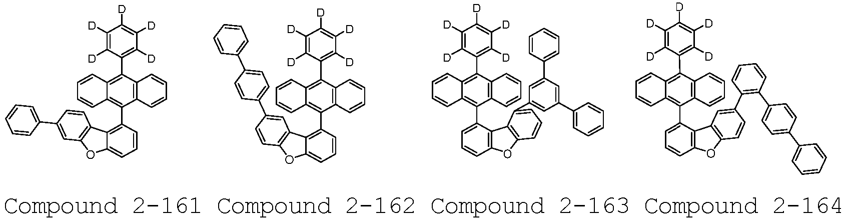

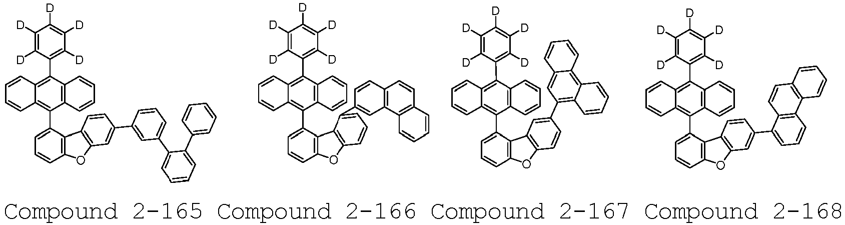

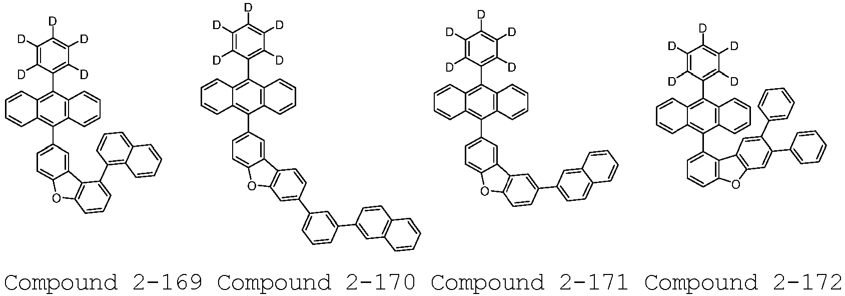

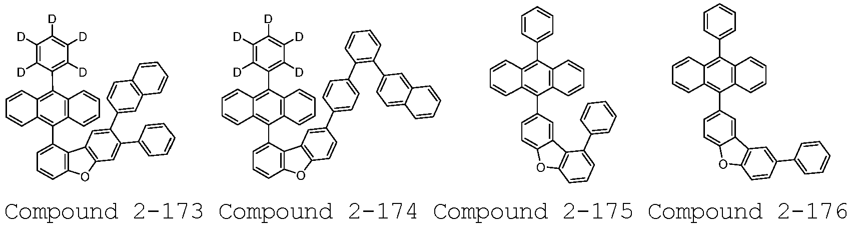

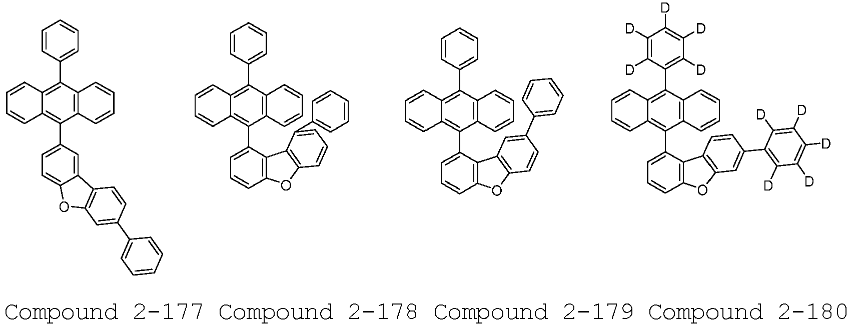

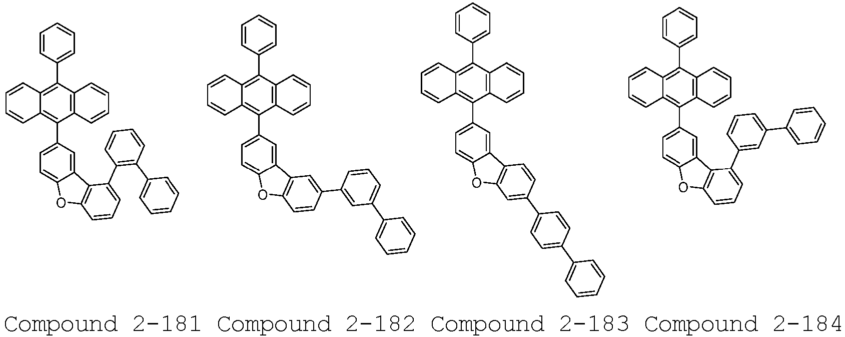

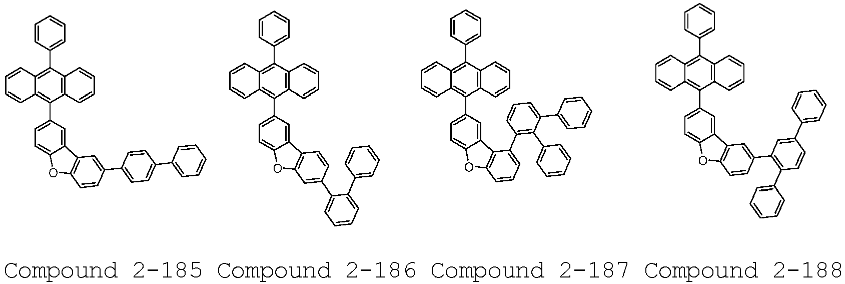

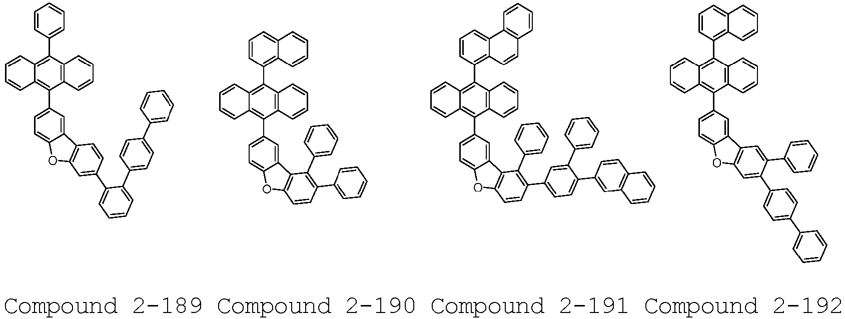

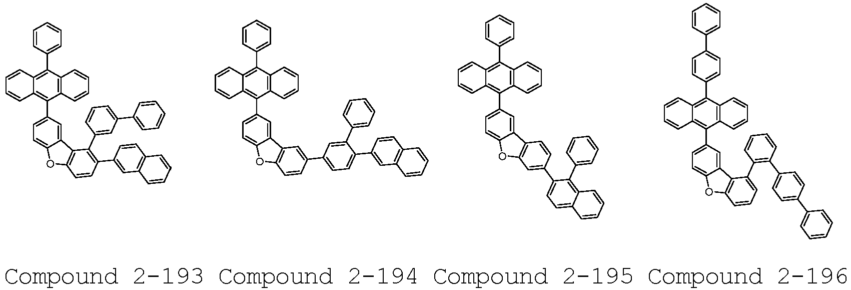

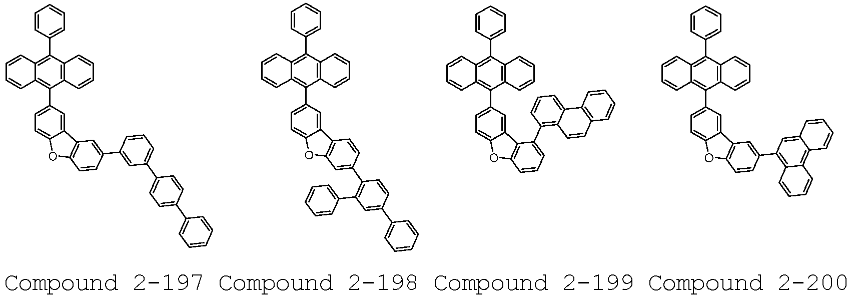

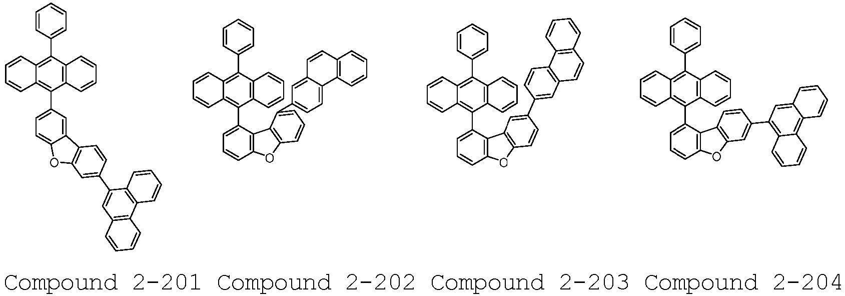

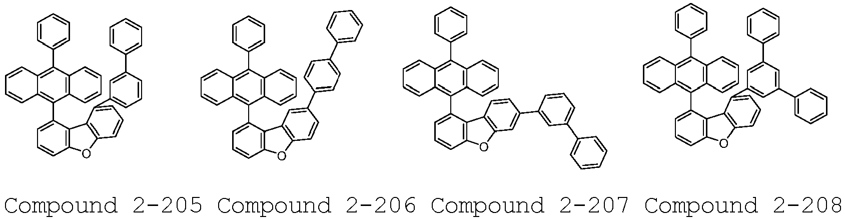

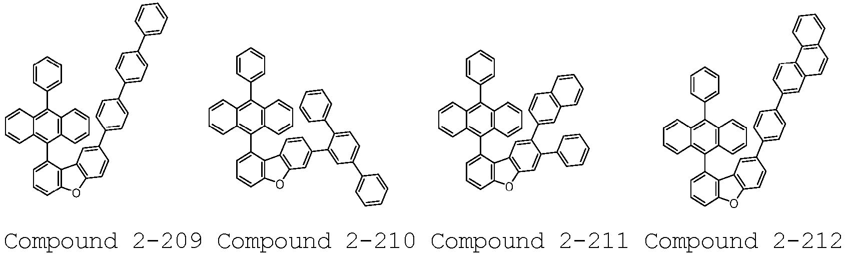

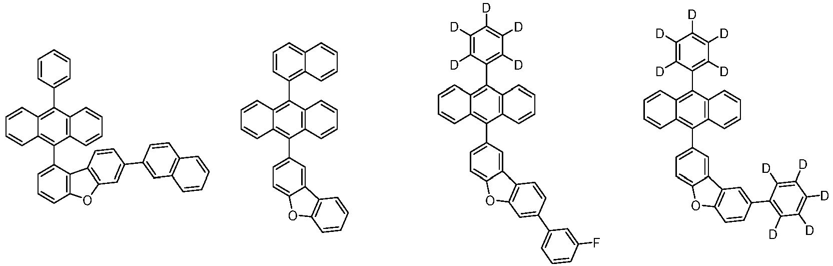

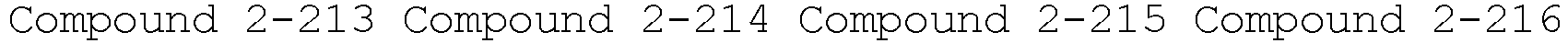

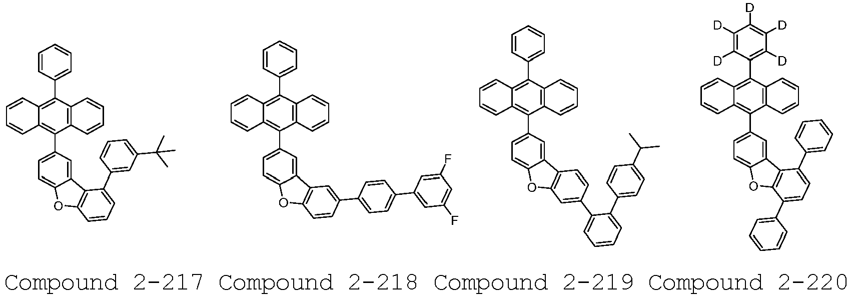

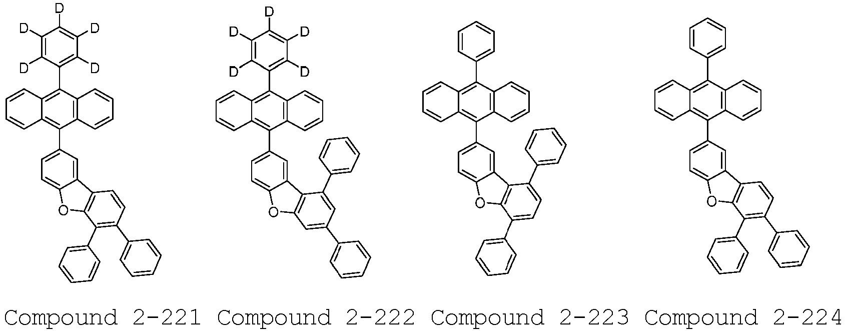

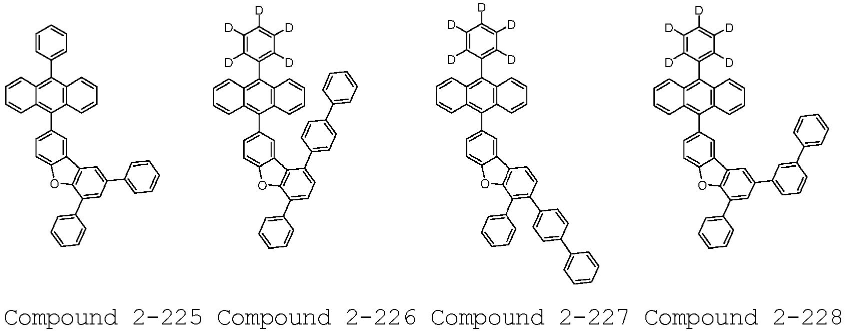

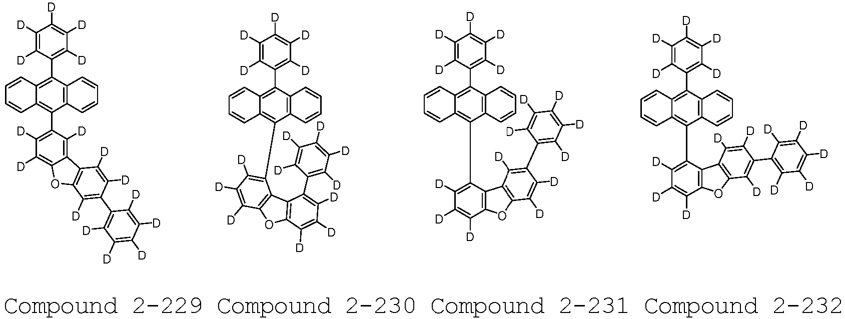

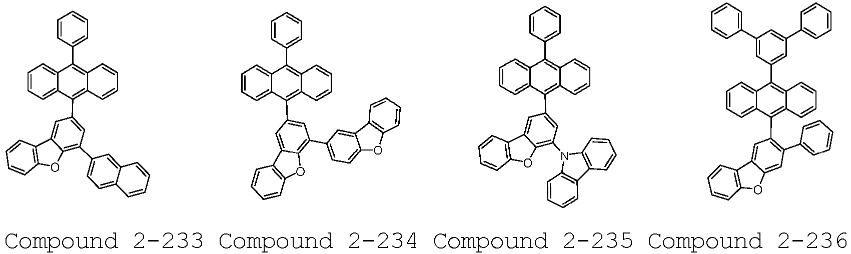

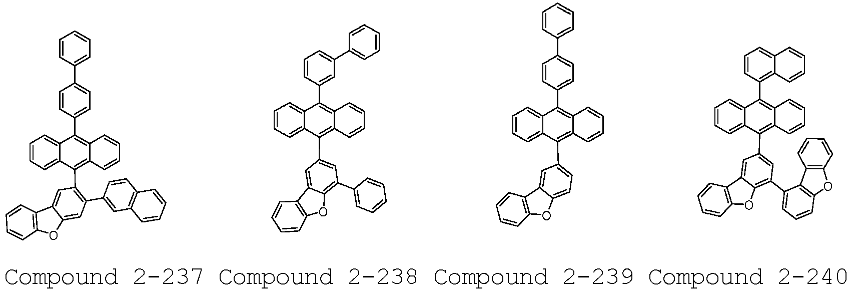

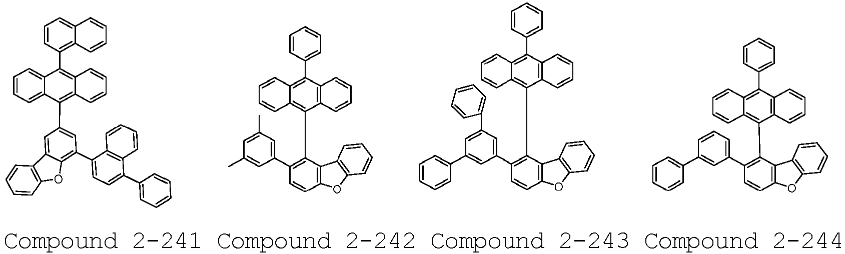

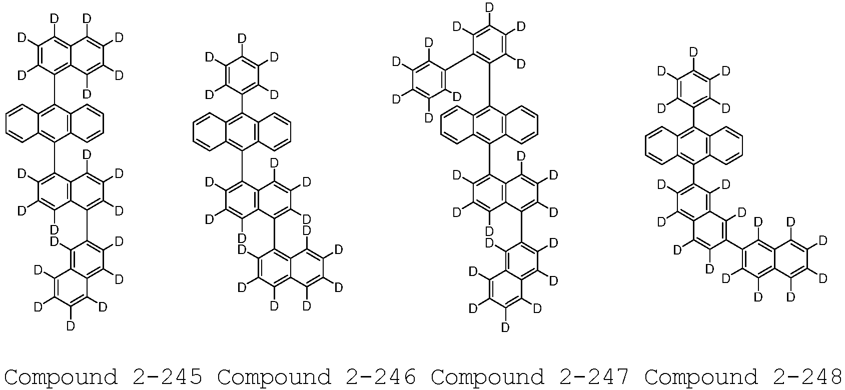

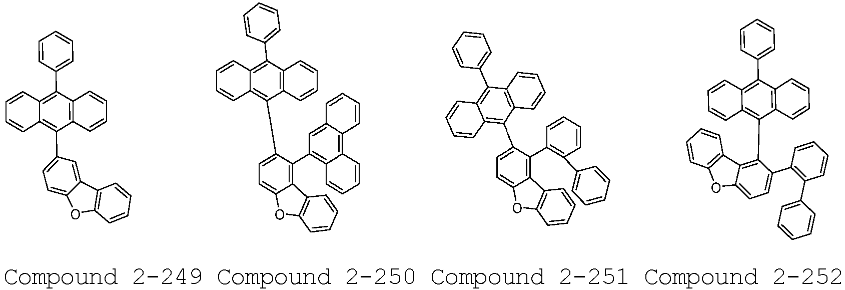

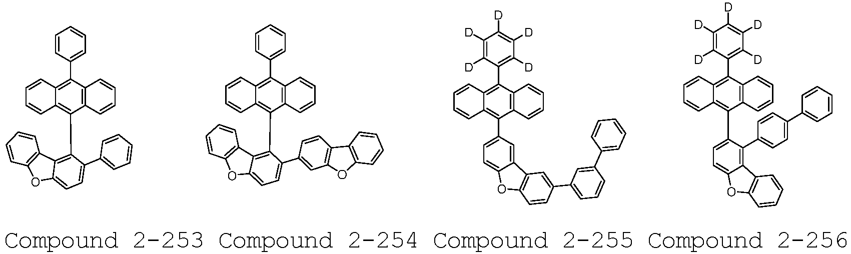

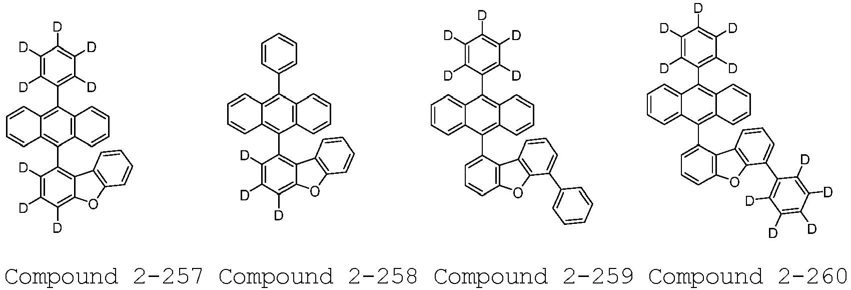

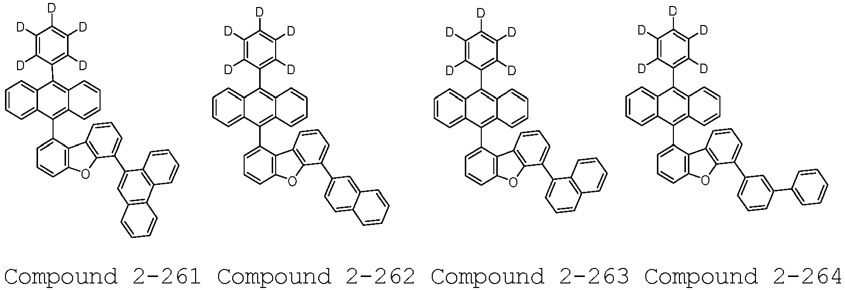

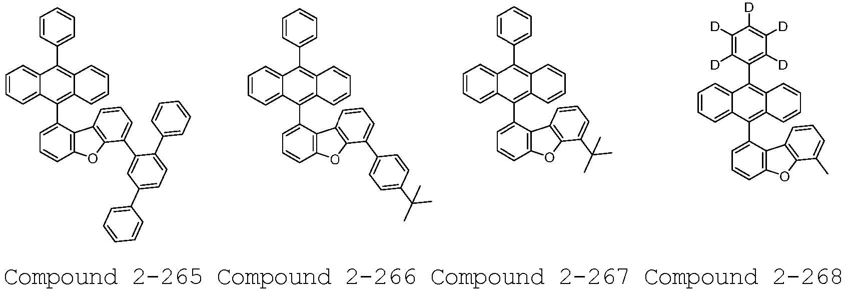

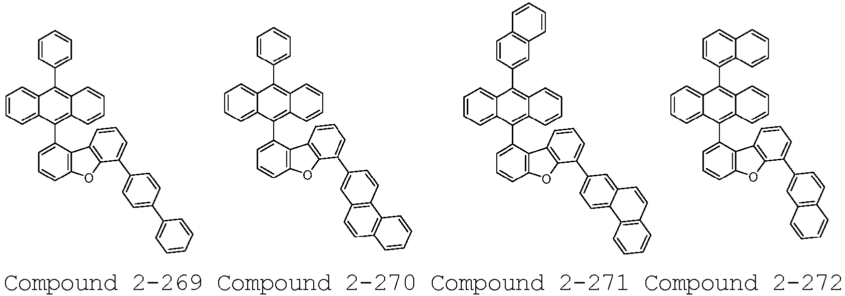

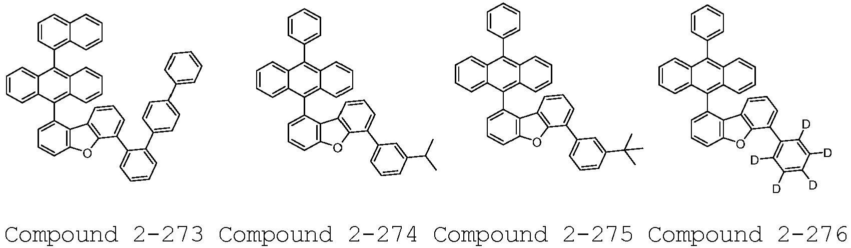

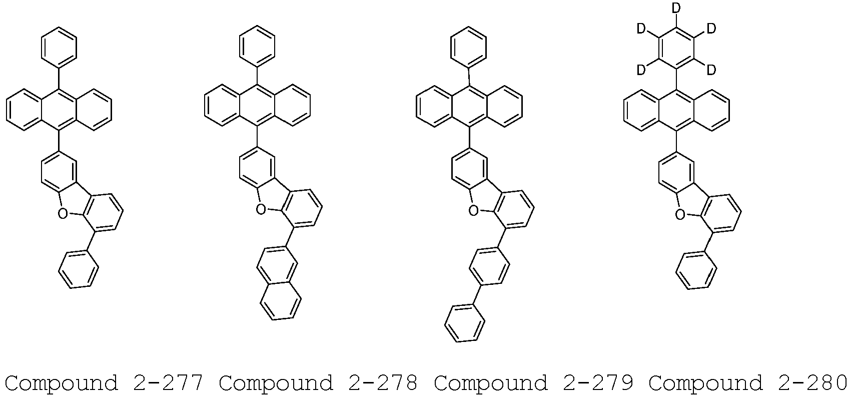

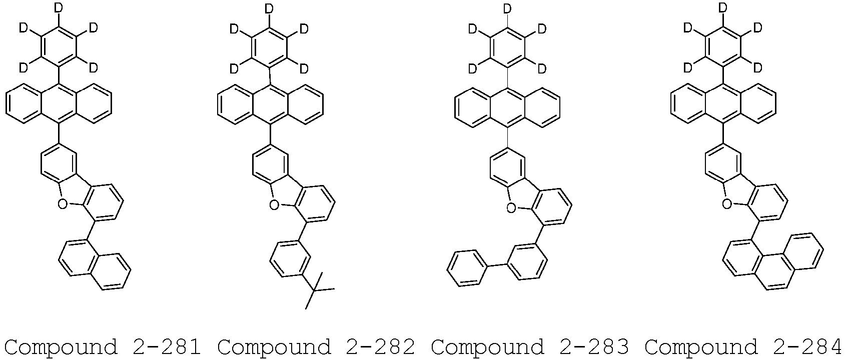

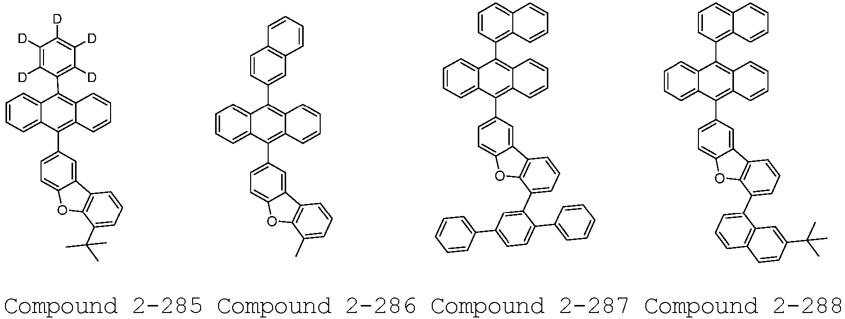

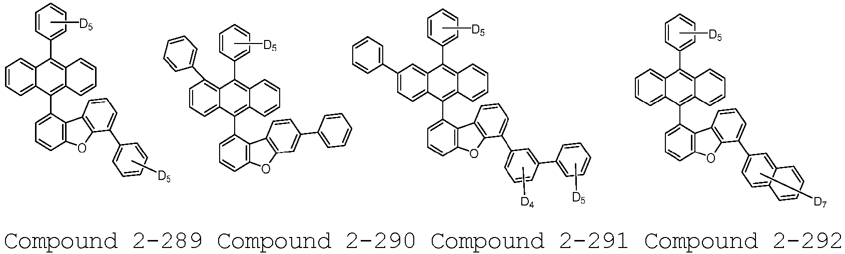

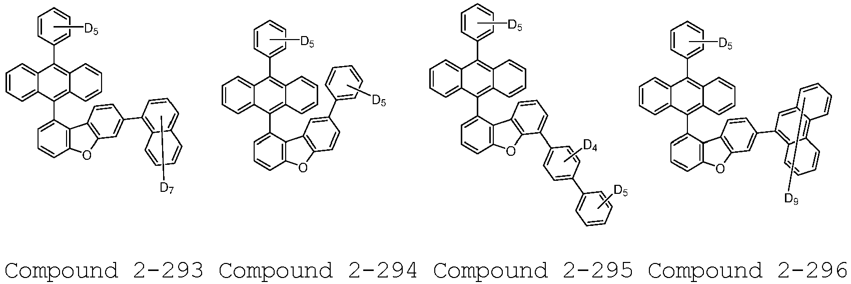

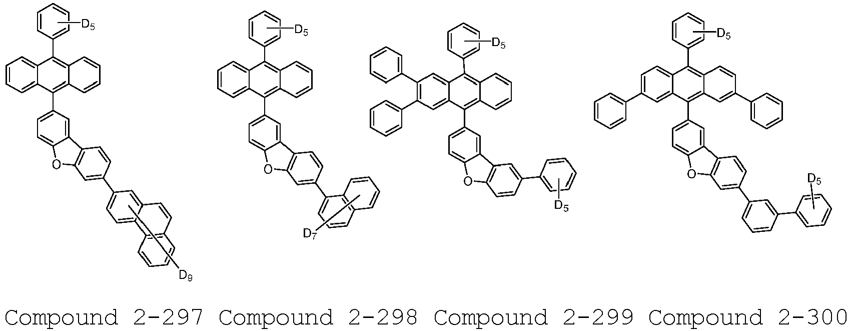

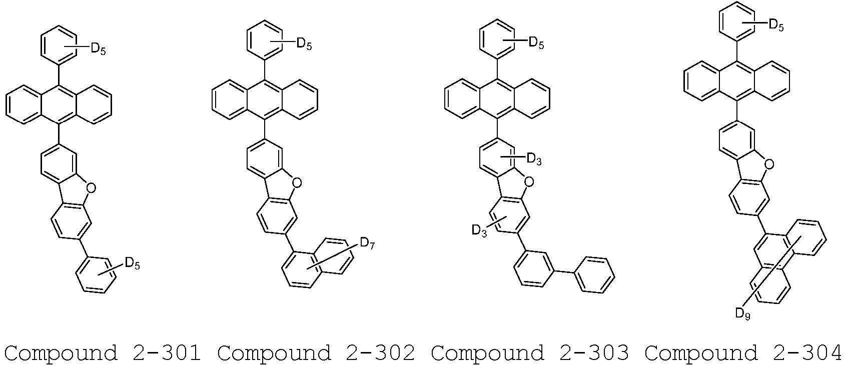

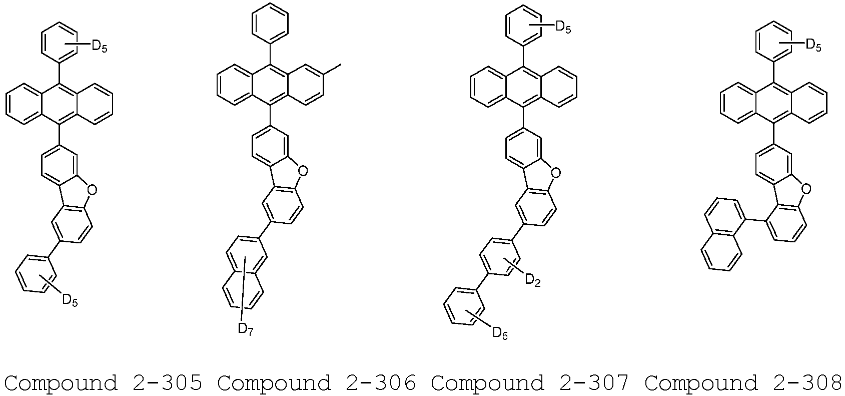

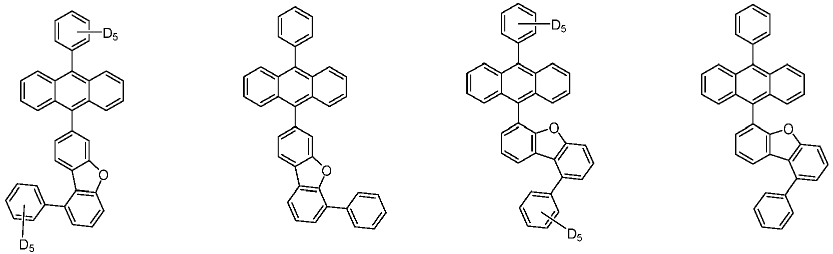

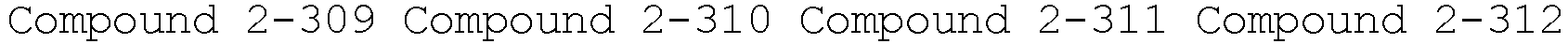

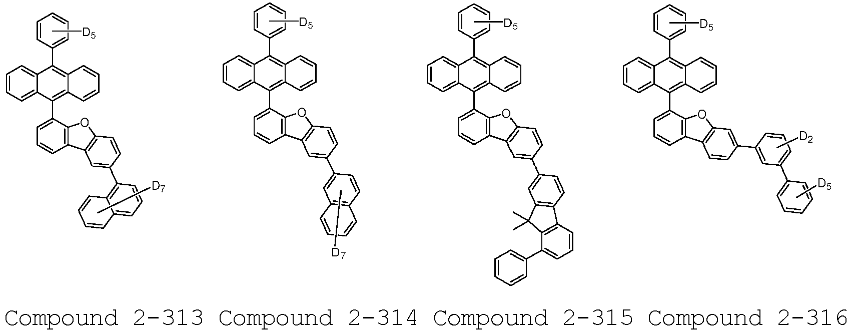

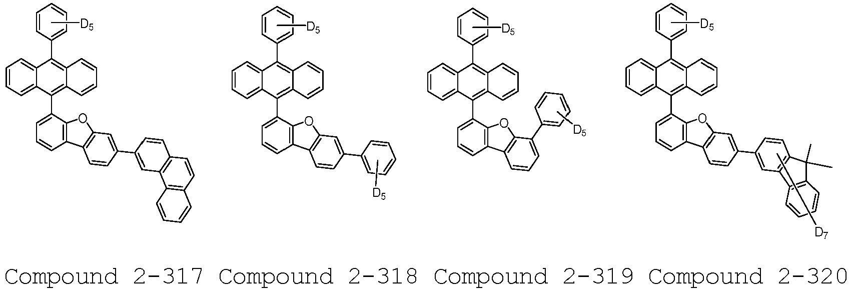

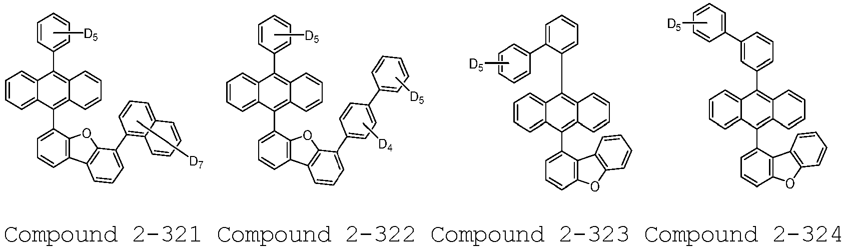

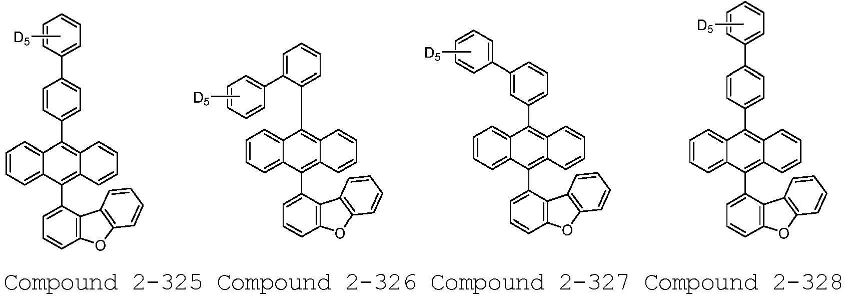

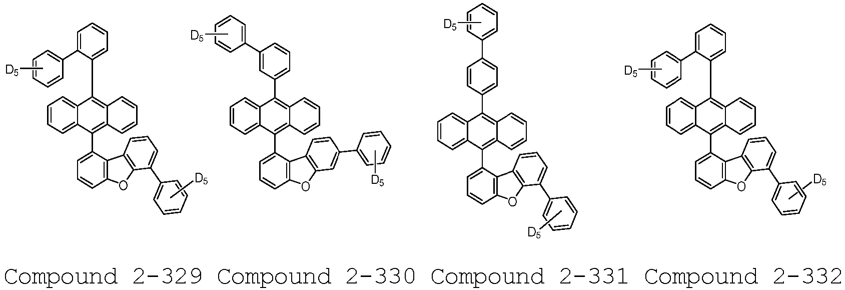

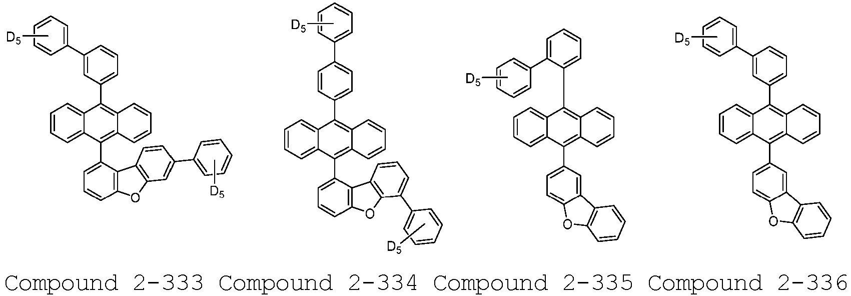

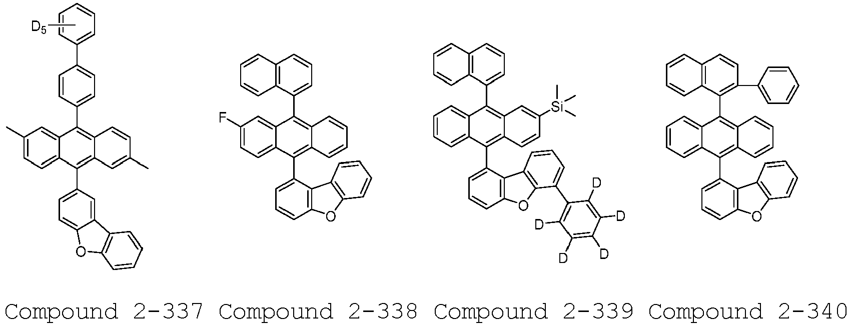

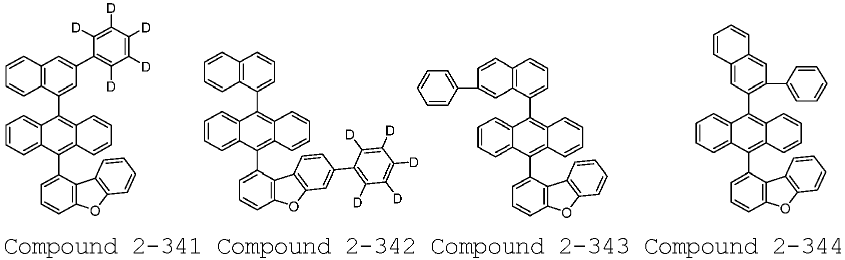

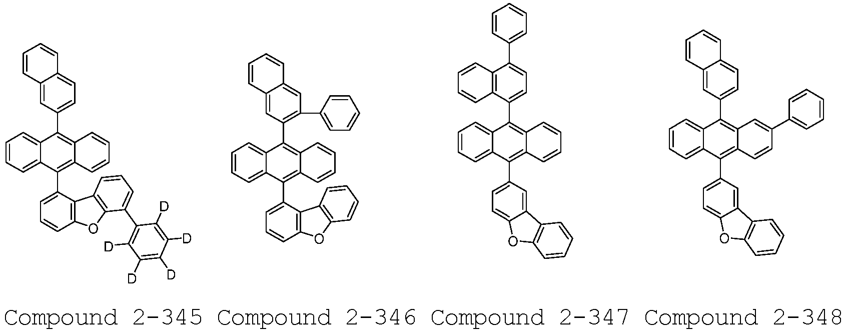

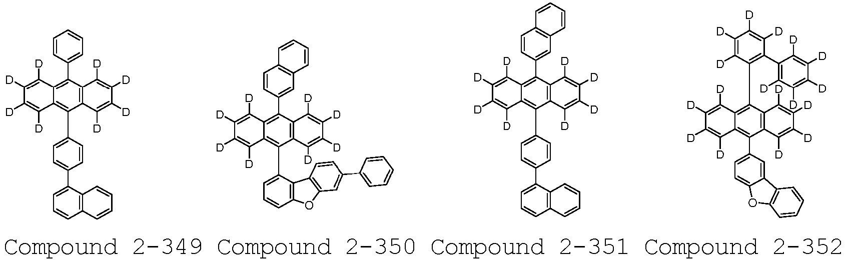

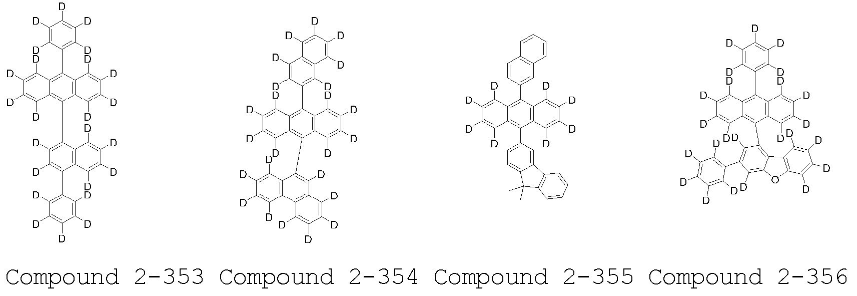

In an embodiment, the organic layer in the organic light-emitting diode of the present disclosure may further include at least one layer selected from a hole injection layer, a hole transport layer, a functional layer capable of both hole injection and hole transport, an electron blocking layer, a hole blocking layer, an electron transport layer, and an electron injection layer between the first electrode and the second electrode.

In an embodiment, the organic light-emitting diode according to the present disclosure may include at least one of a hole transport layer and a hole injection layer between the first electrode and the emission layer, and may include at least one of an electron transport layer and an electron injection layer between the emission layer and the second electrode.

In an embodiment of the organic light-emitting diode according to the present disclosure, the first emission layer may include a first host and a first dopant and the second emission layer may include a second host and a second dopant. In this regard, at least one of the first dopant in the first emission layer and the second dopant in the second emission layer may contain at least one polycyclic compound represented by the following Chemical Formula 3. Preferably, the first dopant in the first emission layer and the second dopant in the second emission layer, which may be same or different, may both contain at least one polycyclic compound represented by Chemical Formula 3: 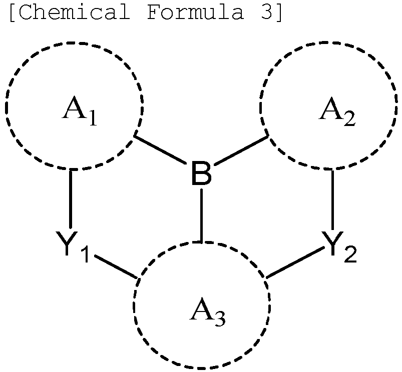 wherein,
Y₁ and Y₂, which are same or different, are each independently any one selected from among O, S, NR₃₁, CR₃₂R₃₃, SiR₃₄R₃₅, and GeR₃₆R₃₇,
A₁ to A₃, which are same or different, are each independently any one selected from a substituted or unsubstituted aromatic hydrocarbon ring of 6 to 50 carbon atoms, a substituted or unsubstituted aliphatic hydrocarbon ring of 5 to 50 carbon atoms, a substituted or unsubstituted, aliphatic hydrocarbon ring-fused aromatic hydrocarbon ring of 8 to 50 carbon atoms, a substituted or unsubstituted heteroaromatic ring of 2 to 50 carbon atoms, and a substituted or unsubstituted, aliphatic hydrocarbon ring-fused heteroaromatic ring of 5 to 50 carbon atoms,
R₃₁ to R₃₇, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted alkoxy of 1 to 30 carbon atoms, a substituted or unsubstituted aryloxy of 6 to 30 carbon atoms, a substituted or unsubstituted cycloalkyloxy of 3 to 30 carbon atoms, a substituted or unsubstituted heteroaryloxy of 2 to 30 carbon atoms, a substituted or unsubstituted alkylthio of 1 to 30 carbon atoms, a substituted or unsubstituted arylthio of 6 to 30 carbon atoms, a substituted or unsubstituted cycloalkylthio of 3 to 30 carbon atoms, a substituted or unsubstituted heteroarylthio of 2 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
R₃₁ to R₃₇ may be connected to the A₁ to A₃ ring moieties to additionally form an aliphatic or aromatic mono-or polycyclic ring, and
linkage may be made between R₃₂ and R₃₃, between R₃₄ and R35, and between R₃₆ and R₃₇ to additionally form respective mono- or polycyclic aliphatic or aromatic rings,
wherein the term "substituted" in the expression "substituted or unsubstituted" used for the compound of Chemical Formula 3 means having at least one substituent selected from the group consisting of a deuterium atom, a tritium atom, a cyano, a halogen, a thiol, a hydroxy, a nitro, alkyl of 1 to 30 carbon atoms, halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, aromatic hydrocarbon ring-fused cycloalkyl of 7 to 30 carbon atoms, a heteroaromatic ring-fused cycloalkyl of 5 to 30 carbon atoms, an aromatic hydrocarbon ring-fused heterocyclic alkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, a substituted or unsubstituted, heteroaliphatic ring-fused aryl of 6 to 30 carbon atoms, a substituted or unsubstituted, heteroaliphatic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthionyl of 6 to 24 carbon atoms, with at least one hydrogen atom on the substituents being substitutable with a deuterium or tritium atom.

In a more preferred embodiment of the present disclosure, A₁ to A₃ may be the same or different, and each may independently be a substituted or unsubstituted aromatic hydrocarbon ring of 6 to 18 carbon atoms, or a substituted or unsubstituted, aliphatic hydrocarbon ring-fused aromatic hydrocarbon ring of 8 to 24 carbon atoms.

In an embodiment, the first dopant in the first emission layer and the second dopant in the second emission layer may both be the same compound and contain at least one polycyclic compound represented by Chemical Formula 3.

In addition, when the first dopant or second dopant within the organic light-emitting diode according to the present disclosure contains one or more types of the polycyclic compound represented by Chemical Formula 3, the polycyclic compound represented by Chemical Formula 3 may be a polycyclic compound represented by the following Chemical Formula 3-1 or 3-2. Preferably, the first dopant in the first emission layer and the second dopant in the second emission layer are same or different and may each be a polycyclic ring compound represented by Chemical Formula 3-1 or 3-2: 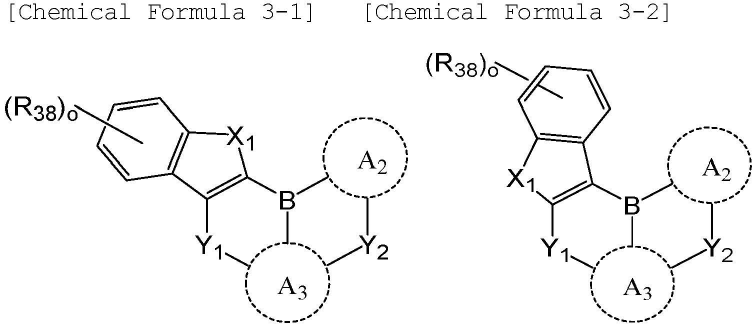 wherein,
X₁ is O or S,
Y₁ and Y₂, which are same or different, are each independently any one of O, S, NR₃₁, CR₃₂R₃₃, SiR₃₄R₃₅, and GeR₃₆R₃₇,
A₂ and A₃, which are same or different, are each independently any one selected from a substituted or unsubstituted aromatic hydrocarbon ring of 6 to 50 carbon atoms, a substituted or unsubstituted aliphatic hydrocarbon ring of 5 to 50 carbon atoms, a substituted or unsubstituted aliphatic hydrocarbon ring-fused aromatic hydrocarbon ring of 8 to 50 carbon atoms, a substituted or unsubstituted heteroaromatic ring of 2 to 50 carbon atoms, and a substituted or unsubstituted aliphatic hydrocarbon ring-fused heteroaromatic ring of 5 to 50 carbon atoms,
R₃₁ to R₃₈, which are same or difference, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted alkoxy of 1 to 30 carbon atoms, a substituted or unsubstituted aryloxy of 6 to 30 carbon atoms, a substituted or unsubstituted cycloalkyloxy of 3 to 30 carbon atoms, a substituted or unsubstituted heteroaryloxy of 2 to 30 carbon atoms, a substituted or unsubstituted alkylthio of 1 to 30 carbon atoms, a substituted or unsubstituted arylthio of 6 to 30 carbon atoms, a substituted or unsubstituted cycloalkylthio of 3 to 30 carbon atoms, a substituted or unsubstituted heteroarylthio of 2 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
R₃₁ to R₃₇ may be linked to A₂ and A₃ ring moieties to form mono- or polycyclic aliphatic or aromatic ring,
a linkage may be made between R₃₂ and R₃₃, between R₃₄ and R₃₅, and between R₃₆ and R₃₇ to form respective mono- or polycyclic aliphatic or aromatic rings,
o is 4, wherein the corresponding R₃₈ are same or different,
adjacent R₃₈ substituents may be linked to each other to additionally form a mono- or polycyclic aliphatic or aromatic ring,
wherein the term "substituted" in the expression "a substituted or unsubstituted" used for the compounds of Chemical Formulas 3-1 and 3-2 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, an aromatic hydrocarbon ring-fused cycloalkyl of 7 to 30 carbon atoms, a heteroaromatic ring-fused cycloalkyl of 5 to 30 carbon atoms, an aromatic hydrocarbon ring-fused heterocyclic alkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, a substituted or unsubstituted heteroaliphatic ring-fused aryl of 6 to 30 carbon atoms, a substituted or unsubstituted heteroaliphatic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with at least one hydrogen atom on the substituents being substitutable with a deuterium or tritium atom.

In an embodiment, concrete examples of the compound represented by Chemical Formula 3 include, but are not limited to:

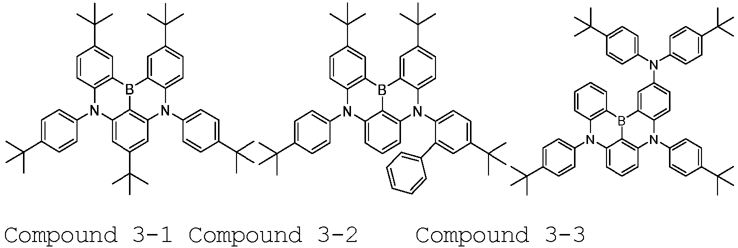

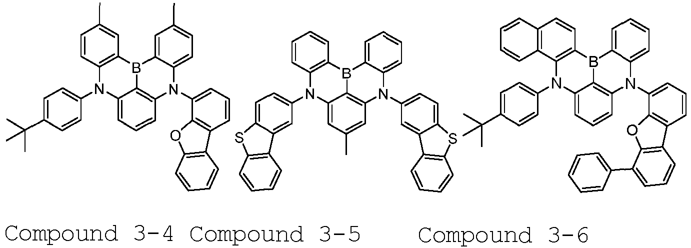

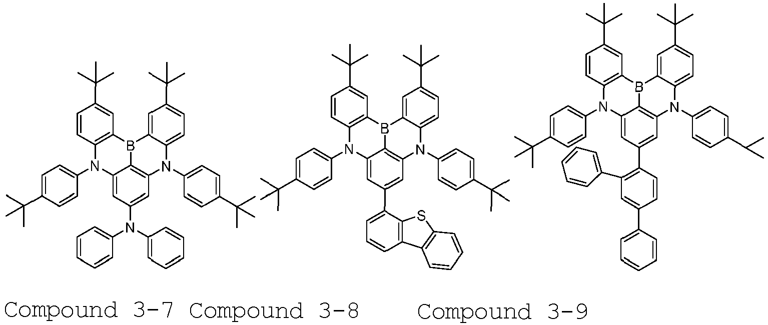

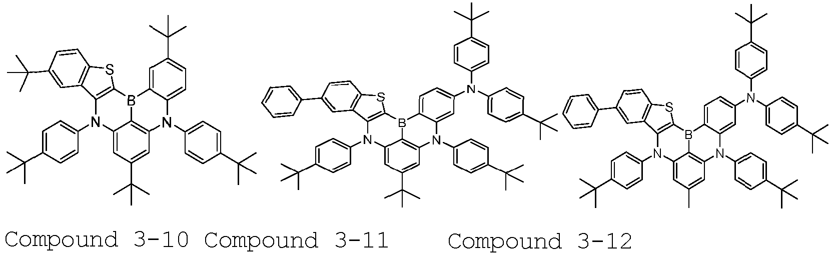

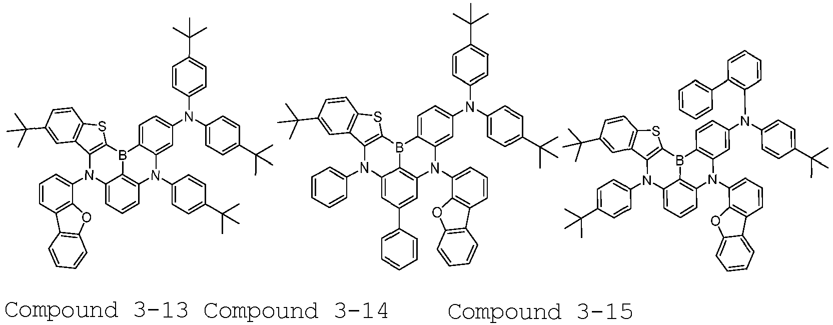

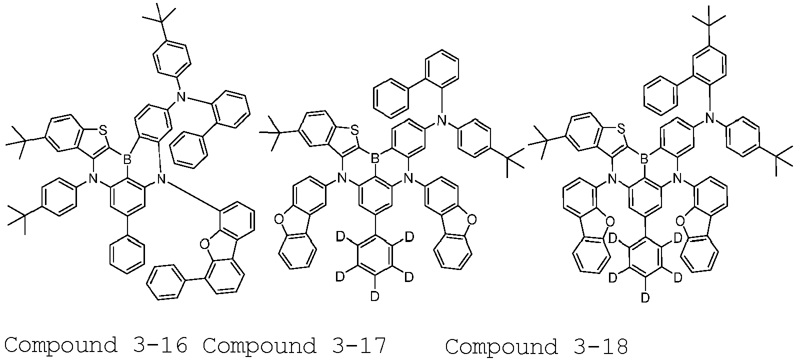

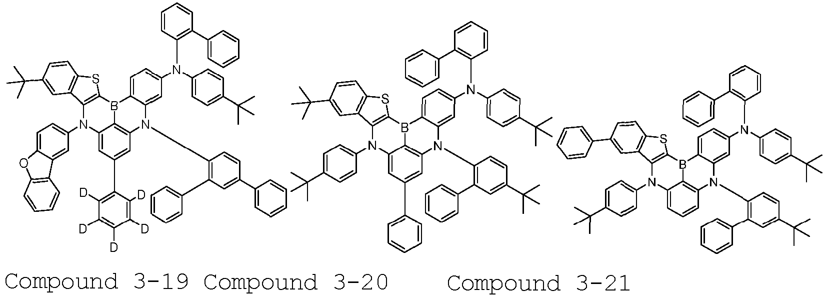

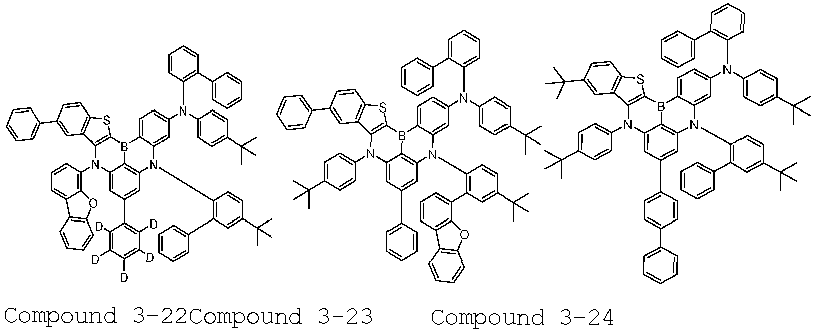

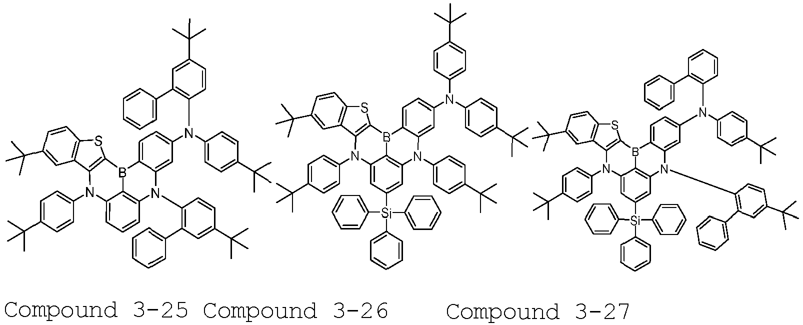

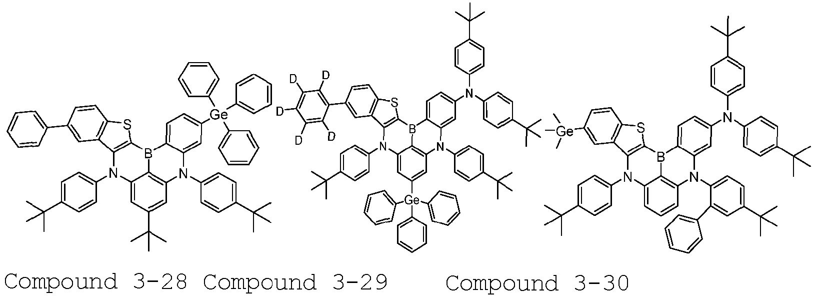

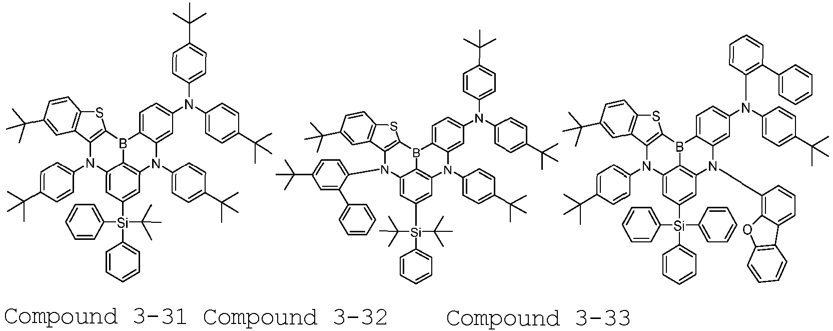

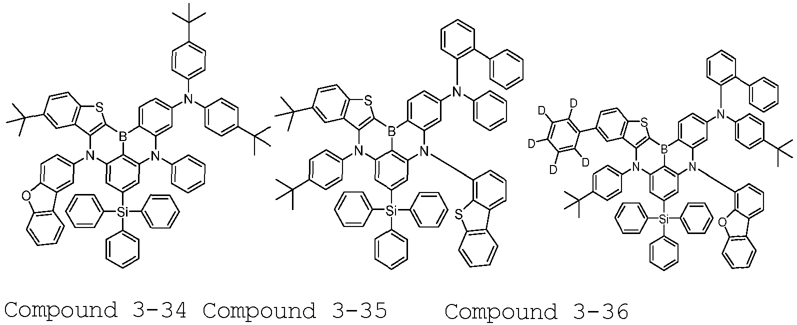

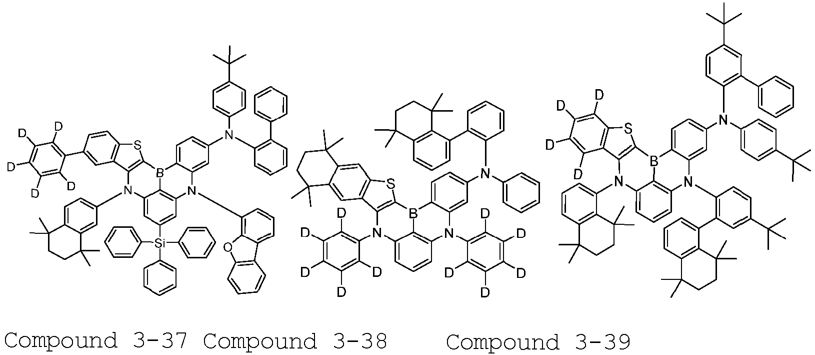

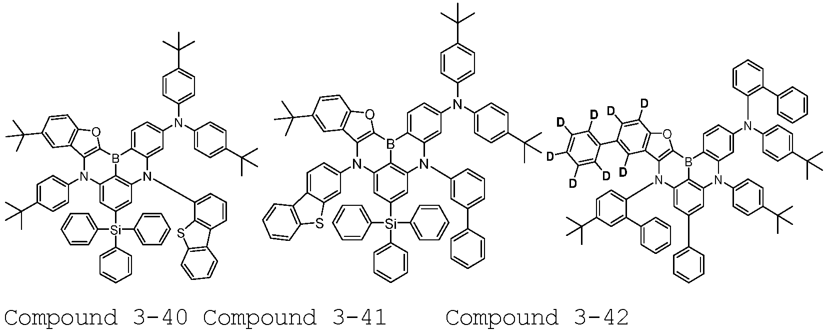

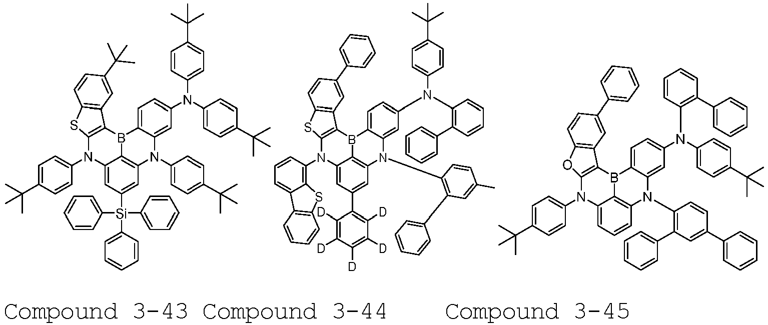

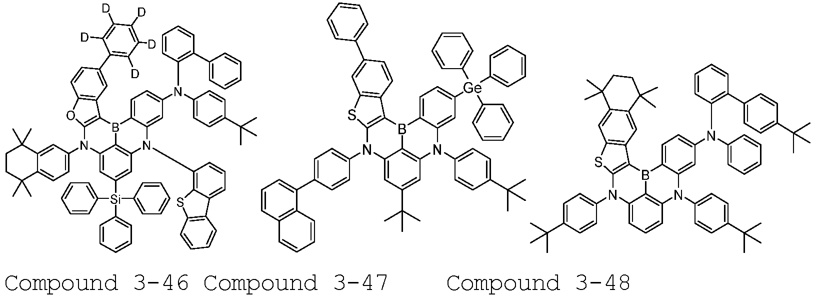

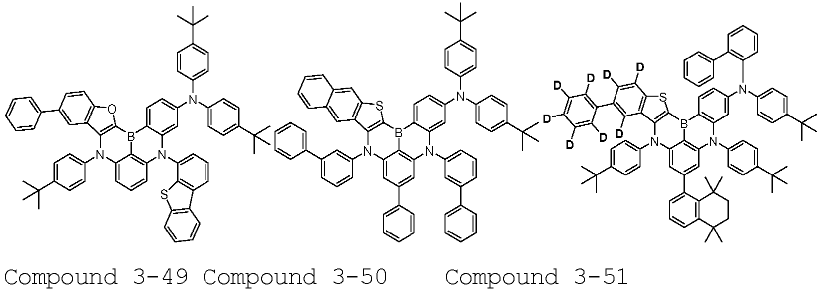

In addition, the present disclosure provides a compound represented by Chemical Formula 1.

Below, with reference to the drawing, a description will be given of the organic light-emitting diode of the present disclosure.

FIGURE is a schematic view illustrating the structure of an organic light-emitting diode according to an embodiment of the present disclosure.

As shown in FIGURE, the organic light-emitting diode according to an embodiment of the present disclosure comprises an anode (20), a hole transport layer (40), a first emission layer (50'), a second emission layer (50), an electron transport layer (60), and a cathode (80), wherein the anode and the cathode serve as a first electrode and a second electrode, respectively, with the interposition of the hole transport layer between the anode and the light emission layer, and the electron transport layer between the emission layer and the cathode.

Furthermore, the organic light-emitting diode according to an embodiment of the present disclosure may include a hole injection layer (30) between the anode (20) and the hole transport layer (40), and an electron injection layer (70) between the electron transport layer (60) and the cathode (80).

Reference is made to FIGURE with regard to the organic light-emitting diode of the present disclosure and the fabrication thereof.

First, a substrate (10) is coated with an anode material to form an anode (20). So long as it is used in a typical organic electroluminescence (EL) device, any substrate may be used as the substrate (10). Preferable is an organic substrate or transparent plastic substrate that exhibits excellent transparency, surface smoothness, ease of handling, and waterproofness. As the anode material, indium tin oxide (ITO), indium zinc oxide (IZO), tin oxide (SnO₂), or zinc oxide (ZnO), which are transparent and superior in terms of conductivity, may be used.

A hole injection layer material is applied on the anode (20) by thermal deposition in a vacuum or by spin coating to form a hole injection layer (30). Subsequently, thermal deposition in a vacuum or by spin coating may also be conducted to form a hole transport layer (40) with a hole transport layer material on the hole injection layer (30).

So long as it is typically used in the art, any material may be selected for the hole injection layer (30) without particular limitations thereto. Examples include, but are not limited to, 2-TNATA [4,4',4"-tris(2-naphthylphenyl-phenylamino)-triphenylamine], NPD[N,N'-di(1-naphthyl)-N,N'-diphenylbenzidine)], TPD[N,N'-diphenyl-N,N'-bis(3-methylphenyl)-1,1'-biphenyl-4,4'-diamine], DNTPD[N,N'-diphenyl-N,N'-bis-[4-(phenyl-m-tolyl-amino)-phenyl]-biphenyl-4,4'-diamine] HAT-CN [1,4,5,8,9,11-Hexaazatriphenylenehexacarbonitrile]

Any material that is typically used in the art may be selected for the hole transport layer (40) without particular limitations thereto. Examples include, but are not limited to, N,N'-bis(3-methylphenyl)-N,N'-diphenyl-[1,1-biphenyl]-4,4'-diamine(TPD) or N,N'-di(naphthalen-1-yl)-N,N'-diphenylbenzidine (a-NPD), N-[[1,1'-biphenyl]-4-yl]-9,9-dimethyl-N-[4-(9-phenyl-9H-carbazol-3-yl)phenyl]-9H-fluorene-2-amine(BCFN), and the like.

In an embodiment of the present disclosure, an electron blocking layer may be additionally disposed on the hole transport layer. Functioning to prevent the electrons injected from the electron injection layer from entering the hole transport layer through the emission layer, the electron blocking layer is adapted to increase the life span and luminous efficiency of the diode. The electron blocking layer may be formed of a well-known material or a combination of well-known materials, as necessary, at a suitable position between the emission layer and the hole injection layer. Particularly, the electron blocking layer may be formed between the emission layer and the hole transport layer.

Next, the first emission layer (50') and the second emission layer (50) may each be deposited or co-deposited on the hole transport layer (40) by deposition in a vacuum or by spin coating.

Here, the compound represented by Chemical Formula 1 or Chemical Formula 2 may be used as a host material in the first or second emission layer of the OLED while the compound represented by Chemical Formula 3 may be used as a dopant material in the first or second emission layer of the OLED. The materials constituting these layers are as previously described.

According to a specific embodiment of the present disclosure, the thickness of the first emission layer (50') and the second emission layer (50) may range from 30 Å to 200 Å and preferably from 50 Å to 150 Å.

The electron transport layer (60) may be deposited on the emitting layer by vacuum deposition or spin coating.

In the present disclosure, the electron transport layer (60) functions to stably transport electrons injected from the electron-injecting electrode (cathode), and known electron transport materials may be used. Examples of such known materials include quinoline derivatives, particularly tris(8-quinolinolato)aluminum (Alq₃), Liq, TAZ, BAlq, beryllium bis(benzoquinolin-10-olate) (Bebq2), Compound 201, Compound 202, BCP, and oxadiazole derivatives such as PBD, BMD, and BND, but are not limited thereto:

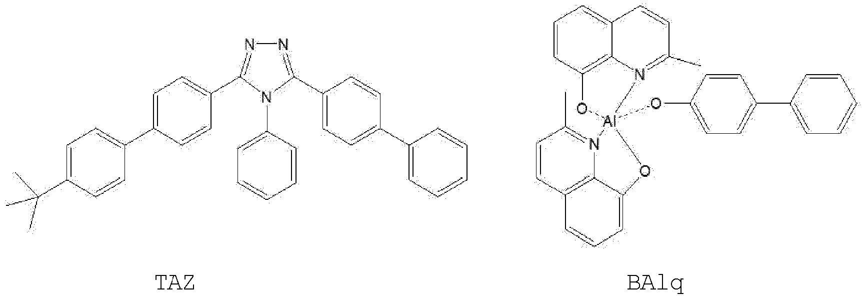

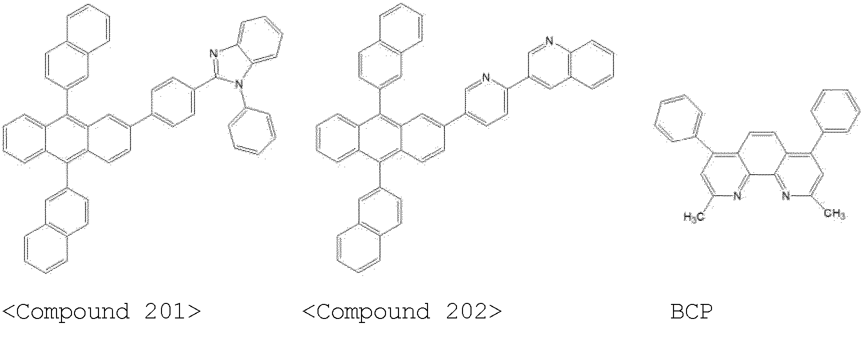

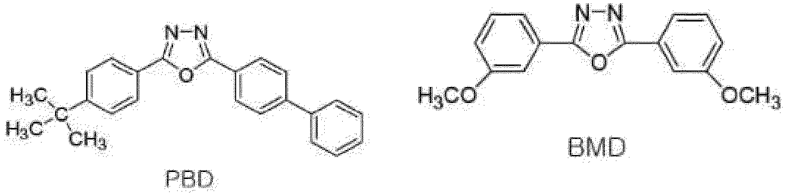

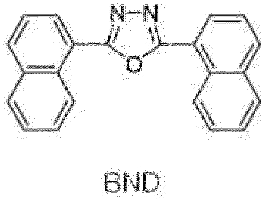

In the organic light-emitting diode of the present disclosure, an electron injection layer (EIL) that functions to facilitate electron injection from the cathode may be formed on the electron transport layer. The material for the EIL is not particularly limited.

Any material that is conventionally used in the art can be available for the electron injection layer (70) without particular limitations. Examples include CsF, NaF, LiF, Li₂O, and BaO. Deposition conditions for the electron injection layer may vary, depending on compounds used, but may be generally selected from condition scopes that are almost the same as for the formation of hole injection layers.

The electron injection layer (70) may range in thickness from about 1 Å to about 100 Å, and particularly from about 3 Å to about 90 Å. Given the thickness range for the electron injection layer, the diode can exhibit satisfactory electron injection properties without actually elevating a driving voltage.

In order to facilitate electron injection, the cathode (80) may be made of a material having a small work function, such as metal or metal alloy such as lithium (Li), magnesium (Mg), calcium (Ca), an alloy aluminum (Al) thereof, aluminum-lithium (Al-Li), magnesium-indium (Mg-In), and magnesium-silver (Mg-Ag). Alternatively, ITO or IZO may be employed to form a transparent cathode for an organic light-emitting diode.

Moreover, the organic light-emitting diode of the present disclosure may further comprise a emission layer containing a blue, green, or red luminescent material that emits radiations in the wavelength range of 380 nm to 800 nm. That is, the emission layer in the present disclosure has a multi-layer structure wherein the blue, green, or red luminescent material may be a fluorescent material or a phosphorescent material.

Furthermore, at least one selected from among the layers may be deposited using a single-molecule deposition process or a solution process.

Here, the deposition process is a process by which a material is vaporized in a vacuum or at a low pressure and deposited to form a layer, and the solution process is a method in which a material is dissolved in a solvent and applied for the formation of a thin film by means of inkjet printing, roll-to-roll coating, screen printing, spray coating, dip coating, spin coating, etc.

Also, the organic light-emitting diode of the present disclosure may be applied to a device selected from among flat display devices; flexible display devices; monochrome or grayscale flat illumination devices; monochrome or grayscale flexible illumination devices; vehicle or aircraft display devices, and display devices for virtual or augmented reality.

A better understanding of the present disclosure may be obtained through the following examples which are set forth to illustrate, but are not to be construed as limiting the present disclosure.

### (EXAMPLES)

### SYNTHESIS EXAMPLE 1. Synthesis of [Compound 1-9]

### Synthesis Example 1-1. Synthesis of A-1

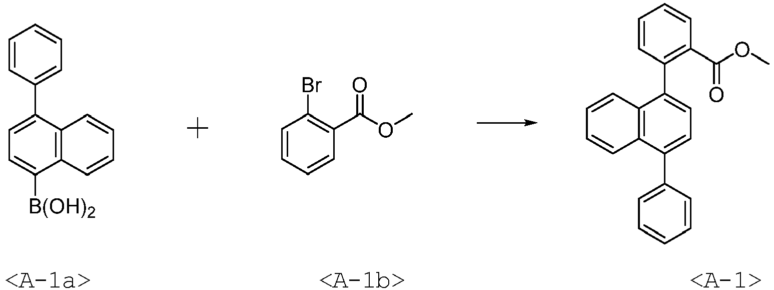

In a round-bottom flask, a mixture of <A-1a> (57.11 g), <A-1b> (45 g), tetrakis(triphenylphosphine)palladium (0) (7.26 g), potassium carbonate (72.3 g), and tetrahydrofuran (600 mL) was refluxed for 12 hours under a nitrogen atmosphere. After completion of the reaction, the reaction mixture was subjected to extraction with water. Purification by column chromatography afforded <A-1> (60.2 g, 85%).

### Synthesis Example 1-2. Synthesis of A-2

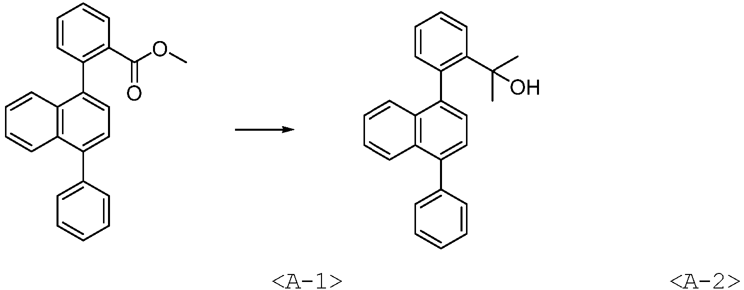

In a round-bottom flask, a mixture of <A-1> (60 g) and tetrahydrofuran (300 mL) was chilled to -10°C under a nitrogen atmosphere, slowly added with drops of 3M methylmagnesium bromide, and then stirred at 50°C for 12 hours. After completion of the reaction, the reaction mixture was subjected to extraction by slowly adding 10% aqueous ammonium chloride solution and toluene. Purification by column chromatography afforded <A-2>. (42 g, 70%)

### Synthesis Example 1-3. Synthesis of A-3

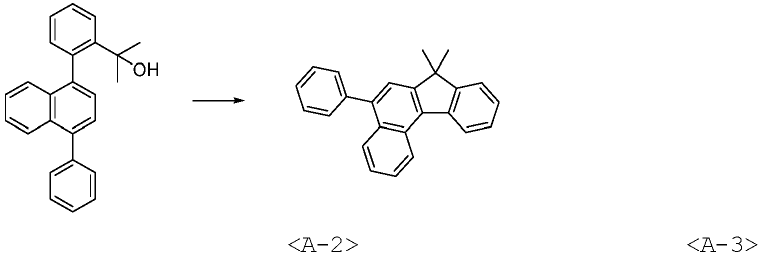

In a round-bottom flask, a mixture of <A-2> (42 g) and acetic acid (420 mL) was added with HCl (10 mL) at 50°C under a nitrogen atmosphere. After completion of the reaction, the mixture was subjected to extraction with water. Purification by column chromatography afforded <A-3>. (34.6 g, 87%)

### Synthesis Example 1-4. Synthesis of A-4

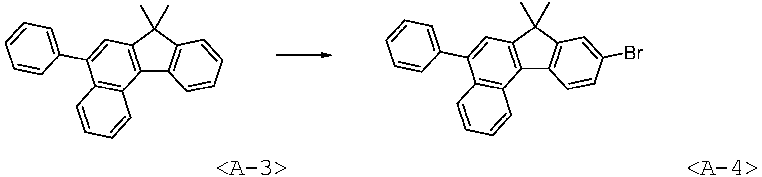

In a round-bottom flask, a mixture of <A-3> (34.6 g) and dichloromethane (350 mL) was chilled to -10°C under a nitrogen atmosphere, slowly added with (20.7 g), and then stirred at room temperature for 12 hours. After completion of the reaction, the mixture was subjected to extraction with water. Purification by column chromatography afforded <A-4>. (32.3 g, 75%)

### Synthesis Example 1-5. Synthesis of A-5

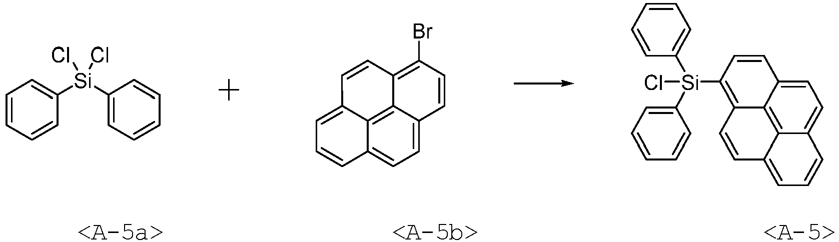

In a round-bottom flask, a mixture of <A-5b> (27 g) and tetrahydrofuran (405 mL) was chilled to -78°C under a nitrogen atmosphere, slowly added with drops of 1.6M n-butyl lithium (72 mL), and then stirred for 1 hour. Subsequently, <A-5a> (26.7 g) was quickly added before stirring at room temperature for 12 hours. After completion of the reaction, the organic solvent was removed by concentration in a vacuum. The concentrate was diluted with n-heptane and filtered. The filtered solution was dried in a vacuum to afford <A-2>. (25.35 g, 63%)

### Synthesis Example 1-6. Synthesis of [Compound 1-9]

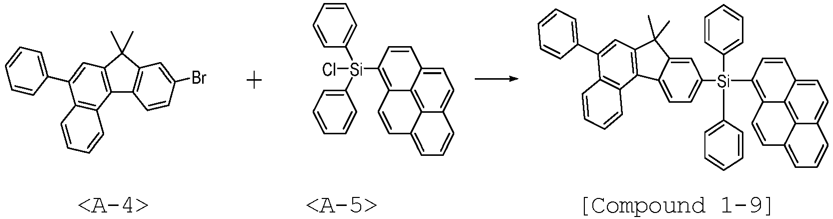

The same synthesis procedure as in Synthesis Example 1-5, with the exception of using <A-5> and <A-4> instead of <A-5a> and <A-5b>, respectively, was carried out to afford [Compound 1-9]. (yield 60%)
MS (MALDI-TOF) : m/z 702.27 [M⁺]

### SYNTHESIS EXAMPLE 2. Synthesis of [Compound 1-11]

### Synthesis Example 2-1. Synthesis of B-1

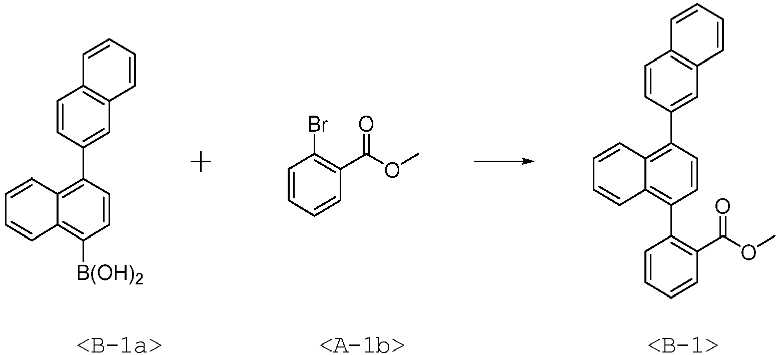

The same synthesis procedure as in Synthesis Example 1-1, with the exception of using <A-1a> instead of <B-1a>, was carried out to afford <B-1>. (yield 90%)

### Synthesis Example 2-2. Synthesis of B-2

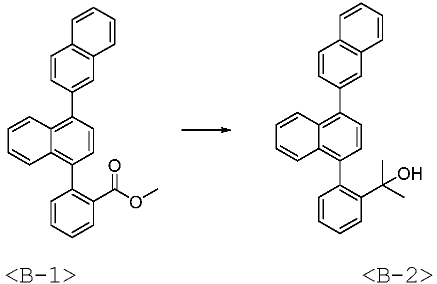

In a round-bottom flask, a mixture of <B-1> (50 g) and tetrahydrofuran (250 mL)was chilled to -10°C under a nitrogen atmosphere, slowly added with drops of 3M methylmagnesium bromide, and then stirred at 50°C for 12 hours. After completion of the reaction, the reaction mixture was subjected to extraction by slowly adding 10% aqueous ammonium chloride solution and toluene. Purification by column chromatography afforded <B-2>. (35 g, 70%)

### Synthesis Example 2-3. Synthesis of B-3

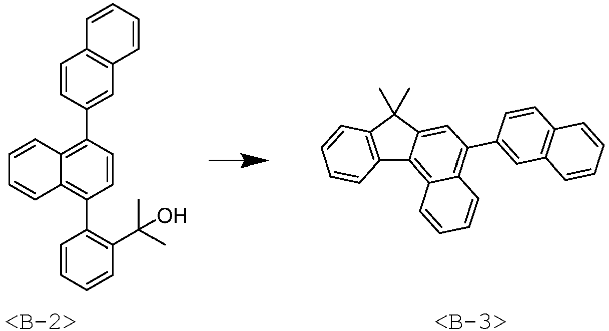

In a round-bottom flask, a mixture of <B-2> (30 g) and acetic acid (300 mL) was added with HCl (10 mL) at 50°C and refluxed for 12 hours. After completion of the reaction, the mixture was subjected to extraction with water. Purification by column chromatography afforded <B-3>. (25 g, 87%)

### Synthesis Example 2-4. Synthesis of B-4

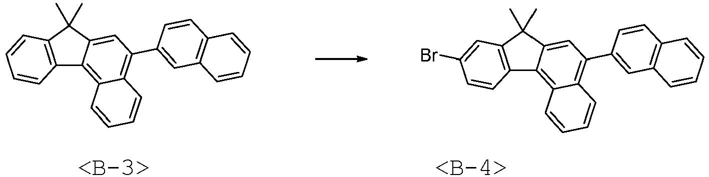

In a round-bottom flask, a mixture of <B-3> (25 g) and dichloromethane (250 mL) was chilled to -10°C under a nitrogen atmosphere, slowly added with bromine (5.7 g), and then stirred at room temperature for 12 hours. After completion of the reaction, the mixture was subjected to extraction with water. Purification by column chromatography afforded <B-5>. (22.8 g, 75%)

### Synthesis Example 2-5. Synthesis of [Compound 1-11]

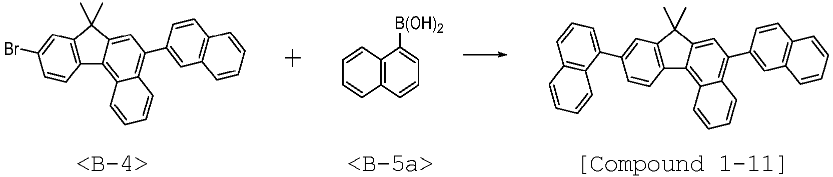

The same synthesis procedure as in Synthesis Example 1-1, with the exception of using <B-5a> and <B-4> instead of <A-1a> and <A-1b>, respectively, was carried out to afford [Compound 1-11]. (yield 71%)
MS (MALDI-TOF) : m/z 496.22 [M⁺]

### SYNTHESIS EXAMPLE 3. Synthesis of [Compound 1-24]

### Synthesis Example 3-1. Synthesis of C-1

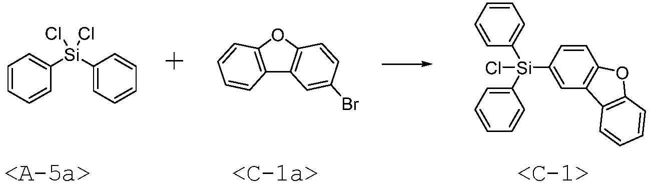

The same synthesis procedure as in Synthesis Example 1-5, with the exception of using <C-1a> instead of <A-5b>, was carried out to afford <C-1>. (yield 68%)

### Synthesis Example 3-2. Synthesis of C-2

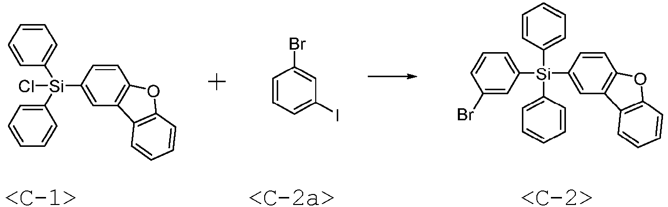

The same synthesis procedure as in Synthesis Example 1-5, with the exception of using <C-1> and <C-2a> instead of <A-5a> and <A-5b>, respectively, was carried out to afford <C-2>. (yield 76%)

### Synthesis Example 3-3. Synthesis of C-3

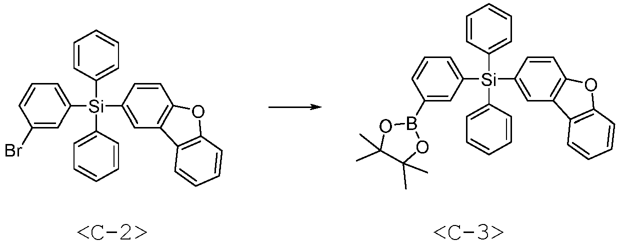

In a round-bottom flask, a mixture of <C-2> (34 g), bis(pinacolato)diboron (25.6 g), [1,1'-bis(diphenylphosphino)ferocene]dichloropalladium (II) (1.48 g), potassium acetate (19.8 g), and 1,4-dioxane (340 mL) was refluxed for 12 hours under a nitrogen atmosphere. After completion of the reaction, the mixture was subjected to extraction with water. Purification by column chromatography afforded <C-3>. (30.1 g, 81%)

### Synthesis Example 3-4. Synthesis of [Compound 1-24]

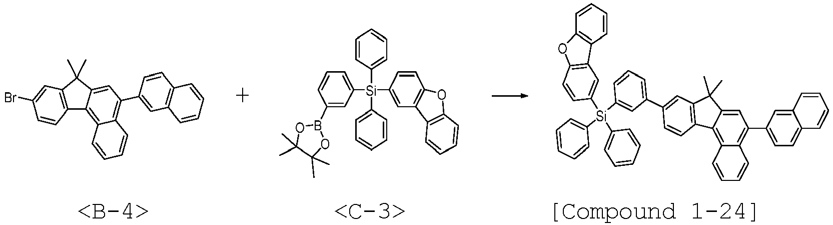

The same synthesis procedure as in Synthesis Example 1-1, with the exception of using <C-3> and <B-4> instead of <A-1a> and <A-1b>, respectively, was carried out to afford [Compound 1-24]. (yield 74%)
MS (MALDI-TOF) : m/z 794.30 [M⁺]

### SYNTHESIS EXAMPLE 4. Synthesis of [Compound 1-34]

### Synthesis Example 4-1. Synthesis of D-1

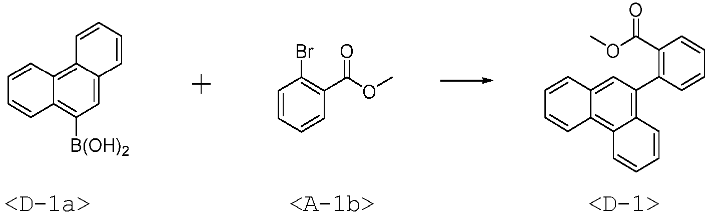

The same synthesis procedure as in Synthesis Example 1-1, with the exception of using <D-1a> instead of <A-1a>, was carried out to afford <D-1>. (yield 87%)

### Synthesis Example 4-2. Synthesis of D-2

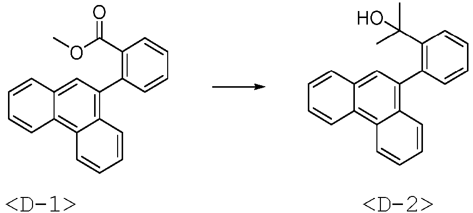

The same synthesis procedure as in Synthesis Example 1-2, with the exception of using <D-1> instead of <A-1>, was carried out to afford <D-2>. (yield 74%)

### Synthesis Example 4-3. Synthesis of D-3

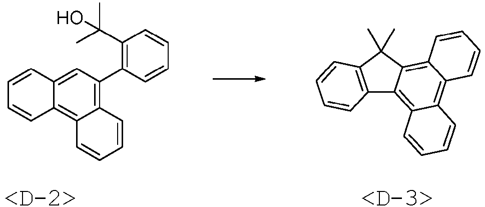

The same synthesis procedure as in Synthesis Example 1-3, with the exception of using <D-2> instead of <A-2>, was carried out to afford <D-3>. (yield 71%)

### Synthesis Example 4-4. Synthesis of D-4

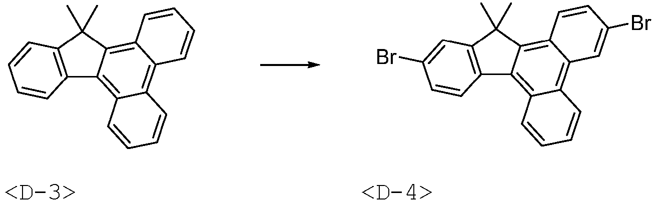

The same synthesis procedure as in Synthesis Example 1-4, with the exception of using <D-3> instead of <A-3>, was carried out to afford <D-4>. (yield 67%)

### Synthesis Example 4-5. Synthesis of [Compound 1-34]

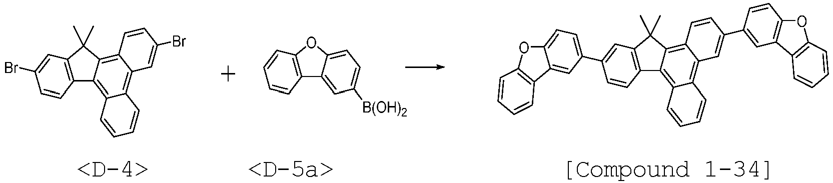

The same synthesis procedure as in Synthesis Example 1-1, with the exception of using <D-5a> and <D-4> instead of <A-1a> and <A-1b>, respectively, was carried out to afford [Compound 1-34]. (yield 73%)
MS (MALDI-TOF) : m/z 626.22 [M⁺]

### SYNTHESIS EXAMPLE 5. Synthesis of [Compound 1-39]

### Synthesis Example 5-1. Synthesis of E-1

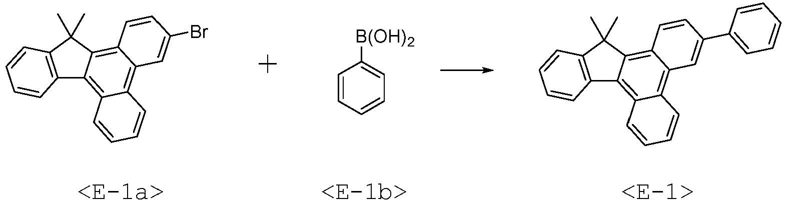

The same synthesis procedure as in Synthesis Example 1-1, with the exception of using <E-1b> and <E-1a> instead of <A-1a> and <A-1b>, respectively, was carried out to afford <E-1>. (yield 79%)

### Synthesis Example 5-2. Synthesis of E-2

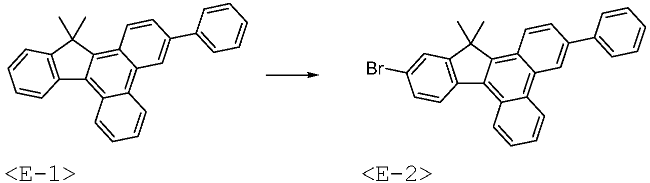

The same synthesis procedure as in Synthesis Example 1-4, with the exception of using <E-1> instead of <A-3>, was carried out to afford <E-2>. (yield 74%)

### Synthesis Example 5-3. Synthesis of [Compound 1-39]

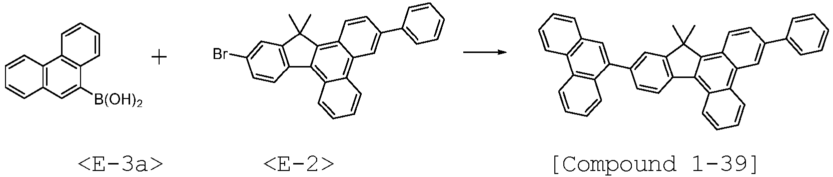

The same synthesis procedure as in Synthesis Example 1-1, with the exception of using <E-3a> and <E-2> instead of <A-1a> and <A-1b>, respectively, was carried out to afford [Compound 1-39]. (yield 82%)
MS (MALDI-TOF) : m/z 546.23 [M⁺]

### SYNTHESIS EXAMPLE 6. Synthesis of [Compound 1-41]

### Synthesis Example 6-1. Synthesis of F-1

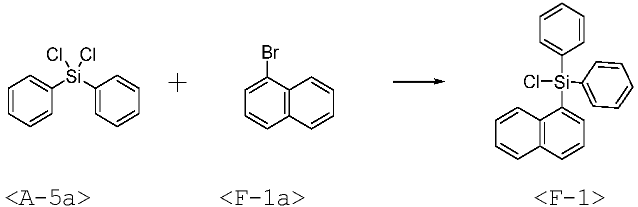

The same synthesis procedure as in Synthesis Example 1-5, with the exception of using <F-1a> instead of <A-5b>, was carried out to afford <F-1>. (yield 63%)

### Synthesis Example 6-2. Synthesis of [Compound 1-41]

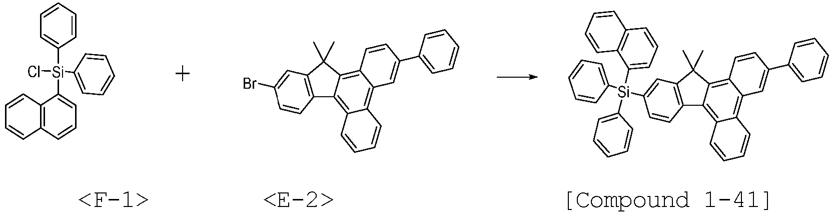

The same synthesis procedure as in Synthesis Example 1-5, with the exception of using <F-1> and <E-2> instead of <A-5a> and <A-5b>, respectively, was carried out to afford [Compound 1-41]. (yield 66%)
MS (MALDI-TOF) : m/z 678.27 [M⁺]

### SYNTHESIS EXAMPLE 7. Synthesis of [Compound 1-64]

### Synthesis Example 7-1. Synthesis of G-1

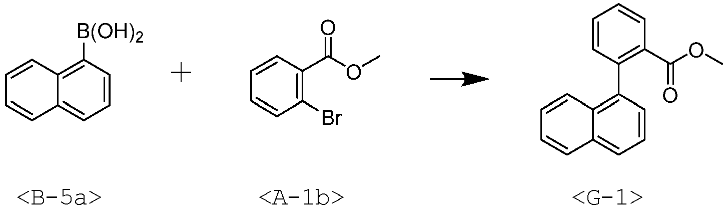

The same synthesis procedure as in Synthesis Example 1-1, with the exception of using <B-5a> instead of <A-1a>, was carried out to afford <G-1>. (yield 89%)

### Synthesis Example 7-2. Synthesis of G-2

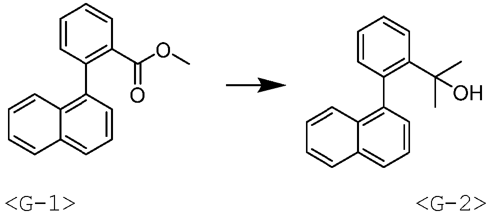

The same synthesis procedure as in Synthesis Example 1-2, with the exception of using <G-1> instead of <A-1>, was carried out to afford <G-2>. (yield 78%)

### Synthesis Example 7-3. Synthesis of G-3

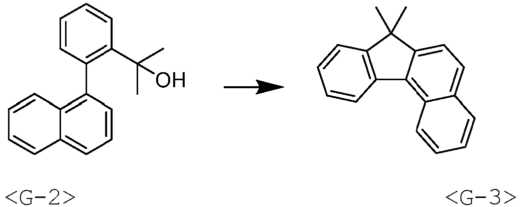

The same synthesis procedure as in Synthesis Example 1-3, with the exception of using <G-2> instead of <A-2>, was carried out to afford <G-3>. (yield 71%)

### Synthesis Example 7-4. Synthesis of G-4

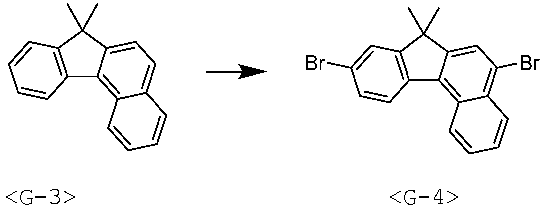

The same synthesis procedure as in Synthesis Example 1-4, with the exception of using <G-3> instead of <A-3>, was carried out to afford <G-4>. (yield 67%)

### Synthesis Example 7-5. Synthesis of [Compound 1-64]

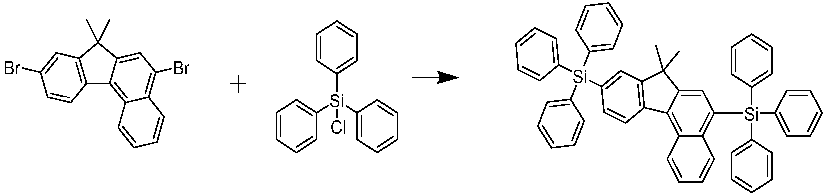

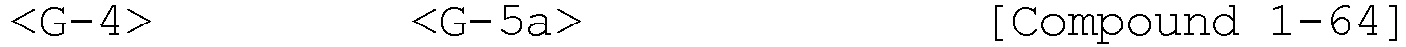

The same synthesis procedure as in Synthesis Example 1-5, with the exception of using <G-5a> and <G-4> instead of <A-5a> and <A-5b>, respectively, was carried out to afford [Compound 1-64]. (yield 66%)
MS (MALDI-TOF) : m/z 760.30 [M⁺]

### SYNTHESIS EXAMPLE 8. Synthesis of [Compound 1-65]

### Synthesis Example 8-1. Synthesis of H-1

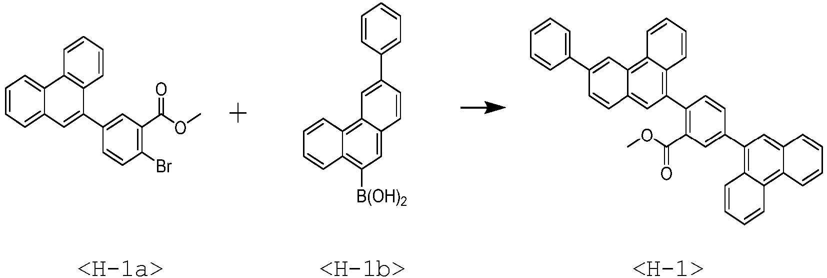

The same synthesis procedure as in Synthesis Example 1-1, with the exception of using <H-1b> and <H-1a> instead of <A-1a> and <A-1b>, respectively, was carried out to afford <H-1>. (yield 74%)

### Synthesis Example 8-2. Synthesis of H-2

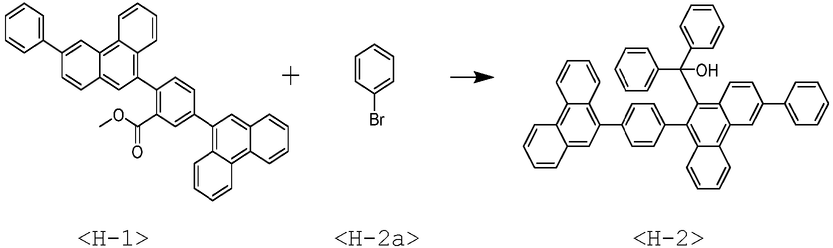

In a round-bottom flask, a mixture of <H-2a> (27.8 g) and tetrahydrofuran (278 mL) was chilled to -78°C under a nitrogen atmosphere and slowly added with drops of 1.6M n-butyl lithium (121.8 mL). After stirring for 1 hour, a solution of <H-1> (50 g) in tetrahydrofuran (250 mL) was slowly added in a dropwise manner and stirred at room temperature for 12 hours. After completion of the reaction, the mixture was subjected to extraction with water. Purification by column chromatography afforded <H-2>. (42.7 g, 70%)

### Synthesis Example 8-3. Synthesis of [Compound 1-65]

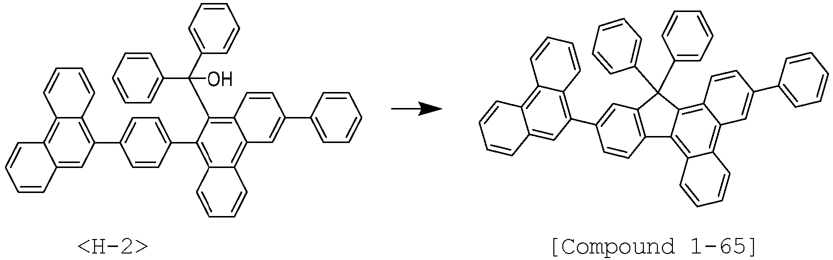

The same synthesis procedure as in Synthesis Example 1-3, with the exception of using <H-2> instead of <A-2>, was carried out to afford [Compound 1-65]. (yield 63%)
MS (MALDI-TOF) : m/z 670.27 [M⁺]

### SYNTHESIS EXAMPLE 9. Synthesis of [Compound 1-70]

### Synthesis Example 9-1. Synthesis of I-1

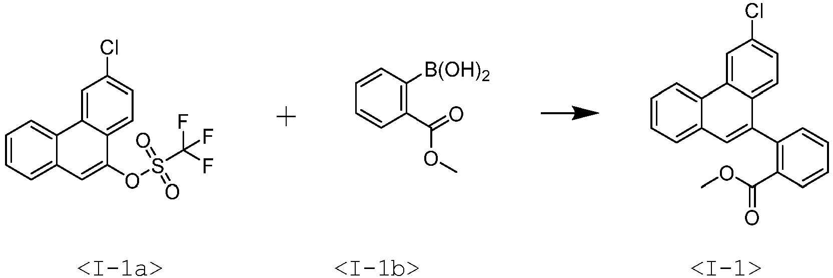

The same synthesis procedure as in Synthesis Example 1-1, with the exception of using <I-1b> and <I-1a> instead of <A-1a> and <A-1b>, respectively, was carried out to afford <I-1>. (yield 66%)

### Synthesis Example 9-2. Synthesis of I-2

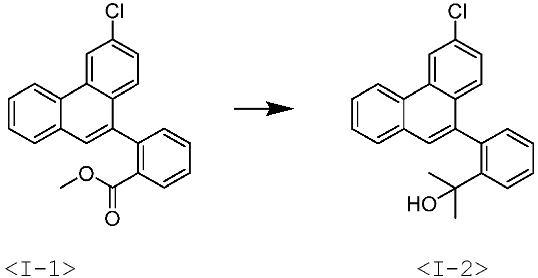

The same synthesis procedure as in Synthesis Example 1-2, with the exception of using <I-1> instead of <A-1>, was carried out to afford <I-2>. (yield 70%)

### Synthesis Example 9-3. Synthesis of I-3

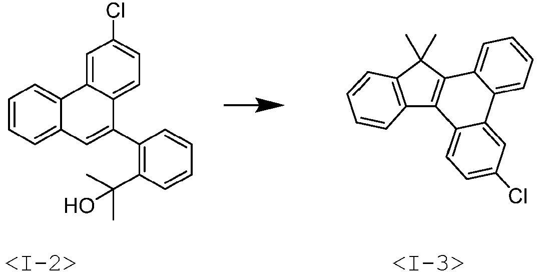

The same synthesis procedure as in Synthesis Example 1-3, with the exception of using <I-2> instead of <A-2>, was carried out to afford <I-3>. (yield 75%)

### Synthesis Example 9-4. Synthesis of I-4

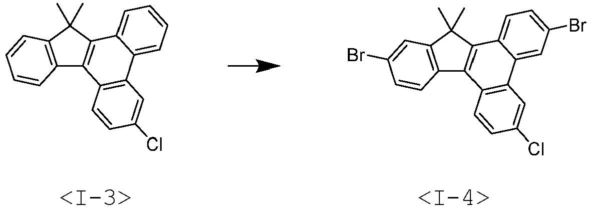

The same synthesis procedure as in Synthesis Example 1-4, with the exception of using <I-3> instead of <A-3>, was carried out to afford <I-4>. (yield 72%)

### Synthesis Example 9-5. Synthesis of I-5

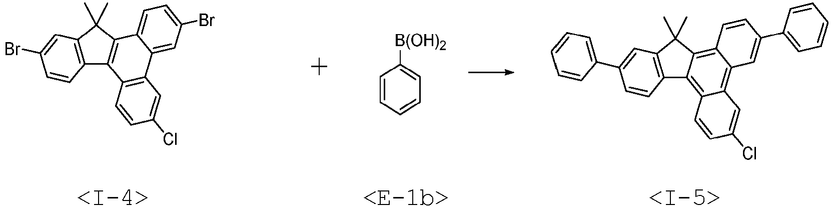

The same synthesis procedure as in Synthesis Example 1-1, with the exception of using <E-1b> and <I-4> instead of <A-1a> and <A-1b>, respectively, was carried out to afford <I-5>. (yield 76%)

### Synthesis Example 9-6. Synthesis of [Compound 1-70]

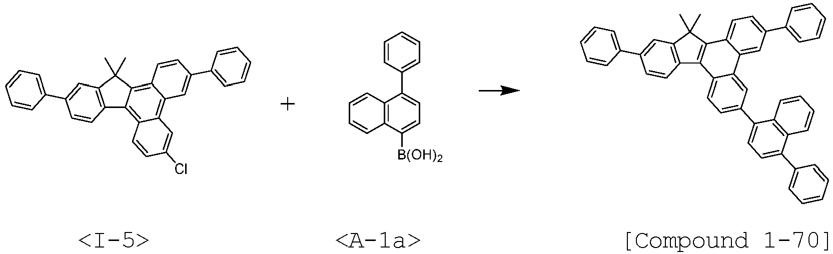

The same synthesis procedure as in Synthesis Example 1-1, with the exception of using <I-5> instead of <A-1b>, was carried out to afford [Compound 1-70]. (yield 73%)
MS (MALDI-TOF) : m/z 648.28 [M⁺]

### SYNTHESIS EXAMPLE 10. Synthesis of [Compound 1-73]

### Synthesis Example 10-1. Synthesis of [Compound 1-73]

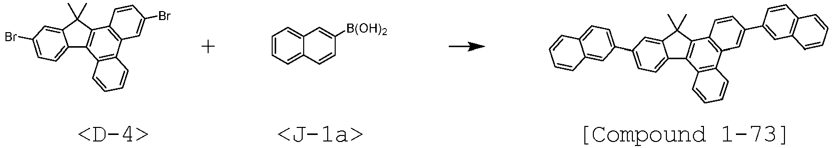

The same synthesis procedure as in Synthesis Example 1-1, with the exception of using <J-la> and <D-4> instead of <A-1a> and <A-1b>, respectively, was carried out to afford [Compound 1-73]. (yield 73%)
MS (MALDI-TOF) : m/z 546.23 [M⁺]

### SYNTHESIS EXAMPLE 11. Synthesis of [Compound 1-77]

### Synthesis Example 11-1. Synthesis of [Compound 1-77]

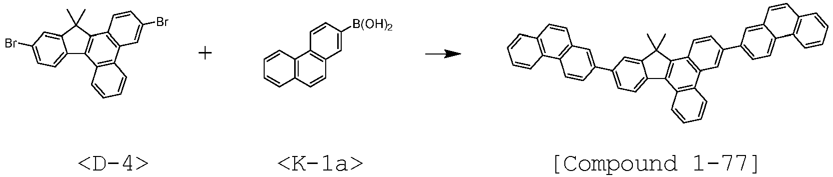

The same synthesis procedure as in Synthesis Example 1-1, with the exception of using <K-1a> and <D-4> instead of <A-1a> and <A-1b>, respectively, was carried out to afford [Compound 1-77]. (yield 71%)
MS (MALDI-TOF) : m/z 646.27 [M⁺]

### SYNTHESIS EXAMPLE 12. Synthesis of [Compound 1-78]

### Synthesis Example 12-1. Synthesis of L-1

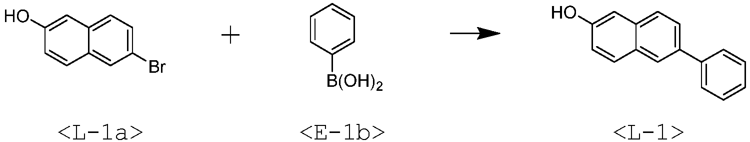

The same synthesis procedure as in Synthesis Example 1-1, with the exception of using <E-1b> and <L-1a> instead of <A-1a> and <A-1b>, respectively, was carried out to afford <L-1>. (yield 80%)

### Synthesis Example 12-2. Synthesis of L-2

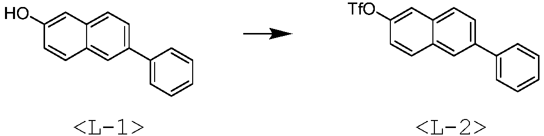

In a round-bottom flask, a mixture of <L-1> (35 g), pyridine (10 g), and dichloromethane (350 mL) was chilled to - 10°C under a nitrogen atmosphere and slowly added with drops of a dilution of trifluoromethanesulfonic anhydride (38 g) in dichloromethane (40 mL) before stirring at room temperature for 12 hours. After completion of the reaction, the reaction was subjected to extraction with water. Purification by column chromatography afforded <L-2>. (51.5 g, 92%)

### Synthesis Example 12-3. Synthesis of L-3

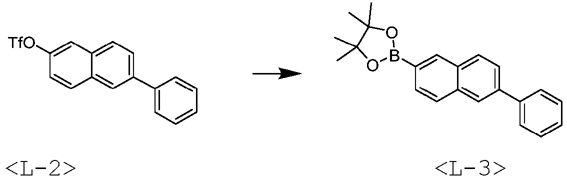

The same synthesis procedure as in Synthesis Example 3-3, with the exception of using <L-2> instead of <C-2>, was carried out to afford <L-3>. (yield 88%)

### Synthesis Example 12-4. Synthesis of [Compound 1-78]

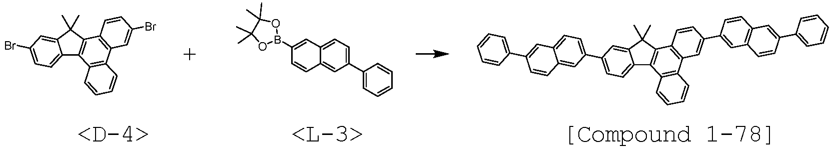

The same synthesis procedure as in Synthesis Example 1-1, with the exception of using <L-3> and <D-4> instead of <A-1a> and <A-1b>, respectively, was carried out to afford [Compound 1-78]. (yield 76%)
MS (MALDI-TOF) : m/z 698.30 [M⁺]

### SYNTHESIS EXAMPLE 13. Synthesis of [Compound 1-84]

### Synthesis Example 13-1. Synthesis of [Compound 1-84]

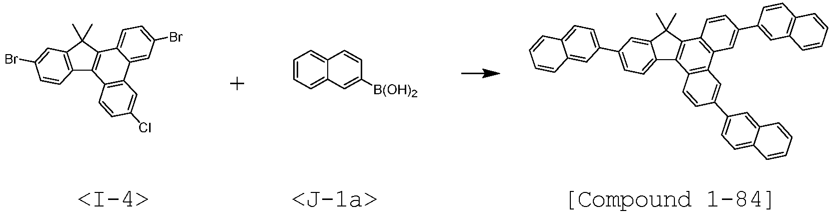

The same synthesis procedure as in Synthesis Example 1-1, with the exception of using <J-la> and <I-4> instead of <A-1a> and <A-1b>, respectively, was carried out to afford [Compound 1-84]. (yield 73%)
MS (MALDI-TOF) : m/z 672.28 [M⁺]

### SYNTHESIS EXAMPLE 14. Synthesis of [Compound 1-85]

### Synthesis Example 14-1. Synthesis of N-1

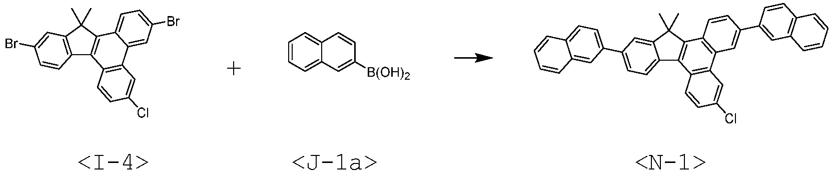

The same synthesis procedure as in Synthesis Example 1-1, with the exception of using <J-la> and <I-4> instead of <A-1a> and <A-1b>, respectively, was carried out to afford <N-1>. (yield 74%)

### Synthesis Example 14-2. Synthesis of [Compound 1-85]

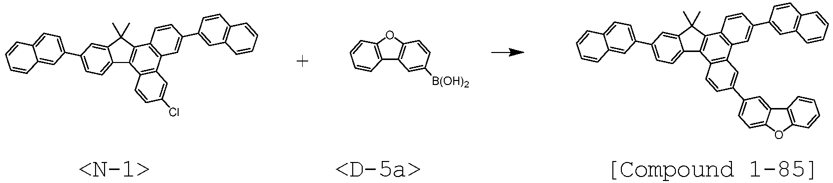

The same synthesis procedure as in Synthesis Example 1-1, with the exception of using <D-5a> and <N-1> instead of <A-1a> and <A-1b>, respectively, was carried out to afford [Compound 1-85]. (yield 82%)
MS (MALDI-TOF) : m/z 712.28 [M⁺]

### SYNTHESIS EXAMPLE 15. Synthesis of [Compound 1-89]

### Synthesis Example 15-1. Synthesis of O-1

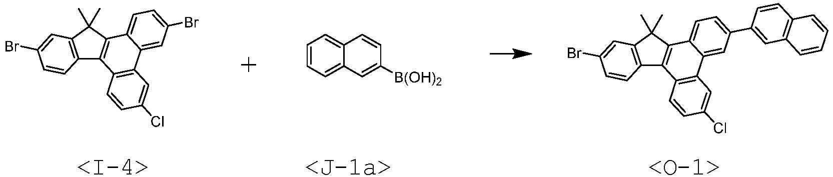

The same synthesis procedure as in Synthesis Example 1-1, with the exception of using <J-la> and <I-4> instead of <A-1a> and <A-1b>, respectively, was carried out to afford <O-1>. (yield 64%)

### Synthesis Example 15-2. Synthesis of O-2

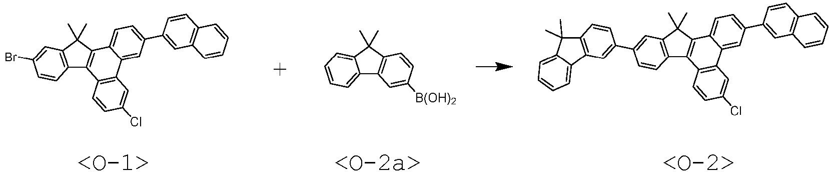

The same synthesis procedure as in Synthesis Example 1-1, with the exception of using <O-2a> and <O-1> instead of <A-1a> and <A-1b>, respectively, was carried out to afford <O-2>. (yield 66%)

### Synthesis Example 15-3. Synthesis of [Compound 1-89]

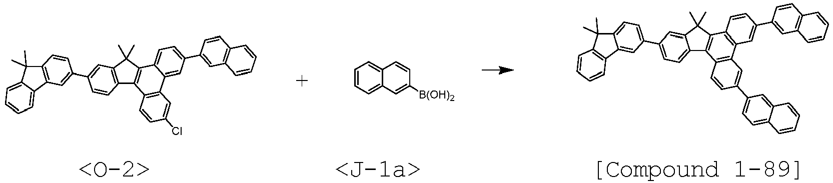

The same synthesis procedure as in Synthesis Example 1-1, with the exception of using <J-la> and <O-2> instead of <A-1a> and <A-1b>, respectively, was carried out to afford [Compound 1-89]. (yield 84%)
MS (MALDI-TOF) : m/z 738.33 [M⁺]

### SYNTHESIS EXAMPLE 16. Synthesis of [Compound 1-92]

### Synthesis Example 16-1. Synthesis of P-1

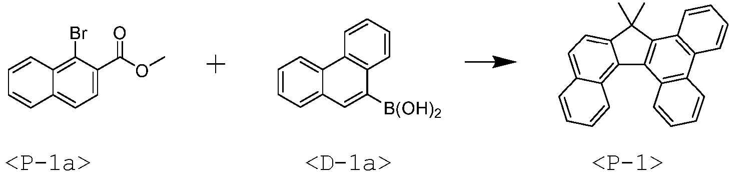

The same synthesis procedure as in Synthesis Example 1-1, with the exception of using <D-1a> and <P-1a> instead of <A-1a> and <A-1b>, respectively, was carried out to afford <P-1>. (yield 89%)

### Synthesis Example 16-2. Synthesis of P-2

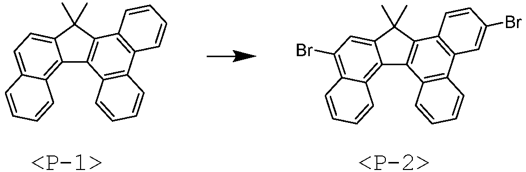

The same synthesis procedure as in Synthesis Example 1-4, with the exception of using <P-1> instead of <A-3>, was carried out to afford <P-2>. (yield 84%)

### Synthesis Example 16-3. Synthesis of [Compound 1-92]

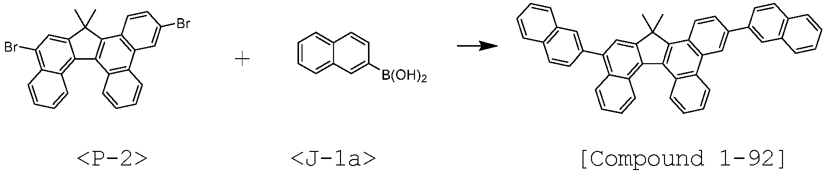

The same synthesis procedure as in Synthesis Example 1-1, with the exception of using <J-la> and <P-2> instead of <A-1a> and <A-1b>, respectively, was carried out to afford [Compound 1-92]. (yield 86%)
MS (MALDI-TOF) : m/z 596.25 [M⁺]

### SYNTHESIS EXAMPLE 17. Synthesis of [Compound 1-95]

### Synthesis Example 17-1. Synthesis of Q-1

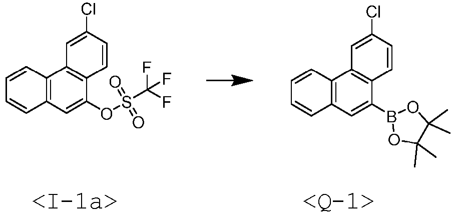

The same synthesis procedure as in Synthesis Example 3-3, with the exception of using <I-1a> instead of <C-2>, was carried out to afford <Q-1>. (yield 87%)

### Synthesis Example 17-2. Synthesis of Q-2

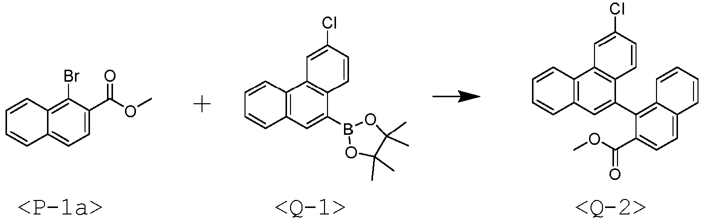

The same synthesis procedure as in Synthesis Example 1-1, with the exception of using <Q-1> and <P-1a> instead of <A-1a> and <A-1b>, respectively, was carried out to afford <Q-2>. (yield 83%)

### Synthesis Example 17-3. Synthesis of Q-3

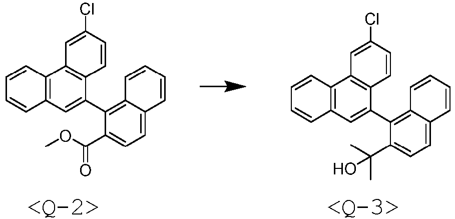

The same synthesis procedure as in Synthesis Example 1-2, with the exception of using <Q-2> instead of <A-1>, was carried out to afford <Q-3>. (yield 76%)

### Synthesis Example 17-4. Synthesis of Q-4

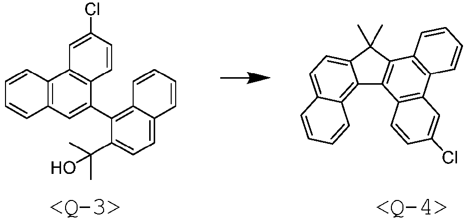

The same synthesis procedure as in Synthesis Example 1-3, with the exception of using <Q-3> instead of <A-2>, was carried out to afford <Q-4>. (yield 68%)

### Synthesis Example 17-5. Synthesis of Q-5

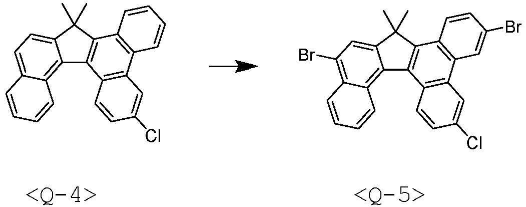

The same synthesis procedure as in Synthesis Example 1-4, with the exception of using <Q-4> instead of <A-3>, was carried out to afford <Q-5>. (yield 62%)

### Synthesis Example 17-6. Synthesis of Q-6

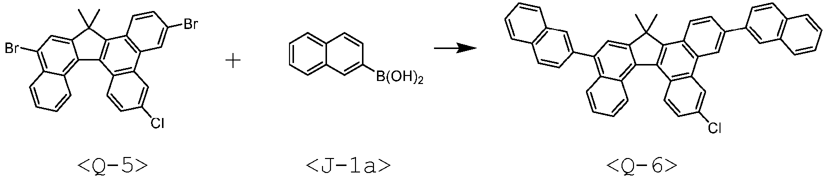

The same synthesis procedure as in Synthesis Example 1-1, with the exception of using <J-la> and <Q-5> instead of <A-1a> and <A-1b>, respectively, was carried out to afford <Q-6>. (yield 71%)

### Synthesis Example 17-7. Synthesis of [Compound 1-95]

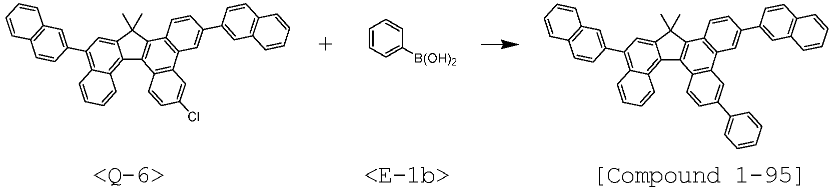

The same synthesis procedure as in Synthesis Example 1-1, with the exception of using <E-1b> and <Q-6> instead of <A-1a> and <A-1b>, respectively, was carried out to afford [Compound 1-95]. (yield 82%)
MS (MALDI-TOF) : m/z 672.28 [M⁺]

### SYNTHESIS EXAMPLE 18. Synthesis of [Compound 1-101]

### Synthesis Example 18-1. Synthesis of R-1

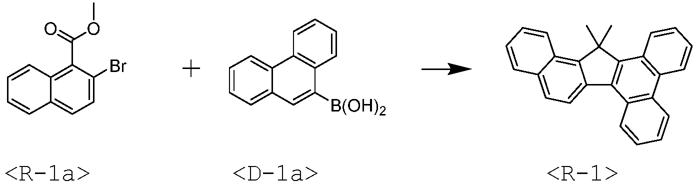

The same synthesis procedure as in Synthesis Example 1-1, with the exception of using <D-1a> and <R-1a> instead of <A-1a> and <A-1b>, respectively, was carried out to afford <R-1>. (yield 76%)

### Synthesis Example 18-2. Synthesis of R-2

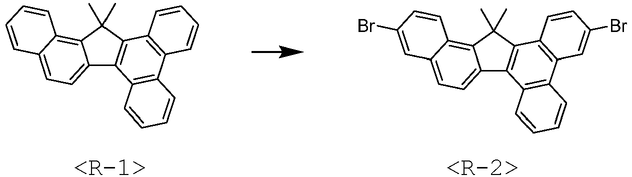

The same synthesis procedure as in Synthesis Example 1-4, with the exception of using <R-1> instead of <A-3>, was carried out to afford <R-2>. (yield 69%)

### Synthesis Example 18-3. Synthesis of [Compound 1-101]

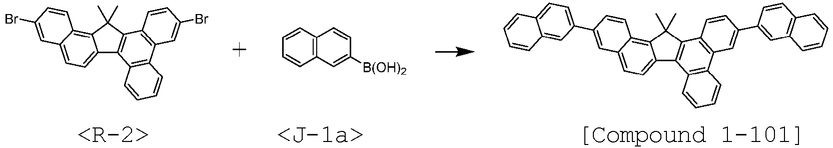

The same synthesis procedure as in Synthesis Example 1-1, with the exception of using <J-1a> and <R-2> instead of <A-1a> and <A-1b>, respectively, was carried out to afford [Compound 1-101]. (yield 84%)
MS (MALDI-TOF) : m/z 596.25 [M⁺]

### SYNTHESIS EXAMPLE 19. Synthesis of [Compound 1-104]

### Synthesis Example 19-1. Synthesis of S-1

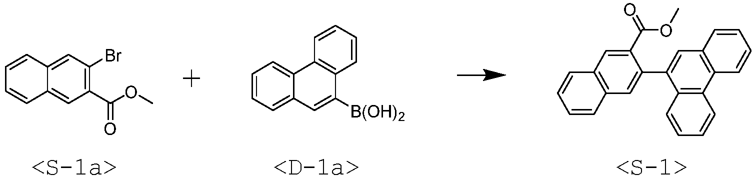

The same synthesis procedure as in Synthesis Example 1-1, with the exception of using <D-1a> and <S-1a> instead of <A-1a> and <A-1b>, respectively, was carried out to afford <S-1>. (yield 80%)

### Synthesis Example 19-2. Synthesis of S-2

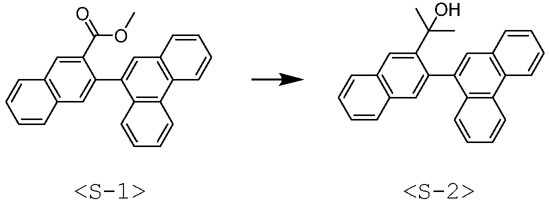

The same synthesis procedure as in Synthesis Example 1-2, with the exception of using <S-1> instead of <A-1>, was carried out to afford <S-2>. (yield 71%)

### Synthesis Example 19-3. Synthesis of S-3

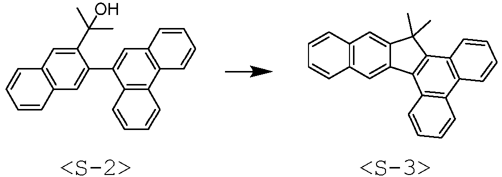

The same synthesis procedure as in Synthesis Example 1-3, with the exception of using <S-2> instead of <A-2>, was carried out to afford <S-3>. (yield 68%)

### Synthesis Example 19-4. Synthesis of S-4

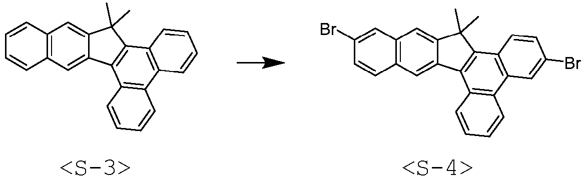

The same synthesis procedure as in Synthesis Example 1-4, with the exception of using <S-3> instead of <A-3>, was carried out to afford <S-4>. (yield 50%)

### Synthesis Example 19-5. Synthesis of [Compound 1-104]

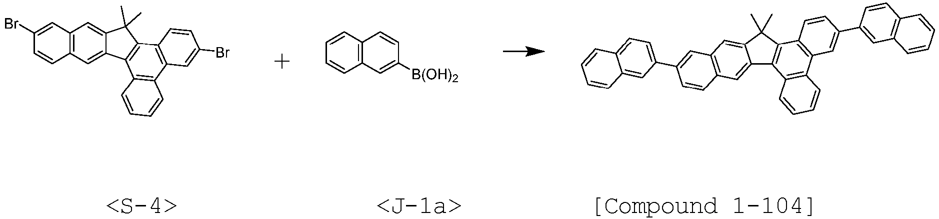

The same synthesis procedure as in Synthesis Example 1-1, with the exception of using <J-1a> and <S-4> instead of <A-1a> and <A-1b>, respectively, was carried out to afford [Compound 1-104]. (yield 71%)
MS (MALDI-TOF) : m/z 596.25 [M⁺]

### SYNTHESIS EXAMPLE 20. Synthesis of [Compound 1-108]

### Synthesis Example 20-1. Synthesis of T-1

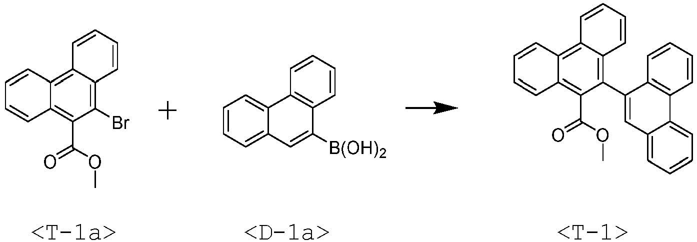

The same synthesis procedure as in Synthesis Example 1-1, with the exception of using <D-1a> and <T-1a> instead of <A-1a> and <A-1b>, was carried out to afford <T-1>. (yield 79%)

### Synthesis Example 20-2. Synthesis of T-2

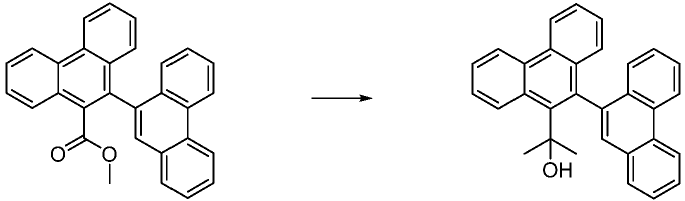

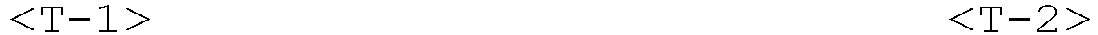

The same synthesis procedure as in Synthesis Example 1-2, with the exception of using <T-1> instead of <A-1>, was carried out to afford <T-2>. (yield 71%)

### Synthesis Example 20-3. Synthesis of T-3

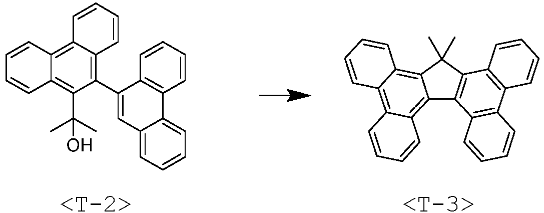

The same synthesis procedure as in Synthesis Example 1-3, with the exception of using <T-2> instead of <A-2>, was carried out to afford <T-3>. (yield 85%)

### Synthesis Example 20-4. Synthesis of T-4

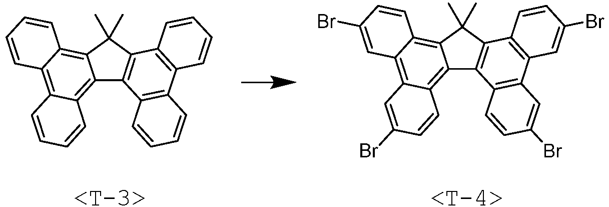

The same synthesis procedure as in Synthesis Example 1-4, with the exception of using <T-3> instead of <A-3>, was carried out to afford <T-4>. (yield 62%)

### Synthesis Example 20-5. Synthesis of [Compound 1-108]

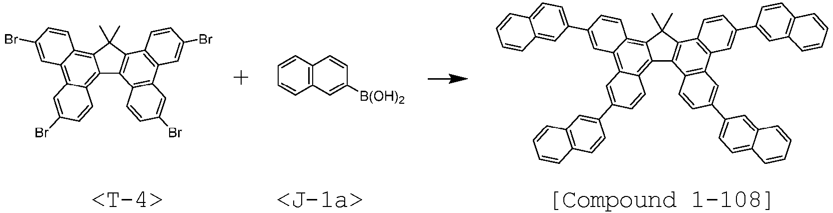

The same synthesis procedure as in Synthesis Example 1-1, with the exception of using <J-1a> and <T-4> instead of <A-1a> and <A-1b>, respectively, was carried out to afford [Compound 1-108]. (yield 68%)
MS (MALDI-TOF) : m/z 898.36 [M⁺]

### EXAMPLES 1 TO 32 : Fabrication of Organic Light-Emitting Diodes including 1^{st} and 2^{nd} Emission Layers

An ITO glass substrate was patterned to have a translucent area of 2 mm x 2 mm and cleansed. The ITO glass was mounted in a vacuum chamber that was then set to have a base pressure of 1x10⁻⁷ torr. On the ITO glass substrate, HAT-CN (700 Å) and α-NPD (300 Å) were sequentially deposited to form a hole injection layer and hole transport layer, respectively. Then, a first emission layer and a second emission layer were sequentially formed as emission layers, wherein the first emission layer was established by forming a film (50 Å) from a combination of a compound represented by Chemical Formula 1 according to the present disclosure and a polycyclic compound (1 wt%) selected from the following [BD-1] to [BD-3] and the second emission layer was established by forming a film (150 Å) from a combination of an anthracene compound represented by the [BH-1] or [BH-2] and a polycyclic compound (1 wt%) selected from the following [BD-1] to [BD-3]. Thereafter, films were formed from a mixture of 1:1 of [E-1] and [E-2] for an electron transport layer (300 Å), [E-2] for an electron injection layer (10 Å), and Al (1000 Å) for a cathode in the order, thereby fabricating an organic light-emitting diode. The organic light-emitting diodes thus obtained were measured at 0.4 mA for luminescence properties:

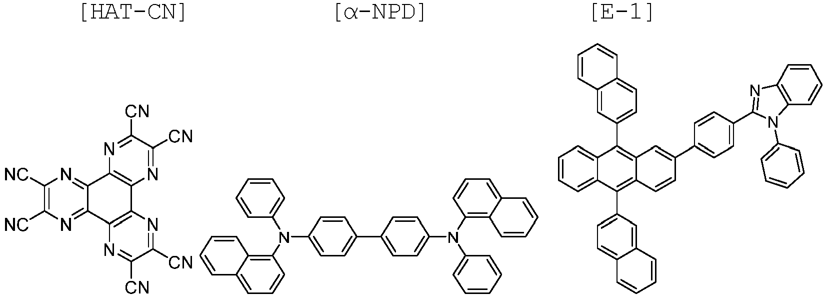

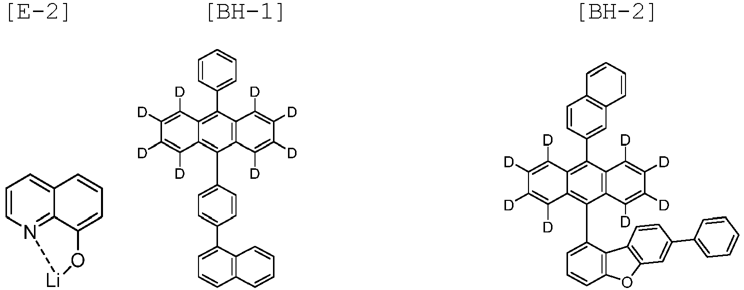

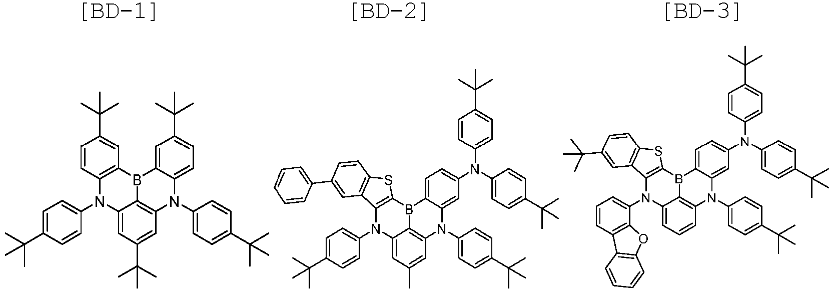

### COMPARATIVE EXAMPLES 1 TO 20

Organic light-emitting diodes for the Comparative Examples were fabricated in the same manner as in the Examples, with the exception of using the following [RH-1] to [RH-6], instead of the compounds according to the present disclosure, as hosts in the first and second emission layers. The OLEDs thus obtained were measured at 0.4 mA for luminescence properties. Here, the structures of [RH-1] to [RH-6] are as follows:

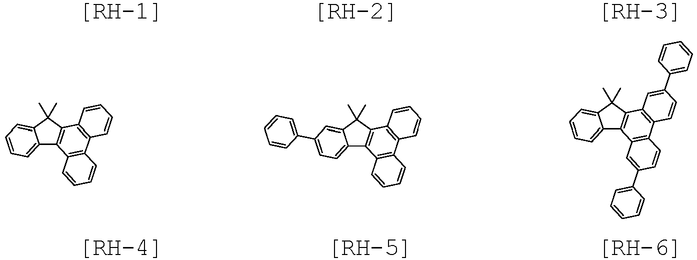

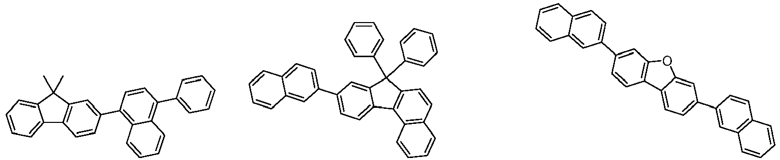

**TABLE 1**

| Ex. No. | 1^{st} Emission layer | | 2^{nd} Emission layer | | Driving Volt. (V) | External Quantum Effici. (EQE, %) | Lifetime (LT97, hr) |
|---|---|---|---|---|---|---|---|
| | Host | Dopant | Host | Dopant | | | |
| Ex. 1 | 1-7 | BD-2 | BH-1 | BD-2 | 3.4 | 11.0 | 278 |
| Ex. 2 | 1-11 | BD-2 | BH-1 | BD-2 | 3.2 | 10.5 | 264 |
| Ex. 3 | 1-24 | BD-2 | BH-1 | BD-2 | 3.3 | 10.6 | 265 |
| Ex. 4 | 1-34 | BD-2 | BH-1 | BD-2 | 3.1 | 10.8 | 261 |
| Ex. 5 | 1-39 | BD-2 | BH-1 | BD-2 | 3.2 | 10.6 | 265 |
| Ex. 6 | 1-41 | BD-2 | BH-1 | BD-2 | 3.3 | 10.6 | 266 |
| Ex. 7 | 1-64 | BD-2 | BH-1 | BD-2 | 3.3 | 10.6 | 260 |
| Ex. 8 | 1-73 | BD-2 | BH-1 | BD-2 | 3.2 | 10.8 | 285 |
| Ex. 9 | 1-84 | BD-2 | BH-1 | BD-2 | 3.4 | 11.0 | 290 |
| Ex. 10 | 1-95 | BD-2 | BH-1 | BD-2 | 3.4 | 11.0 | 282 |
| Ex. 11 | 1-101 | BD-2 | BH-1 | BD-2 | 3.4 | 10.8 | 274 |
| Ex. 12 | 1-108 | BD-2 | BH-1 | BD-2 | 3.4 | 11.0 | 288 |
| Ex. 13 | 1-11 | BD-2 | BH-2 | BD-2 | 3.1 | 11.5 | 274 |
| Ex. 14 | 1-41 | BD-2 | BH-2 | BD-2 | 3.2 | 11.6 | 276 |
| Ex. 15 | 1-64 | BD-2 | BH-2 | BD-2 | 3.2 | 11.6 | 270 |
| Ex. 16 | 1-84 | BD-2 | BH-2 | BD-2 | 3.3 | 12.0 | 300 |
| Ex. 17 | 1-95 | BD-2 | BH-2 | BD-2 | 3.3 | 12.0 | 292 |
| Ex. 18 | 1-101 | BD-2 | BH-2 | BD-2 | 3.3 | 11.8 | 284 |
| Ex. 19 | 1-11 | BD-3 | BH-2 | BD-3 | 3.2 | 12.0 | 279 |
| Ex. 20 | 1-24 | BD-3 | BH-2 | BD-3 | 3.3 | 12.1 | 280 |
| Ex. 21 | 1-41 | BD-3 | BH-2 | BD-3 | 3.3 | 12.1 | 281 |
| Ex. 22 | 1-64 | BD-3 | BH-2 | BD-3 | 3.3 | 12.1 | 275 |
| Ex. 23 | 1-95 | BD-3 | BH-2 | BD-3 | 3.4 | 12.5 | 297 |
| Ex. 24 | 1-101 | BD-3 | BH-2 | BD-3 | 3.4 | 12.3 | 289 |
| Ex. 25 | 1-7 | BD-1 | BH-1 | BD-1 | 3.5 | 10.5 | 268 |
| Ex. 26 | 1-11 | BD-1 | BH-1 | BD-1 | 3.3 | 10.0 | 254 |
| Ex. 27 | 1-39 | BD-1 | BH-1 | BD-1 | 3.3 | 10.1 | 255 |
| Ex. 28 | 1-41 | BD-1 | BH-1 | BD-1 | 3.4 | 10.1 | 256 |
| Ex. 29 | 1-84 | BD-1 | BH-2 | BD-1 | 3.4 | 11.5 | 290 |
| Ex. 30 | 1-95 | BD-1 | BH-2 | BD-1 | 3.4 | 11.5 | 282 |
| Ex. 31 | 1-101 | BD-1 | BH-2 | BD-1 | 3.4 | 11.3 | 274 |
| Ex. 32 | 1-108 | BD-1 | BH-2 | BD-1 | 3.4 | 11.5 | 288 |
| C. Ex. 1 | RH-1 | BD-1 | BH-1 | BD-1 | 4.0 | 6.5 | 86 |
| C. Ex. 2 | RH-1 | BD-2 | BH-1 | BD-2 | 3.9 | 6.8 | 91 |
| C. Ex. 3 | RH-2 | BD-1 | BH-1 | BD-1 | 4.1 | 6.6 | 88 |
| C. Ex. 4 | RH-2 | BD-2 | BH-1 | BD-2 | 4.0 | 6.9 | 93 |
| C. Ex. 5 | RH-2 | BD-2 | BH-2 | BD-2 | 3.9 | 7.2 | 98 |
| C. Ex. 6 | RH-3 | BD-1 | BH-1 | BD-1 | 4.1 | 6.5 | 92 |
| C. Ex. 7 | RH-3 | BD-2 | BH-1 | BD-2 | 4.0 | 6.8 | 97 |
| C. Ex. 8 | RH-3 | BD-2 | BH-2 | BD-2 | 3.9 | 7.1 | 102 |
| C. Ex. 9 | RH-4 | BD-1 | BH-1 | BD-1 | 4.2 | 6.2 | 67 |
| C. Ex. 10 | RH-4 | BD-2 | BH-1 | BD-2 | 4.1 | 6.5 | 72 |
| C. Ex. 11 | RH-5 | BD-1 | BH-1 | BD-1 | 4.0 | 7.0 | 15 |
| C. Ex. 12 | RH-5 | BD-2 | BH-1 | BD-2 | 3.9 | 6.9 | 17 |
| C. Ex. 13 | RH-6 | BD-1 | BH-1 | BD-1 | 3.9 | 7.0 | 36 |
| C. Ex. 14 | RH-6 | BD-2 | BH-1 | BD-2 | 3.8 | 6.9 | 41 |
| C. Ex. 15 | 1-7 | BD-1 | | | 3.9 | 8.5 | 50 |
| C. Ex. 16 | 1-11 | BD-1 | | | 3.7 | 8.0 | 46 |
| C. Ex. 17 | 1-39 | BD-2 | | | 3.7 | 8.1 | 48 |
| C. Ex. 18 | 1-41 | BD-2 | | | 3.8 | 8.1 | 47 |
| C. Ex. 19 | 1-84 | BD-3 | | | 3.7 | 9.5 | 54 |
| C. Ex. 20 | 1-95 | BD-3 | | | 3.7 | 9.5 | 51 |

As shown in Table 1, it was confirmed that the organic light-emitting diodes (OLEDs) including the compounds according to the present disclosure in the first emission layer and the second emission layer exhibited high efficiency and long lifetime characteristics, with improved luminous efficiency and device lifetime at lower driving voltages, in comparison to the OLEDs using comparative compounds according to conventional technologies (Comparative Examples 1 to 14).

In addition, when compared to the OLEDs (Comparative Examples 15 to 20) using single emission layers having the same total thickness as the combined thicknesses of the first and second emission layers of the OLEDs according to the present disclosure, rather than a two-layered emission structure, it was confirmed that the OLEDs of the present disclosure exhibited significantly superior luminous efficiency and lifetime characteristics in all aspects.

This is presumed to be due to the efficient energy transfer enabled by the formation of excitons in the first light-emitting and their subsequent transfer to the second emission layer, where triplet-triplet fusion occurs, in the multi-layered emission structure of the present disclosure, thereby providing high efficiency and long lifetime.

In contrast, in the case of a device employing a single emission layer, as in the comparative examples, all emission processes must occur within a single layer, which reduces the probability of triplet-triplet fusion. This, in turn, is presumed to lead to device degradation, resulting in reduced efficiency and lifetime. Therefore, it was confirmed that it is easier to fabricate a high-efficiency, long-lifetime OLED by using both the first and second emission layers, compared to a single emission layer.

### EXAMPLES 33 TO 60 : Fabrication of Organic Light-Emitting Diodes Including 1^{st} and 2^{nd} Emission Layers

Organic light-emitting diodes with first and second emission layers are fabricated in the same manner as in Examples 1 to 32, with the exception that either or both of the emission layers employed two compounds of the following [BH-3] to [BH-7] as a host and each of the emission layers employed the following [BD-4] as a dopant. Here, the structures of [BH-3] to [BH-7] and [BD-4] are as follows and the OLEDs thus obtained were measured at 0.4 mA for luminescence properties.

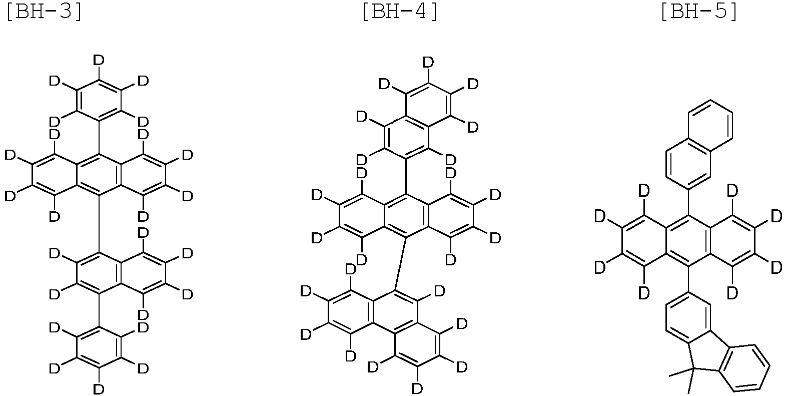

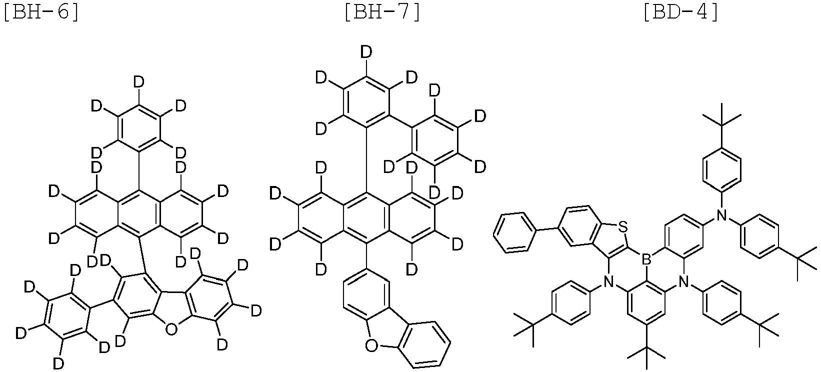

**TABLE 2**

| Ex. No. | 1^{st} Emission layer | | 2^{nd} Emission layer | | Driving Volt. (V) | External Quantum Effici. (EQE, %) | Lifetime (LT97, hr) |
|---|---|---|---|---|---|---|---|
| | Host | | Host | | | | |
| Ex. 33 | 1-32 | | BH-3 | BH-6 | 3.2 | 12.1 | 266 |
| Ex. 34 | 1-33 | | BH-3 | BH-6 | 3.2 | 12.3 | 290 |
| Ex. 35 | 1-39 | | BH-3 | BH-6 | 3.3 | 12.1 | 250 |
| Ex. 36 | 1-41 | | BH-3 | BH-6 | 3.3 | 12.0 | 248 |
| Ex. 37 | 1-57 | | BH-3 | BH-6 | 3.1 | 11.9 | 260 |
| Ex. 38 | 1-78 | | BH-3 | BH-6 | 3.3 | 12.0 | 245 |
| Ex. 39 | 1-89 | | BH-3 | BH-6 | 3.3 | 12.2 | 240 |
| Ex. 40 | 1-92 | | BH-3 | BH-6 | 3.2 | 12.3 | 264 |
| Ex. 41 | 1-32 | | BH-4 | BH-6 | 3.3 | 12.0 | 263 |
| Ex. 42 | 1-33 | | BH-4 | BH-6 | 3.3 | 12.2 | 287 |
| Ex. 43 | 1-39 | | BH-5 | BH-6 | 3.4 | 12.2 | 240 |
| Ex. 44 | 1-41 | | BH-5 | BH-6 | 3.4 | 12.1 | 238 |
| Ex. 45 | 1-57 | | BH-3 | BH-4 | 3.3 | 12.0 | 250 |
| Ex. 46 | 1-78 | | BH-4 | BH-5 | 3.5 | 12.1 | 235 |
| Ex. 47 | 1-89 | | BH-5 | BH-7 | 3.3 | 12.3 | 230 |
| Ex. 48 | 1-92 | | BH-4 | BH-7 | 3.3 | 12.4 | 254 |
| Ex. 49 | 1-32 | 1-33 | BH-3 | | 3.3 | 12.3 | 275 |
| Ex. 50 | 1-39 | 1-41 | BH-4 | | 3.5 | 12.3 | 248 |
| Ex. 51 | 1-57 | 1-78 | BH-5 | | 3.5 | 12.3 | 240 |
| Ex. 52 | 1-89 | 1-92 | BH-6 | | 3.4 | 12.1 | 282 |
| Ex. 53 | 1-32 | 1-33 | BH-7 | | 3.1 | 12.2 | 252 |
| Ex. 54 | 1-39 | 1-41 | BH-3 | BH-5 | 3.5 | 12.4 | 238 |
| Ex. 55 | 1-57 | 1-78 | BH-3 | BH-7 | 3.5 | 12.4 | 230 |
| Ex. 56 | 1-89 | 1-92 | BH-4 | BH-5 | 3.5 | 12.3 | 262 |
| Ex. 57 | 1-32 | 1-33 | BH-4 | BH-6 | 3.2 | 12.4 | 295 |
| Ex. 58 | 1-39 | 1-41 | BH-4 | BH-7 | 3.5 | 12.4 | 238 |
| Ex. 59 | 1-57 | 1-78 | BH-5 | BH-6 | 3.4 | 12.2 | 260 |
| Ex. 60 | 1-89 | 1-92 | BH-6 | BH-7 | 3.5 | 12.2 | 255 |

As shown in Table 2, it was confirmed that the device comprising two or more different compounds as a host in at least one of the first emission layer and the second emission layer of the OLED according to the present disclosure exhibited superior characteristics in terms of low driving voltage, luminous efficiency, and device lifetime.

## Claims

1. An organic light-emitting diode, comprising:
a first electrode;
a second electrode facing the first electrode; and
a first emission layer containing a first host and a first dopant and a second emission layer containing a second host and a second dopant, both layers being interposed between the first electrode and the second electrode,
wherein at least one of the first host and the second host includes a compound represented by the following Chemical Formula 1: 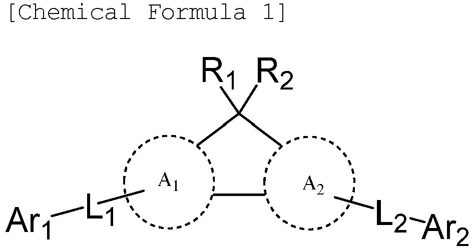
wherein,
A₁ is a substituted or unsubstituted aromatic hydrocarbon ring of 6 to 14 carbon atoms,
A₂ is a substituted or unsubstituted aromatic hydrocarbon ring of 6 to 14 carbon atoms, or a substituted or unsubstituted aliphatic hydrocarbon ring-fused aromatic hydrocarbon ring of 8 to 20 carbon atoms wherein the aromatic ring moiety within the aromatic hydrocarbon ring fused to the aliphatic hydrocarbon ring has 6 to 14 carbon atoms,
the substituents R₁ and R₂, which are same or different, are each independently any one selected from a substituted or
unsubstituted alkyl of 1 to 30 carbon atoms, a substituted or unsubstituted aryl of 6 to 50 carbon atoms, a substituted or unsubstituted heteroaryl of 2 to 50 carbon atoms, and a substituted or unsubstituted aliphatic hydrocarbon ring-fused aryl of 8 to 30 carbon atoms,
the substituents Ar₁ and Ar₂, which are same or different, are each independently any one selected from a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted alkoxy of 1 to 30 carbon atoms, a substituted or unsubstituted aryloxy of 6 to 30 carbon atoms, a substituted or unsubstituted cycloalkyloxy of 3 to 30 carbon atoms, a substituted or unsubstituted heteroaryloxy of 2 to 30 carbon atoms, a substituted or unsubstituted alkylthio of 1 to 30 carbon atoms, a substituted or unsubstituted arylthio of 6 to 30 carbon atoms, a substituted or unsubstituted cycloalkylthio of 3 to 30 carbon atoms, a substituted or unsubstituted heteroarylthio of 2 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
L₁ and L₂, which are same or different, are each independently a linker selected from a single bond, a substituted or unsubstituted arylene of 6 to 24 carbon atoms, a substituted or unsubstituted heteroarylene of 3 to 24 carbon atoms, and a substituted or unsubstituted aliphatic hydrocarbon ring-fused arylene of 8 to 24 carbon atoms,
wherein the term "substituted" in the expression "a substituted or unsubstituted" used for the compounds of Chemical Formula 1 means having at least one substituent selected from the group consisting of a deuterium atom, a tritium atom, a cyano, a halogen, a thiol, a hydroxy, a nitro, alkyl of 1 to 30 carbon atoms, halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocyclic alkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with at least one hydrogen atom on the substituent being substitutable with a deuterium or tritium atom.

2. The organic light-emitting diode of claim 1, wherein R₁ and R₂ in Chemical Formula 1 are same or different and are each independently a substituted or unsubstituted alkyl of 1 to 20 carbon atoms.

3. The organic light-emitting diode of claim 1, wherein A₁ in Chemical Formula 1 is a substituted or unsubstituted phenanthrene ring.

4. The organic light-emitting diode of claim 1, wherein A₂ in Chemical Formula 1 is a substituted or unsubstituted aromatic hydrocarbon ring of 6 to 14 carbon atoms.

5. The organic light-emitting diode of claim 1, wherein Ar₁ and Ar₂ in Chemical Formula 1 are same or different and are each independently any one selected from a substituted or unsubstituted aryl of 6 to 18 carbon atoms, a substituted or unsubstituted heteroaryl of 2 to 18 carbon atoms, a substituted or unsubstituted aliphatic hydrocarbon ring-fused aryl of 8 to 20 carbon atoms, a substituted or unsubstituted a silyl of 1 to 30 carbon atoms, and a substituted or unsubstituted germanium of 1 to 30 carbon atoms.

6. The organic light-emitting diode of claim 1, wherein the compound represented by Chemical Formula 1 is any one selected from the group consisting of the following Compounds 1-1 to 1-108:

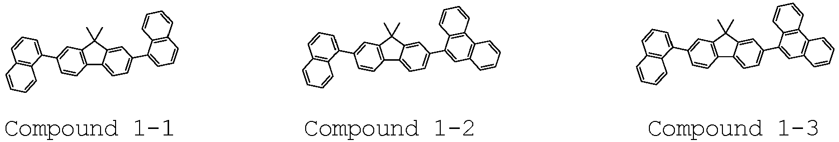

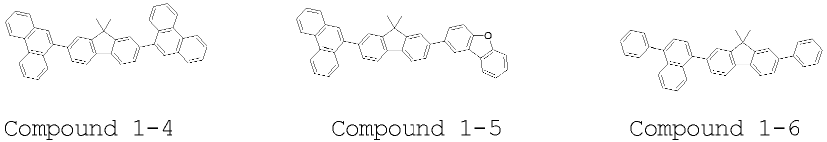

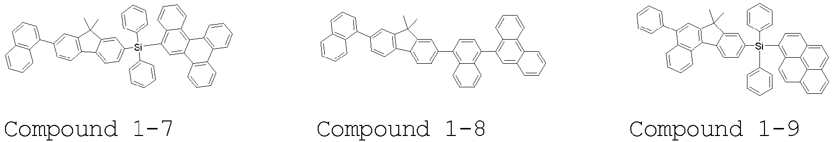

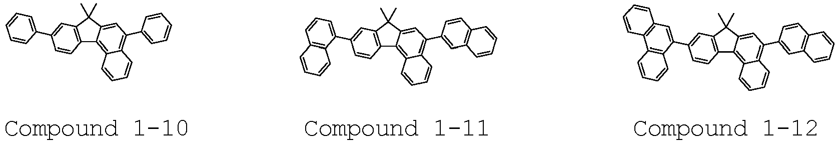

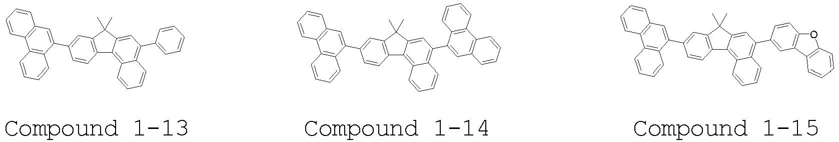

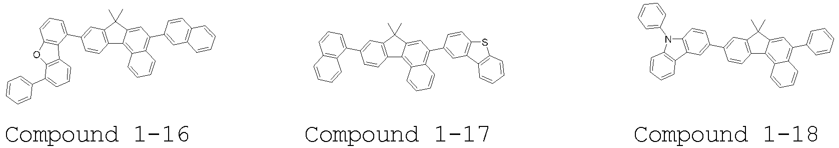

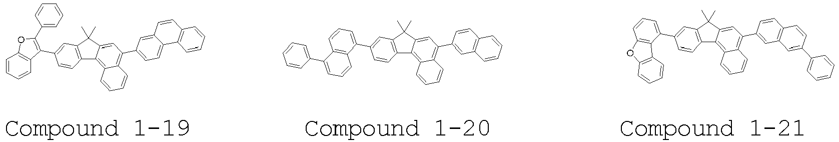

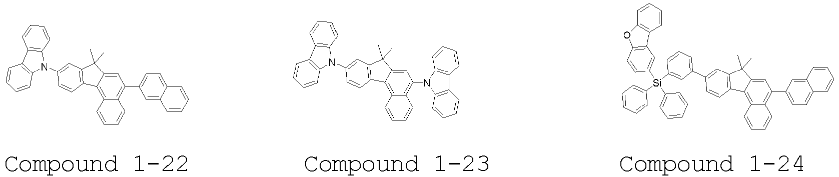

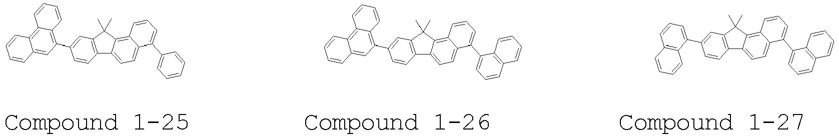

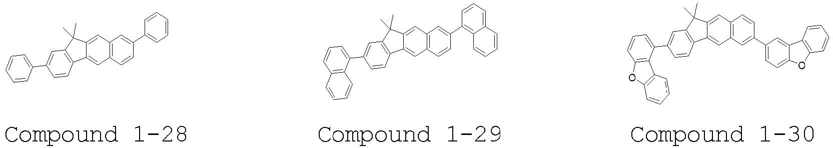

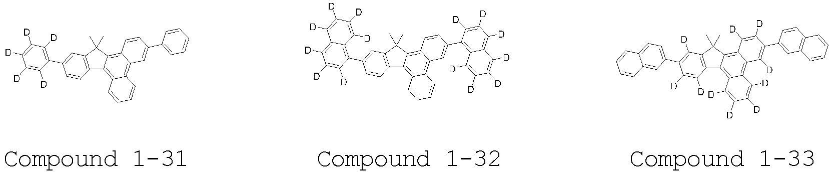

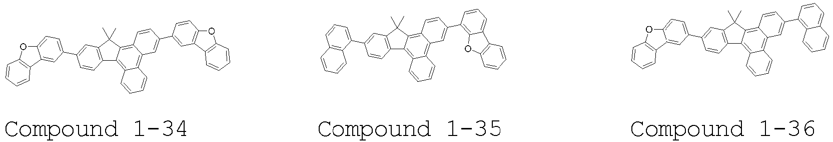

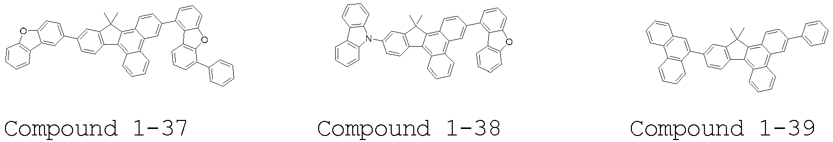

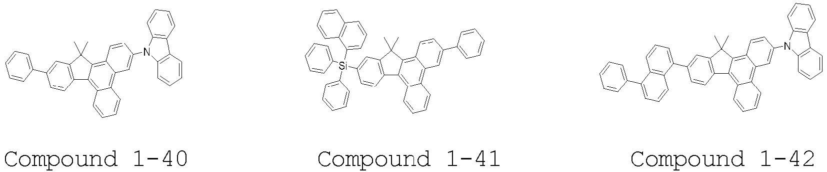

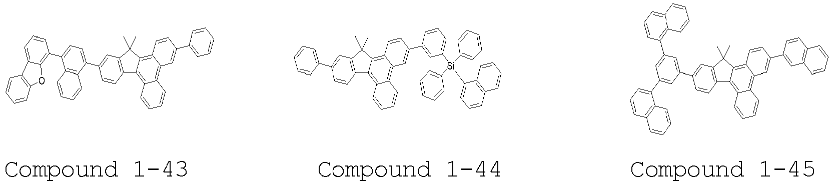

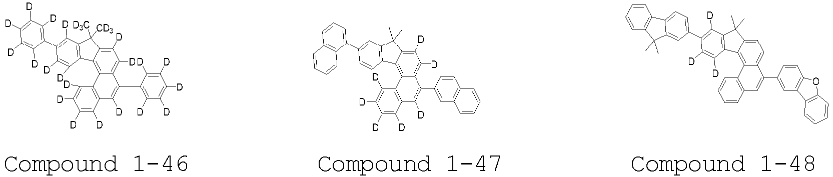

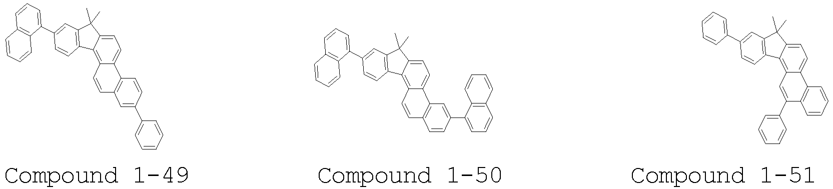

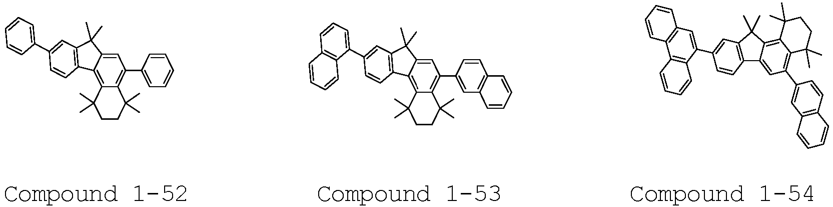

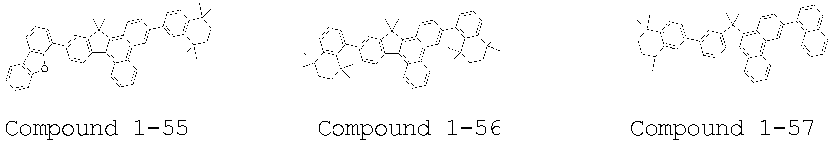

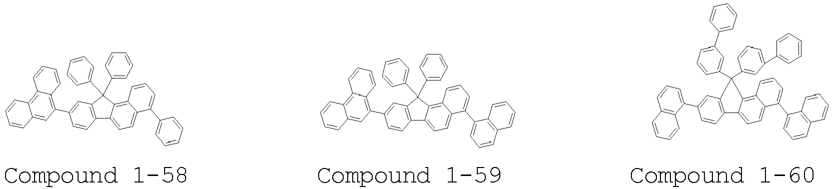

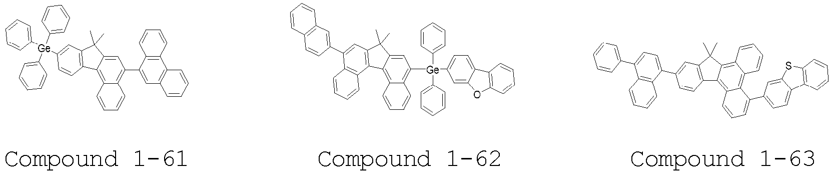

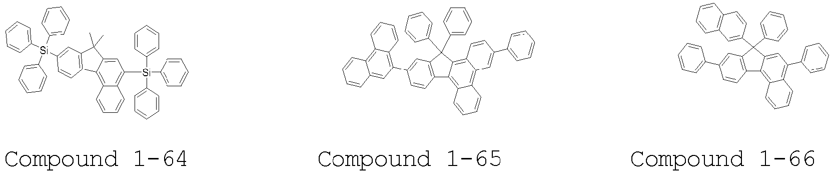

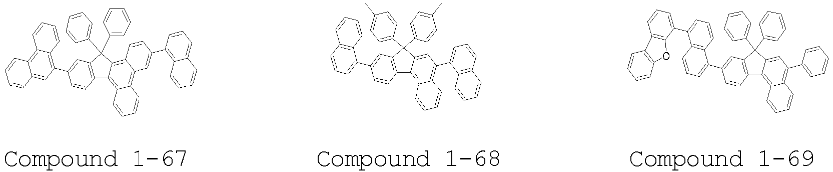

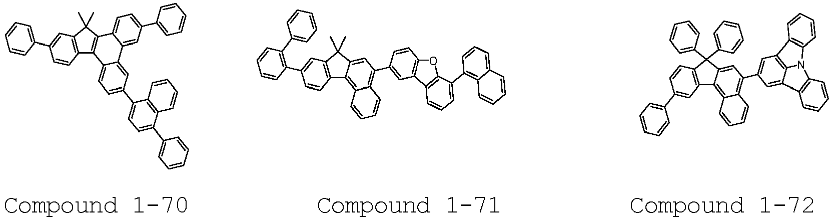

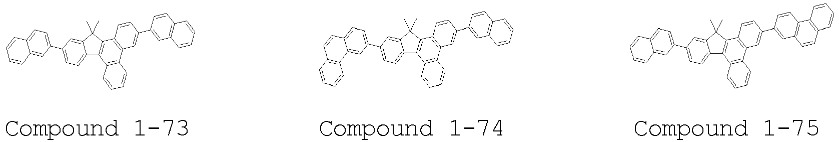

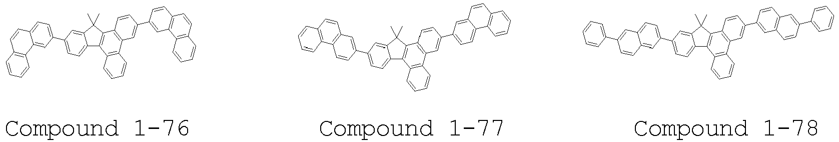

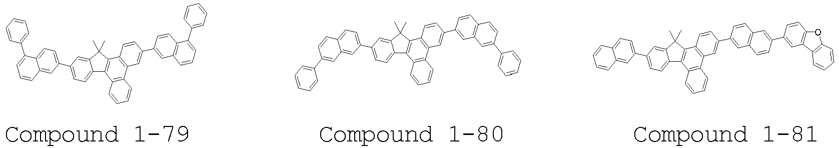

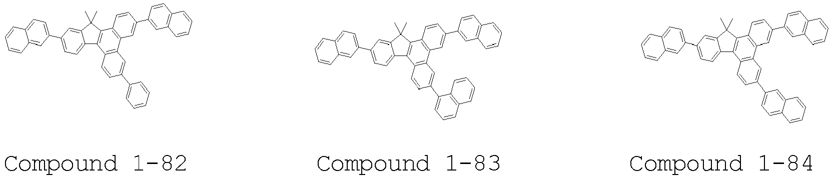

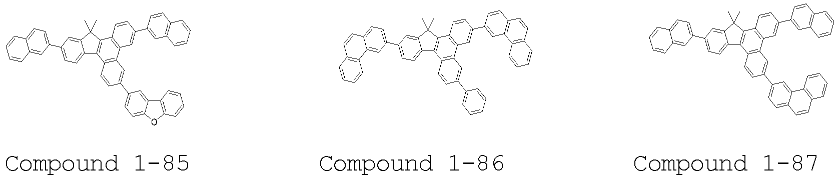

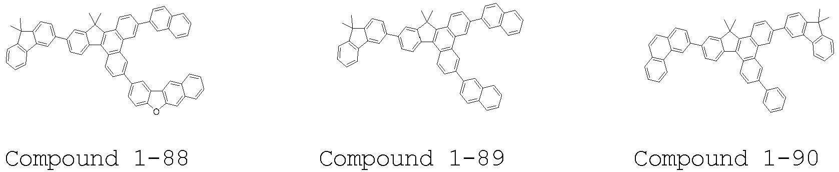

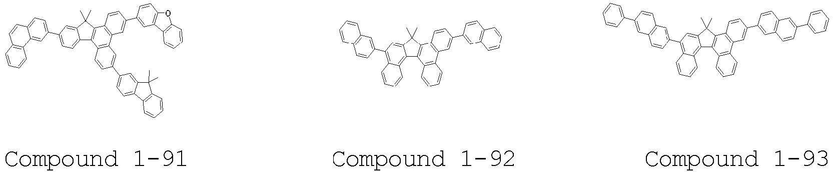

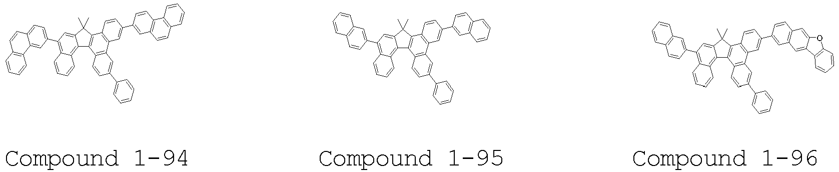

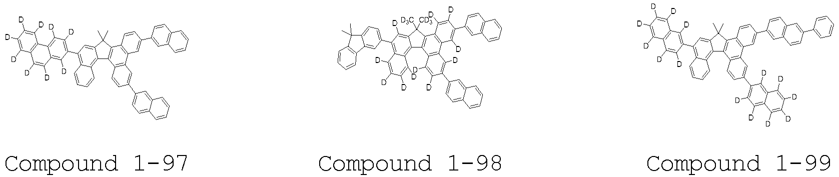

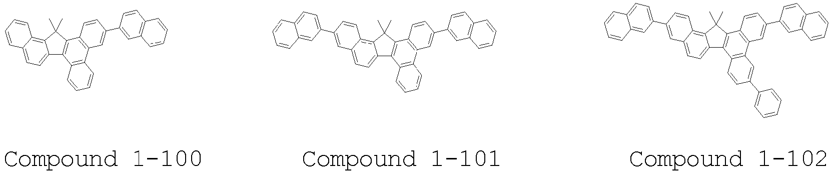

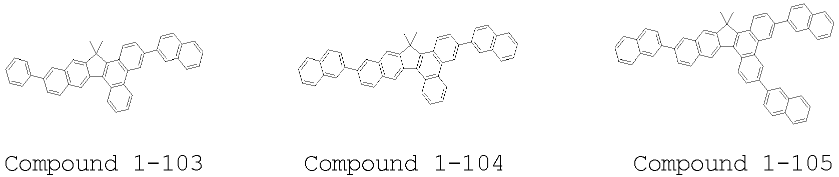

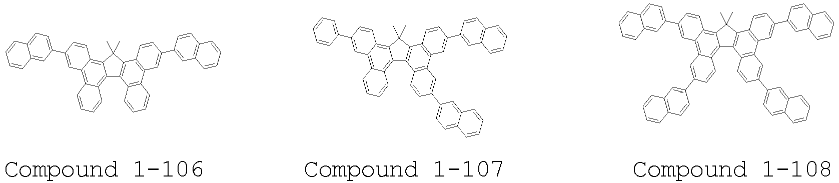

7. The organic light-emitting diode of claim 1, wherein one of the first host and the second host contains at least one type of the compound represented by Chemical Formula 1 and the remaining one contains at least one type of the compound represented by the following Chemical Formula 2: 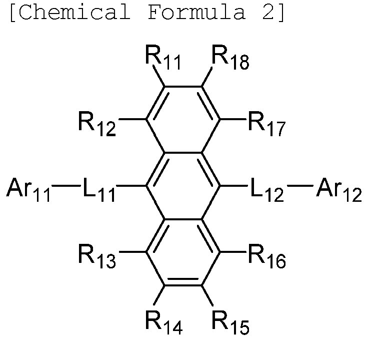 wherein,
the substituents R₁₁ to R₁₈, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or
unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted alkoxy of 1 to 30 carbon atoms, a substituted or unsubstituted aryloxy of 6 to 30 carbon atoms, a substituted or unsubstituted cycloalkyloxy of 3 to 30 carbon atoms, a substituted or unsubstituted heteroaryloxy of 2 to 30 carbon atoms, a substituted or unsubstituted alkylthio of 1 to 30 carbon atoms, a substituted or unsubstituted arylthio of 6 to 30 carbon atoms, a substituted or unsubstituted cycloalkylthio of 3 to 30 carbon atoms, a substituted or unsubstituted heteroarylthio of 2 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
L₁₁ and L₁₂, which are same or different, are each independently any one linker selected from a single bond, a substituted or unsubstituted arylene of 6 to 24 carbon atoms, a substituted or unsubstituted heteroarylene of 3 to 24 carbon atoms, and a substituted or unsubstituted aliphatic hydrocarbon ring-fused arylene of 8 to 24 carbon atoms,
Ar₁₁ and Ar₁₂, which are same or different, are each independently any one selected from a substituted or unsubstituted aryl of 6 to 30 carbon atoms, a substituted or unsubstituted heteroaryl of 3 to 30 carbon atoms, a substituted or unsubstituted cycloalkyl of 3 to 30 carbon atoms, a substituted or unsubstituted heterocyclic alkyl of 3 to 30 carbon atoms, and a substituted or unsubstituted, aliphatic hydrocarbon ring-fused aryl of 8 to 24 carbon atoms,
with the hydrogen atoms bonded to the carbon atoms within Chemical Formula 2 being substitutable with 0 to 60 deuterium atoms or tritium atoms,
wherein the term "substituted" in the expression "substituted or unsubstituted" used for the compound of Chemical Formula 2 means having at least one substituent selected from the group consisting of a deuterium atom, a tritium atom, a cyano, a halogen, a thiol, a hydroxy, a nitro, alkyl of 1 to 30 carbon atoms, halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, aromatic hydrocarbon ring-fused cycloalkyl of 7 to 30 carbon atoms, aromatic heterocyclic ring-fused cycloalkyl of 5 to 30 carbon atoms, aromatic hydrocarbon ring-fused heterocyclic alkyl of 6 to 30 carbon atoms, an aliphatic hydrocarbon ring-fused aryl of 7 to 30 carbon atoms, aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with one or more hydrogen atoms within the substituents being substitutable with deuterium atoms or tritium atoms.

8. The organic light-emitting diode of claim 7, wherein the Ar₁₂ in the anthracene compound represented by Chemical Formula 2 is a substituent represented by the following Structural Formula 12: 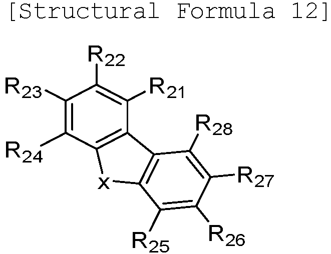 wherein,
X is O or S,
one of R₂₁ to R₂₄ is a single bond to L₁₂ in Chemical Formula 2, and
R₂₁ to R₂₈ exclusive of the single bond to L₁₂ are same or different and are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
wherein the term "substituted" in the expression "substituted or unsubstituted" used for the compound of Structural Formula 12 means having at least one substituent selected from the group consisting of a deuterium atom, a tritium atom, a cyano, a halogen, a thiol, a hydroxy, a nitro, alkyl of 1 to 30 carbon atoms, halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, aromatic hydrocarbon ring-fused cycloalkyl of 7 to 30 carbon atoms, aromatic heterocyclic ring-fused cycloalkyl of 5 to 30 carbon atoms, aromatic hydrocarbon ring-fused heterocyclic alkyl of 6 to 30 carbon atoms, an aliphatic hydrocarbon ring-fused aryl of 7 to 30 carbon atoms, aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, a thioxy of 1 to 30 carbon atoms, and a thionyl of 1 to 30 carbon atoms, with one or more hydrogen atoms within the substituents being substitutable with deuterium atoms or tritium atoms.

9. The organic light-emitting diode of claim 7, wherein the compound represented by Chemical Formula 2 comprises at least one deuterium atom.

10. The organic light-emitting diode of claim 7, wherein one of the first host and the second host within the organic light-emitting diode at least two different types of the anthracene compound represented by Chemical Formula 2.

11. The organic light-emitting diode of claim 10, wherein the Ar₁₂ in at least one type of the anthracene compound represented by Chemical Formula 2 is a substituted or unsubstituted aryl of 6 to 30 carbon atoms while the Ar₁₂ in at least one different type of the anthracene compound represented by Chemical Formula 2 is a substituted or unsubstituted heteroaryl of 2 to 30 carbon atoms.

12. The organic light-emitting diode of claim 11, wherein the Ar₁₂ in the at least one different type of the anthracene compound represented by Chemical Formula 2 is a substituent represented by the same Structural Formula as defined in claim 8.

13. The organic light-emitting diode of claim 7, wherein the first emission layer with in the organic light-emitting diode comprises a compound represented by Chemical Formula 1 as the first host and a host compound different from the compound represented by Chemical Formula 1 at a ratio of 1:9 to 9:1.

14. The organic light-emitting diode of claim 10, wherein the second emission layer within the organic light-emitting layer comprises two different types of the anthracene compound represented by Chemical Formula 2 at a ratio of 1:9 to 9:1.

15. A compound represented by the following Chemical Formula 1: 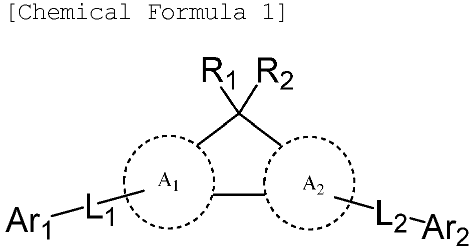 wherein,
A₁ is a substituted or unsubstituted aromatic hydrocarbon ring of 6 to 14 carbon atoms,
A₂ is a substituted or unsubstituted aromatic hydrocarbon ring of 6 to 14 carbon atoms, or a substituted or unsubstituted aliphatic hydrocarbon ring-fused aromatic hydrocarbon ring of 8 to 20 carbon atoms wherein the-aromatic ring moiety within the aromatic hydrocarbon ring fused to the aliphatic hydrocarbon ring has 6 to 14 carbon atoms,
the substituents R₁ and R₂, which are same or different, are each independently any one selected from a substituted or unsubstituted alkyl of 1 to 30 carbon atoms, a substituted or unsubstituted aryl of 6 to 50 carbon atoms, a substituted or unsubstituted heteroaryl of 2 to 50 carbon atoms, and a substituted or unsubstituted aliphatic hydrocarbon ring-fused aryl of 8 to 30 carbon atoms,
the substituents Ar₁ and Ar₂, which are same or different, are each independently any one selected from a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic hydrocarbon ring-fused heterocyclic alkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted alkoxy of 1 to 30 carbon atoms, a substituted or unsubstituted aryloxy of 6 to 30 carbon atoms, a substituted or unsubstituted cycloalkyloxy of 3 to 30 carbon atoms, a substituted or unsubstituted heteroaryloxy of 2 to 30 carbon atoms, a substituted or unsubstituted alkylthio of 1 to 30 carbon atoms, a substituted or unsubstituted arylthio of 6 to 30 carbon atoms, a substituted or unsubstituted cycloalkylthio of 3 to 30 carbon atoms, a substituted or unsubstituted heteroarylthio of 2 to 30 carbon atoms, a substituted or
unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
L₁ and L₂, which are same or different, are each independently a linker selected from a single bond, a substituted or unsubstituted arylene of 6 to 24 carbon atoms, a substituted or unsubstituted heteroarylene of 3 to 24 carbon atoms, and a substituted or unsubstituted aliphatic hydrocarbon ring-fused arylene of 8 to 24 carbon atoms,
wherein the term "substituted" in the expression "a substituted or unsubstituted" used for the compounds of Chemical Formula 1 means having at least one substituent selected from the group consisting of a deuterium atom, a tritium atom, a cyano, a halogen, a thiol, a hydroxy, a nitro, alkyl of 1 to 30 carbon atoms, halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an alkoxy of 1 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocyclic alkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an aryloxy of 6 to 24 carbon atoms, and an arylthio of 6 to 24 carbon atoms, with at least one hydrogen atom on the substituent being substitutable with a deuterium or tritium atom.
